# EUROPÄISCHE PATENTSCHRIFT

(11) **EP 1 272 504 B1**
(45) Veröffentlichungstag und Bekanntmachung des Hinweises auf die Patenterteilung: **30.05.2007**
(21) Anmeldenummer: 01931609.0
(22) Anmeldetag: 12.04.2001
(51) Int. Cl.: C07J 1/00, A61K 31/565, C07J 41/00, A61P 5/30

(54) **8.BETA.-HYDROCARBYL-SUBSTITUIERTE ESTRATRIENE ALS SELEKTIV WIRKSAME ESTROGENE**
8BETA-HYDROCARBYL-SUBSTITUTED ESTRATRIENES FOR USE AS SELECTIVE ESTROGENS
ESTRATRIENES A SUBSTITUTION 8BETA-HYDROCARBYLE UTILISES COMME OESTROGENES A ACTION SELECTIVE

(30) Priorität: 12.04.2000 DE 10019167; 26.05.2000 US 207370 P
(43) Veröffentlichungstag der Anmeldung: 08.01.2003
(73) Patentinhaber: Bayer Schering Pharma Aktiengesellschaft, 13353 Berlin (DE)
(72) Erfinder: PETERS, Olaf, 07745 Jena (DE); HILLISCH, Alexander, 07743 Jena (DE); THIEME, Ina, 07616 Graitschen (DE); ELGER, Walter, 14195 Berlin (DE); HEGELE-HARTUNG, Christa, 45470 Mülheim a. d. Ruhr (DE); KOLLENKIRCHEN, Uwe, 12209 Berlin (DE); FRITZEMEIER, Karl-Heinrich, 13505 Berlin (DE); PATCHEV, Vladimir, 07749 Jena (DE)
(86) Internationale Anmeldenummer: PCT/EP2001/004290
(87) Internationale Veröffentlichungsnummer: WO 2001/077139

(56) Entgegenhaltungen:
- DE-A- 4 018 828
- US-A- 3 681 407
- US-A- 3 736 345
- US-A- 3 806 546
- US-A- 4 961 931
- DATABASE CHEMABS [Online] CHEMICAL ABSTRACTS SERVICE, COLUMBUS, OHIO, US; NAGATA, WATARU ET AL: "8.BETA.-Cyanoestanes derivatives" retrieved from STN Database accession no. 73:109984 XP002175034 & JP 45 024572 B (SHIONOGI AND CO., LTD.) 15. August 1970 (1970-08-15)
- DATABASE CHEMABS [Online] CHEMICAL ABSTRACTS SERVICE, COLUMBUS, OHIO, US; NAGATA, WATARU ET AL: "8.beta.-Methylestradiols" retrieved from STN Database accession no. 73:25750 XP002175035 & JP 45 004059 B (SHIONOGI AND CO., LTD.) 10. Februar 1970 (1970-02-10)
- DATABASE CHEMABS [Online] CHEMICAL ABSTRACTS SERVICE, COLUMBUS, OHIO, US; NAGATA, WATARU ET AL: "8.beta.-Methylestranes" retrieved from STN Database accession no. 73:25749 XP002175036 & JP 45 004060 B (SHIONOGI AND CO., LTD.) 10. Februar 1970 (1970-02-10)
- DATABASE CHEMABS [Online] CHEMICAL ABSTRACTS SERVICE, COLUMBUS, OHIO, US; NAGATA, WATARU ET AL: "8.beta.-Methyl-9-dehydroestrone 3-ethers" retrieved from STN Database accession no. 73:25748 XP002175037 & JP 45 004061 B (SHIONOGI AND CO., LTD.) 10. Februar 1970 (1970-02-10)
- ELGER ET AL: "Novel oestrogen sulfamates: a new approach to oral hormone therapy" EXPERT OPINION ON INVESTIGATIONAL DRUGS, ASHLEY PUBLICATIONS LTD., LONDON, GB, Bd. 7, Nr. 4, April 1998 (1998-04), Seiten 575-589, XP002121926 ISSN: 1354-3784
- FEVIG T L ET AL: "ESTROGEN RECEPTOR BINDING TOLERANCE OF 16-ALPHA-SUBSTITUTED ESTRADIOL DERIVATIVES" STEROIDS: STRUCTURE, FUNCTION, AND REGULATION, ELSEVIER SCIENCE PUBLISHERS, NEW YORK, NY, US, Bd. 51, Nr. 5-6, 1988, Seiten 471-498, XP002159339 ISSN: 0039-128X
- DATABASE BIOSIS [Online] BIOSCIENCES INFORMATION SERVICE, PHILADELPHIA, PA, US; 1995 FERNANDEZ A I ET AL: "Influence of hormonal status in relaxant effect of diethylstilbestrol and nifedipine on isolated rat uterus contraction." Database accession no. PREV199598457779 XP002175038 & GENERAL PHARMACOLOGY, Bd. 26, Nr. 6, 1995, Seiten 1281-1287, ISSN: 0306-3623

## Beschreibung

### Feld der Erfindung

Die vorliegende Erfindung bezieht sich auf neue Verbindungen als pharmazeutische Wirkstoffe, die in vitro eine höhere Affinität an Estrogenrezeptorpräparationen von Rattenprostata als an Estrogenrezeptorpräparationen von Rattenuterus und in vivo eine präferentielle Wirkung am Knochen im Vergleich zum Uterus und/oder ausgeprägte Wirkung hinsichtlich Stimulierung der Expression von 5HT2a-Rezeptor und -transporter aufweisen, deren Herstellung, ihre therapeutische Anwendung und pharmazeutischen Darreichungsformen, die die neuen Verbindungen enthalten. Bei den chemischen Verbindungen handelt es sich um neuartige steroidale gewebeselektive Estrogene.

### Hintergrund der Erfindung

Etablierte Estrogentherapien zur Behandlung von hormondefizienzbedingten Beschwerden und die protektive Wirkung von Estrogenen auf Knochen, Gehirn, Gefäß und andere Organsysteme.

Die Effizienz von Estrogenen in der Behandlung von hormondefizienzbedingten Symptomen wie Hitzewallungen, Atrophie von Estrogenzielorganen und Inkontinenz, sowie die erfolgreiche Anwendung von Estrogen-Therapien zur Verhinderung von Knochenmasseverlust bei peri- und postmenopausalen Frauen, ist gut belegt und allgemein akzeptiert (Grady et al.1992, Ann Intern Med 117: 1016-1037). Ebenso ist gut dokumentiert, daß die Estrogenersatztherapie bei postmenopausalen Frauen oder bei Frauen mit anders bedingter ovarieller Dysfunktion, das Risiko von Herzkreislauferkrankungen gegenüber nicht estrogenbehandelten Frauen reduziert (Grady et al., loc. cit.). Neuere Untersuchungen belegen zudem eine protektive Wirkung von Estrogenen gegen neurodegenerative Erkrankungen, wie z.B. Alzheimersche Krankheit (Henderson 1997, Neurology 48 (Suppl 7): S27-S35; Birge 1997, Neurology 48 (Suppl 7): S36-S41), eine schützende Wirkung auf Gehirnfunktionen, wie Gedächtnisleistung und Lemfähigkeit (McEwen et al. 1997, Neurology 48 (Suppl 7): S8-S15; Sherwin 1997, Neurology 48 (Suppl 7): S21-S26), sowie gegen hormondefiziensbedingte Stimmungsschwankungen (Haibreich 1997, Neurology 48 (Suppl 7): S16-S20).

Weiterhin hat sich Estrogenersatztherapie als effektiv hinsichtlich der Reduktion der Inzidenz von Kolonrektalkarzinom erwiesen (Calle EF et al., 1995, J Natl Cancer Inst 87: 517-523).

In der herkömmlichen Estrogen- oder Hormonersatztherapie (Hormone Replacement Therapy = HRT) werden natürliche Estrogene, wie Estradiol und konjugierte Estrogene aus Pferdeurin entweder allein oder in Kombination mit einem Gestagen eingesetzt. Anstelle der natürlichen Estrogene können auch durch Veresterung erhaltene Derivate, wie z.B. das 17β-Estradiol-valerat, eingesetzt werden. Wegen der stimulierenden Wirkung der verwendeten Estrogene auf das Endometrium, die zu einer Erhöhung des Endometriumkarzinomrisikos führt (Harlap S 1992, Am J Obstet Gynecol 166: 1986-1992), werden in der Hormonersatztherapie vorzugsweise Estrogen/Gestagen-Kombinationspräparate eingesetzt. Die gestagene Komponente in der Estrogen/Gestagen-Kombination vermeidet eine Hypertrophie des Endometriums, allerdings ist mit der gestagen-haltigen Kombination auch das Auftreten ungewünschter Zwischenblutungen verknüpft.

Eine neuere Alternative zu den Estrogen/Gestagen-Kombinationspräparaten stellen selektive Estrogene dar. Bisher werden unter selektiven Estrogenen solche Verbindungen verstanden, die estrogenartig auf Gehirn, Knochen und Gefäßsystem, aufgrund ihrer antiuterotrophen (d.h. antiestrogenen) Partialwirkung aber nicht proliferativ auf das Endometrium wirken.

Eine Klasse von Substanzen, die das gewünschte Profil eines selektiven Estrogens teilweise erfüllen, sind die sogenannten ,Selective Estrogen Receptor Modulators' (SERM) (R.F. Kauffman, H.U. Bryant 1995, DNAP 8 (9): 531-539). Es handelt sich hierbei um Partialagonisten des Estrogenrezeptorsubtyps ,ERα'. Dieser Typ von Substanzen ist allerdings ineffektiv hinsichtlich der Therapie akuter postmenopausaler Beschwerden, wie z.B. Hitzewallungen. Als Beispiel für ein SERM sei das kürzlich für die Indikation Osteoporose eingeführte Raloxifen genannt.

### Estrogenrezeptor beta (ERβ)

Kürzlich wurde der Estrogenrezeptor-β (ERβ) als zweiter Subtyp des Estrogenrezeptors entdeckt (Kuiper et al. (1996), Proc. Natl. Acad. Sci. 93:5925-5930; Mosselman, Dijkema (1996) Febs Letters 392: 49-53; Tremblay et al. (1997), Molecular Endocrinology 11: 353-365). Das Expressionsmuster von ERβ unterscheidet sich von dem des ERα (Kuiper et al. (1996), Endocrinology 138: 863-870). So überwiegt ERβ gegenüber ERα in der Rattenprostata, während in Rattenuterus ERα gegenüber ERβ überwiegt. Im Gehirn wurden Areale identifiziert, in denen jeweils nur einer der beiden ER-Subtypen exprimiert wird (Shugrue et al. (1996), Steroids 61: 678-681; Li et al. (1997), Neuroendocrinology 66:63-67). ERβ wird u.a. in Arealen exprimiert, denen Bedeutung für kognitive Prozesse und ,Stimmung' zugewiesen wird (Shugrue et al. 1997, J Comparative Neurology 388:507-525).

Molekulare Targets für ERβ in diesen Gehimarealen könnten der 5HT2a-Rezeptor und der Serotonintransporter sein (G. Fink & B.E.H. Sumner 1996 Nature 383:306; B. E.H. Sumner et al. 1999 Molecular Brain Research, in press). Der Neurotransmitter Serotonin (5-Hydroxytryptamin) ist an der Regulation einer Vielzahl von Prozessen beteiligt, die in der Menopause beeinträchtigt sein können. Insbesondere die Effekte der Menopause auf Stimmung und Kognition werden mit dem serotonergen System in Verbindung gebracht. Estrogenersatztherapie hat sich als effektiv hinsichtlich Behandlung dieser Estrogendefizienz-bedingten Beschwerden erwiesen, möglicherweise durch Modulation von Serotoninrezeptor-und -Transporterexpression.

Weitere Organsysteme mit vergleichsweise hoher ERβ-Expression umfassen den Knochen (Onoe Y et al., 1997, Endocrinology 138:4509-4512), das Gefäßsystem (Register TC, Adams MR 1998, J Steroid Molec Biol 64: 187-191), den Urogenitaltrakt (Kuiper GJM et al. 1997, Endocrinology 138: 863-870), den Gastrointestinaltrakt (Campbell-Thopson 1997, BBRC 240: 478-483), sowie die Testis (Mosselmann S et al. 1996 Febs Lett 392 49-53) einschließlich der Spermatiden (Shugrue et al. 1998, Steroids 63: 498-504). Die Gewebeverteilung legt nahe, daß Estrogene über ERβ Organfunktionen regulieren. Daß ERβ in dieser Hinsicht funktionell ist, ergibt sich auch durch Untersuchungen an ERα- (ERKO) bzw. ERβ-(βERKO)-Knockout-Mäusen:Ovariektomie bewirkt Knochenmasseverlust in ERKO-Mäusen, der durch Estrogensubstitution aufgehoben werden kann (Kimbro et al. 1998, Abstract OR7-4, Endocrine Society Meeting New Orleans). Ebenso hemmt Estradiol in Blutgefäßen weiblicher ERKO-Mäuse die Gefäßmedia- und Glattmuskelzellproliferation (lafrati MD et al. 1997, Nature Medicine 3: 545-548). Diese protektiven Wirkungen von Estradiol erfolgen in der ERKO-Maus vermutlich über ERβ.

Beobachtungen an βERKO-Mäusen liefern einen Hinweis auf eine Funktion von ERβ in Prostata und Blase: bei älteren männlichen Mäusen treten Symptome von Prostata- und Blasenhyperplasie auf (Krege JH et al. 1998, Proc Natl Acad Sci 95: 15677-15682). Außerdem weisen weibliche (Lubahn DB et al. 1993, Proc Natl Acad Sci 90:11162-11166) und männliche ERKO-Mäuse (Hess RA et al. 1997, Nature 390: 509-512) sowie weibliche βERKO-Mäuse (Krege JH, 1998) Fertilitätsstörungen auf. Hierdurch wird die wichtige Funktion von Estrogenen hinsichtlich Aufrechterhaltung von Testis- und Ovarfunktion sowie Fertilität belegt.

Eine selektive Estrogenwirkung auf bestimmte Zielorgane könnte aufgrund der unterschiedlichen Gewebe- bzw. Organverteilungverteilung der beiden Subtypen des ERs durch subtypspezifische Liganden erreicht werden. Substanzen mit Präferenz für ERβ verglichen mit ERα im in vitro Rezeptorbindungstest wurden von Kuiper et al. beschrieben (Kuiper et al. (1996), Endocrinology 138: 863-870). Eine selektive Wirkung von subtypspezifischen Liganden des Estrogenrezeptors auf estrogensensitive Parameter in vivo wurde bisher nicht gezeigt.

Aufgabe der vorliegenden Erfindung ist es deshalb, Verbindungen bereitzustellen, die in vitro eine Dissoziation hinsichtlich Bindung an Estrogenrezeptorpräparationen von Rattenprostata und Rattenuterus und die in vivo eine Dissoziation hinsichtlich Knochen- im Vergleich zur Uteruswirkung aufweisen. Die Verbindungen sollen in vitro eine höhere Affinität an Estrogenrezeptorpräparationen von Rattenprostata als an Estrogenrezeptorpräparationen von Rattenuterus und in vivo eine höhere Potenz hinsichtlich Protektion gegen hormondefizienz-bedingten Knochenmasseverlust im Vergleich zur uterusstimulierenden Wirkung Uterus und/oder ausgeprägte Wirkung hinsichtlich Stimulierung der Expression von 5HT2a-Rezeptor und -transporter aufweisen.

US-A-3,806,546 beschreibt 8β-methyl-3-methoxy-estra-1,3,5(10)-trien Verbindungen mit generisch estrogen Aktivität.

Im weiteren Sinne soll durch die vorliegende Erfindung eine Struktur-Wirkungsbeziehung zur Verfügung gestellt werden, die den Zugang zu Verbindungen gestattet, die das oben formulierte pharmakologische Profil, bessere estrogene Wirkung am Knochen als am Uterus, besitzen.

Erfindungsgemäß gelöst wird die vorstehende Aufgabe durch die Bereitstellung der 8β-substituierten Estra-1,3,5(10)-trienderivate der allgemeinen Formel I' 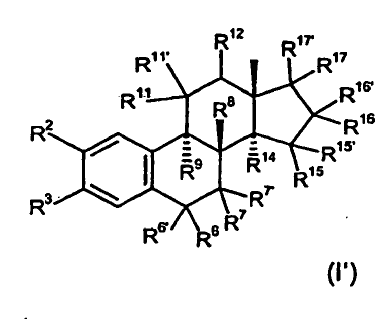 worin
- R²: ein Wasserstoffatom, ein Halogenatom;
ein Rest R¹⁸- oder R¹⁸-O-, wobei R¹⁸ ein Wasserstoffatom oder einen gerad- oder verzweigtkettigen, gesättigten oder ungesättigten Kohlenwasserstoffrest mit bis zu 6 Kohlenstoffatomen, eine Trifluormethylgruppe;
eine Gruppe R¹⁹SO₂-O-, worin R¹⁹ eine R²⁰R²¹N - Gruppe ist, wobei R²⁰ und R²¹, unabhängig voneinander ein Wasserstoffatom, einen C₁ - C⁵-Alkylrest, eine Gruppe C(O)R²², worin R²² einen gegebenenfalls substituierten, gerad- oder verzweigtkettigen, gesättigten oder bis zu dreifach ungesättigten, gegebenenfalls teilweise oder vollständig halogenierten Kohlenwasserstoffrest mit bis zu 10 Kohlenstoffatomen, einen gegebenenfalls substituierten C₃ - C₇ - Cycloalkylrest, einen gegebenenfalls substituierten C₄ - C₁₅ - Cycloalkylalkylrest oder einen gegebenenfalls substituierten Aryl-, Heteroaryl- oder Aralkylrest darstellt, oder, zusammen mit dem N-Atom, einen Polymethyleniminorest mit 4 bis 6 C-Atomen oder einen Morpholinorest, bedeuten;
- R³: eine Gruppe R¹⁸-O-, R¹⁹SO₂-O- oder -O-C(O)R²². mit R¹⁸, R¹⁹ und R²² jeweils in der unter R² angegebenen Bedeutung, wobei für R¹⁸ zusätzlich ein Aryl-, Heteroaryl- oder Aralkylrest stehen kann;
- R⁶ und R⁷: je ein Wasserstoffatom oder zusammen eine zusätzliche Bindung;
- R^{6'} und R^{7'}: unabhängig voneinander ein Wasserstoffatom, ein Halogenatom, eine Gruppe R¹⁸-O-, R¹⁹SO₂-O- oder-R²², mit R¹⁸, R¹⁹ und R²² jeweils in der unter R² angegebenen Bedeutung;
- R⁸: einen gerad- oder verzweigtkettigen, gegebenenfalls teilweise oder vollständig halogenierten Alkyl- oder Alkenylrest mit bis zu 5 Kohlenstoffatomen, einen Ethinyl- oder Prop-1-inylrest;
- R⁹: ein Wasserstoffatom, einen gerad- oder verzweigtkettigen, gesättigten oder ungesättigten Kohlenwasserstoffrest mit bis zu 5 Kohlenstoffatomen, oder gemeinsam mit R¹¹ eine zusätzliche Bindung:
- R¹¹: ein Wasserstoffatom oder zusammen mit R⁹ oder zusammen mit R¹² eine zusätzliche Bindung;
- R^{11'}: ein Wasserstoffatom, ein Halogenatom, einen gesättigten oder ungesättigten, gegebenenfalls teilweise oder vollständig halogenierten Kohlenwasserstoffrest, der eine maximale lineare Kettenlänge von 4 Kohlenstoffatomen aufweist, oder eine Gruppe-X- R^{18'}, worin X ein Sauerstoff- oder Schwefelatom ist und R^{18'} ein Alkylrest mit 1 bis 3 Kohlenstoffatomen ist;
- R¹²: ein Wasserstoffatom oder zusammen mit R¹¹ eine zusätzliche Bindung;
- R¹⁴: ein Wasserstoffatom oder zusammen mit R¹⁵ eine zusätzliche Bindung;
- R¹⁵: ein Wasserstoffatom oder zusammen mit R¹⁴ oder zusammen mit R¹⁶ eine zusätzliche Bindung;
- R¹⁶: ein Wasserstoffatom oder zusammen mit R¹⁵ eine zusätzliche Bindung;
- R^{15'} und R^{16'}: unabhängig voneinander ein Wasserstoffatom, ein Halogenatom, eine Gruppe R¹⁸-O-, R¹⁹SO₂-O- oder-R²², mit R¹⁸, R¹⁹ und R²² jeweils in der unter R² angegebenen Bedeutung;
- R¹⁷ und R^{17'}: je ein Wasserstoffatom; ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder-O-C(O)R²²; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R² angegebenen Bedeutung; oder
- R¹⁷ und R^{17'}: gemeinsam eine Gruppe =CR²³R²⁴, worin R²³ und R²⁴ unabhängig voneinander ein Wasserstoffatom und ein Halogenatom darstellen, oder gemeinsam ein Sauerstoffatom;
bedeuten.

als ER-β selektive Wirksame Estrogene zur Herstellung eines Arzneimittels zur Behandlung der nachstehenden estrogendefizienz-bedingter Krankheiten und Zustände,

Die möglichen. Substituenten an den Kohlenstoffatomen 6, 7, 11, 15, 16 und 17 können jeweils In der α- oder β-Position stehen.

Gemäß einer Variante der Erfindung werden vorzugsweise Verbindungen der allgemeinen Formel I' verwendet,
worin
- R²: ein Wasserstoff- oder Halogenatom oder eine Hydroxygruppe
- R³: eine Gruppe R¹⁸-O-, R¹⁹SO₂-O- oder-O-C(O)R²², mit R¹⁸, R¹⁹ und R²² jeweils in der unter R² angegebenen Bedeutung, wobei für R¹⁸ zusätzlich ein Aryl- oder Aralkylrest stehen kann;
- R⁶ und R⁷: je ein Wasserstoffatom;
- R^{6'}: ein Wasserstoffatom, ein Hydroxygruppe, eine Gruppe R²² in der unter R² angegebenen Bedeutung;
- R^{7'}: ein Wasserstoffatom, ein Halogenatom, eine Gruppe R¹⁸-O-, R¹⁹SO₂-O-oder-R²², mit R¹⁸, R¹⁹ und R²² jeweils in der unter R² angegebenen. Bedeutung,
- R⁸: einen gerad- oder verzweigtkettigen, gegebenenfalls teilweise oder vollständig halogenierten Alkyl- oder Alkenylrest mit bis zu 5 Kohlenstoffatomen, einen Ethinyl- oder Prop-1-inylrest;
- R⁹: ein Wasserstoffatom oder zusammen mit R¹¹ eine zusätzliche Bindung;
- R¹¹: ein Wasserstoffatom oder zusammen mit R⁹ eine zusätzliche Bindung;
- R^{11'}: ein Wasserstoffatom, ein Halogenatom, einen gesättigten oder ungesättigten, gegebenenfalls teilweise oder vollständig halogenierten Kohlenwasserstoffrest, der eine maximale lineare Kettenlänge von 4 Kohlenstoffatomen aufweist, oder eine Gruppe -X- R^{18'}, worin X ein Schwefelatom ist und R^{18'} ein Alkylrest mit 1 bis 3 Kohlenstoffatomen ist;
- R¹², R¹⁴, R¹⁵, R^{15'} und R¹⁶: jeweils ein Wasserstoffatom;
- R^{16'}: ein Wasserstoffatom, ein Halogenatom, eine Gruppe R¹⁸-O-, R¹⁹SO₂-O-oder -R²², mit R¹⁸, R¹⁹ und R²² jeweils in der unter R² angegebenen Bedeutung;
- R¹⁷ und R^{17'}: jeweils ein Wasserstoffatom; ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder-O-C(O)R²² ; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R² angegebenen Bedeutung; sowie
- R¹⁷ und R^{17'}: gemeinsam eine Gruppe =CR²³R²⁴, worin R²³ und R²⁴ unabhängig voneinander ein Wasserstoffatom und ein Halogenatom darstellen, oder gemeinsam ein Sauerstoffatom;
bedeuten.

Eine weitere bevorzugte Variante der vorliegenden Erfindung sieht die Verwendung solcher Verbindungen der allgemeinen Formel I' vor,
worin
- R²: ein Wasserstoff- oder Fluoratom oder eine Hydroxygruppe,
- R³: eine Gruppe R¹⁸-O-, R¹⁹SO₂-O- oder -O-C(O)R²², mit R¹⁸, R¹⁹ und R²² jeweils in der unter R² angegebenen Bedeutung, wobei für R¹⁸ zusätzlich ein Aryl- oder Aralkylrest stehen kann;
- R⁶ und R⁷: jeweils ein Wasserstoffatom:
- R^{6'}: ein Wasserstoffatom oder eine Hydroxygruppe,
- R^{7'}: ein Wasserstoffatom, ein Fluor- oder Chloratom, eine Gruppe R¹⁸-O-, R¹⁹SO₂-O- oder-R²², mit R¹⁸, R¹⁹ und R²² jeweils in der unter R² angegebenen Bedeutung;
- R⁸: einen gerad- oder verzweigtkettigen, gegebenenfalls teilweise oder vollständig fluorierten Alkyl- oder Alkenylrest mit bis zu 5 Kohlenstoffatomen, einen Ethinyl- oder Prop-1-inylrest;
- R⁹: unabhängig voneinander ein Wasserstoffatom oder zusammen mit R¹¹ eine zusätzliche Bindung;
- R^{11'}: ein Wasserstoffatom, ein Fluor oder Chloratom, eine gesättigte, gerad- oder verzweigtkettige C₁-C₄-Alkylgruppe, eine Gruppe -X- R^{18'}, worin X ein Schwefelatom ist und R^{18'} eine C₁-C₃-Alkylgruppe;
- R¹², R¹⁴, R¹⁵, R^{15'} und R¹⁶: jeweils ein Wasserstoffatom;
- R^{16'}: ein Wasserstoffatom, ein Fluor- oder Chloratom oder eine Gruppe R¹⁸-O oder -R²², mit R¹⁸ und R²² jeweils in der unter R² angegebenen Bedeutung;
- R¹⁷ und R^{17'}: jeweils ein Wasserstoffatom; ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder -O-C(O)R²²; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R² angegebenen Bedeutung; oder
- R¹⁷ und R^{17'}: gemeinsam eine Gruppe =CR²³R²⁴, worin R²³ und R²⁴ unabhängig voneinander ein Wasserstoffatom und ein Halogenatom darstellen, oder gemeinsam ein Sauerstoffatom;
bedeuten:

Gemäß einer weiteren Variante kommen 8β-substituierte Estra-1,3,5(10)-trien-derivate der allgemeinen Formel I` zur Verwendung,
worin
R^{6'}, R^{7'}, R⁹, R¹¹, R¹⁴, R¹⁵, R^{15'} und R¹⁶ jeweils für ein Wasserstoffatom oder R^{6'}, R⁷', R¹⁴, R¹⁵, R^{15'} und R¹⁶ jeweils für ein Wasserstoffatom sowie R⁹ und R¹¹ zusammen für eine zusätzliche Bindung stehen und alle anderen Substituenten die in Anspruch 1 angegebenen Bedeutungen haben.

Wenn die Estratrien-derivate der allgemeinen Formel I' weitere Doppelbindungen im B-, C- und/oder D-Ring enthalten, dann ist vorzugsweise in der Position 9(11), 14(15) oder 15(16) eine Doppelbindung oder sind in den Positionen 9(11) und 14(15) bzw. 15(16) zwei Doppelbindungen vorhanden.

Eine weitere Variante der Erfindung sind Estratrien-derivate der allgemeinen Formel I'
worin
R¹⁷ und R^{17'} eine Gruppe R¹⁸-O- und eine Gruppe R¹⁸-; eine Gruppe R¹⁸- und eine Gruppe -O-C(O)R²², mit R¹⁸ und R²² jeweils in der unter R² angegebenen Bedeutung;
ist.

Von diesen letztgenannten sind wiederum solche Gonatrien-derivate bevorzugt
worin
R¹⁷ und R^{17'} eine Hydroxygruppe und ein Wasserstoffatom, eine C₁- C₄-Alkyl- oder C₂-C₄-Alkenylgruppe
ist
und insbesondere bevorzugt diejenigen
worin
R¹⁷ und R^{17'}eine Hydroxygruppeund ein Wasserstoffatom, eine Methyl-, Ethinyl- oder Prop-1-inylgruppe
ist.

Schließlich besteht eine Ausführungsform darin, daß R^{16'} für eine Gruppe R¹⁸-O- oder R¹⁹SO₂-O- mit R¹⁸ und R¹⁹ jeweils in der unter R² angegebenen Bedeutung steht, R¹⁷ und R¹⁷' je für ein Wasserstoffatom steht sowie alle anderen Substituenten die in der allgemeinen Formel I' angegebenen Bedeutungen haben können.

Bevorzugt gemäß vorliegender Erfindung ist die Verwendung einer oder mehrerer der folgenden Verbindungen
8β-Methyl-estra-1,3,5(10),9(11)-tetraen-3,17β-diol
3-Methoxy-8β-methyl-estra-1,3,5(10),9(11)-tetraen-17β-ol
8β-Methyl-estra-1,3,5(10)-trien-3,17β-diol
3-Methoxy-8β-methyl-estra-1,3,5(10)-trien-17β-ol
8β-Vinyl-estra-1,3,5(10),9(11)tetraen-3,17β-diol
3-Methoxy-8β-vinyl-estra-1,3,5(10),9(11)-tetraen-17β-ol
8β-(2',2'-Difluorvinyl)-estra-1,3,5(11),9(11)-tetraen-3,17β-diol
8β-(2',2'-Difluorvinyl)-3-methoxy-estra-1,3,5(10),9(11)-tetraen-17β-ol
8β-Vinyl-estra-1,3,5(10)trien-3,17β-diol
3-Methoxy-8β-vinyl-estra-1,3,5(10)-trien-17β-ol
8β-(2',2'-Difluorvinyl)-estra-1,3,5(10)-trien-3.17β-diol
8β-(2',2'-Difluorvinyl)-3-methoxy-estra-1,3,5(10)-trien-17β-ol
8β-Ethyl-estra-1,3,5(10)-trien-3,17β-diol
8β-Ethyl-3-methoxy-estra-1,3,5(10)-trien-17β-ol
8β-Vinyl-estradiol-3-sulfamat
8β-Vinyl-estradiol-3,17-disulfamat
8β-Vinyl-estradiol-3-(N-acetyl)sulfamat
8β-Vinyl-estron-3-sulfamat
8β-Vinyl-estron-3-acetat
8β-Vinyl-estriol
8β-Vinyl-estriol-3-sulfamat
8β-Methyl-estron-3-sulfamat
8β-Methyl-estriol
8β-(Prop-(Z)-enyl)-estradiol
8β-(n-Propyl)-estradiol
8β-Ethinyl-estradiol
17α-Ethinyl-8β-vinyl-estra-1,3,5(10)-trien-3,17β-diol
17α-Methyl-8β-vinyl-estra-1,3,5(10)-trien-3,17β-diol
16α-Fluor-8β-methyl-estra-1,3,5(10)-trien-3,17β-diol
8β-Vinyl-estra-1,3,5(10)-trien-3,17α-diol
8β-Methyl-estra-1,3,5(10)-trien-3,17α-diol
8β-Vinyl-estradiol-diacetat
8β-Methyl-estradiol-diacetat
8β-Vinyl-estradiol-17-valerianat
17β-Acetoxy-8β-vinyl-estra-1,3,5(10)-trien-3-ol

Weitere Ausgestaltungsmöglichkeiten der vorliegenden Erfindung ergeben sich aus den Unteransprüchen.

Neben der vorstehenden Verwendung der Verbindungen der allgemeinen Formel I' betrifft die Erfindung auch die Verbindungen der allgemeinen Formel I selbst. Das sind die Verbindungen der allgemeinen Formel l' aber worin R³ eine Gruppe R¹⁸-O-, R¹⁹-SO₂-O- oder O-C(O)R²² darstellt, mit R¹⁹ und R²² jeweils in der unter R² angegebenen Bedeutung, wobei für R¹⁸ ein Wasserstoffatom, eine Trifluoromethylgruppe, Isopropyl, Butyl, Isobutyl, Tert-butyl, Pentyl, Isopentyl, Neopentyl, Heptyl, Hexylrest oder ein Aryl-, Heteroaryl-, oder Aralkylrest stehen kann und ausgenommen der Verbindungen der allgemeinen Formel I', worin
R³ eine Hydroxy-, oder Acetyoxylgruppe ist, und gleichzeitig
R² ein Wasserstoffatom,
R⁶, R^{6'}, R⁷ und R^{7'} jeweils ein Wasserstoffatom:
R⁸ eine Methylgruppe,
R⁹ ein Wasserstoffatom oder
R⁹ und R¹¹ zusammen eine zusätzliche Bindung,
R^{11'} und R¹² jeweils ein Wasserstoffatom,
R¹⁴, R¹⁵, R^{15'}, R¹⁶ und R^{16'} jeweils ein Wasserstoffatom, sowie
R¹⁷ und R^{17'} für eine β-Hydroxygruppe und ein Wasserstoffatom: für eine β-(2-Bromacetyl)oxygruppe und ein Wasserstoffatom: für eine β-Acetylgruppe und ein Wasserstoffatom; oder
R¹⁷ und R^{17'} gemeinsam ein Sauerstoffatom darstellen.

Diese aus dem Umfang der allgemeinen Formel **I'** disclaimten Gruppe von Verbindungen ist bereits aus den folgenden Patent- und Literaturstellen bekannt:
Los, Marinus; US 3806546
Los. Marinus; US 3736345
Los, Marinus; US 3681407
Los, Marinus; US 3501530
Nagata, Wataru; Itazaki, Hiroshi; JP 45024573
Nagata, Wataru; Itazaki, Hiroshi; Takegawa, Bunichi; JP 45024139
Nagata, Wataru; Aoki, Tsutomu; Itazaki, Hiroshi; JP 45004060
Nagata, Wataru; Aoki, Tsutomu; Itazaki, Hiroshi; JP 45004059
Nagata, Wataru; Aoki, Tsutomu; Itazaki, Hiroshi; JP 45004058
Sakai, Klyoshi; Amemiya, Shigeo; Chem. Pharm. Bull. (1970), 18(3), 641-3
Yoshioka, Kouichi; Goto, Gilchi; Hiraga, Kentaro; Miki, Takuichi; Chem. Pharm.
Bull. (1973), 21(11), 2427-31
Tori, K; Editor(s): James, Vivian H. T; Horm. Steroids, Proc. Int. Congr., 3rd (1971), Meeting Date 1970, 205-13
Tsukuda, Yoshisuke; Sato, Tomohiro; Shiro, Motoo; Koyama, Hirozo; J. Chem. Soc. B (1969). (4), 336-41
Tsukuda, Yoshiko; Itazaki, Hiroshi; Nagata, Wataru; Sato, Tomohiro: Shiro,
Motoo; Koyama, Hirozo; Chem. Ind. (London) (1967), (48). 2047-8
Nakal, Hisayoshi; Koyama, Hirozo: Acta Crystallogr. (1967), 23(4). 674.

US 3806546, US 3681407 und JP 45004059 beschreiben die Bereitstellung estrogenaktiver Verbindungen, insbesondere 8β-methyl-3-methoxy-estra-1,3,5 (10)-trien Derivate.

Eine selektive estrogene Wirkung und die Verwendung der bekannten Verbindungen im Sinne vorliegender Erfindung ist bisher aber nicht beschrieben.

Die bereits bekannten Estratriene sind meist als Intermediate, als Estrogene im herkömmlichen Sinne oder zur Verwendung in analytischen Verfahren beschrieben.

In den Verbindungen der allgemeinen Formeln I und I' kann für ein Halogenatom immer ein Fluor-, Chlor-, Brom- oder lodatom stehen; ein Fluoratom ist jeweils bevorzugt. Für die 11β-Position ist insbesondere auch ein Chloratom als Substituent zu nennen. Insbesondere handelt es sich bei den Kohlenwasserstoffresten, die teilweise oder vollständig halogeniert sein können, um fluorierte Reste.

Der Kohlenwasserstoffrest R¹⁸ ist beispielsweise ein Methyl, Ethyl, Propyl, Isopropyl, Butyl, Isobutyl, tert.-Butyl, Pentyl, Isopentyl, Neopentyl, Heptyl, Hexylrest.

Die Alkoxygruppen OR¹⁸ in den Verbindungen der allgemeinen Formeln I und I' können jeweils 1 bis 6 Kohlenstoffatome enthalten, wobei Methoxy-, Ethoxy- Propoxy- Isopropoxy- und t-Butyloxygruppen bevorzugt sind.

Vertreter für die C₁-C₅-Alkylreste R²⁰ und R²¹ sind Methyl, Ethyl, Propyl, Isopropyl, Butyl, Isobutyl, tert.-Butyl, Pentyl, Isopentyl, Neopentyl.

Als Vertreter für gerad- oder verzweigtkettige Kohlenwasserstoffreste R²² mit 1 bis max. 10 Kohlenstoffatomen sind beispielsweise Methyl, Ethyl, Propyl, Isopropyl, Butyl, Isobutyl, tert.-Butyl, Pentyl, Isopentyl, Neopentyl, Heptyl, Hexyl und Decyl zu nennen; Methyl, Ethyl, Propyl und Isopropyl sind bevorzugt.

Als perfluorierte Alkylgruppen seien beispielsweise Trifluormethyl, Pentafluorethyl und Nonafluorbutyl genannt. Vertreter der teilweise fluorierten Alkylgruppen sind zum Beispiel 2,2,2-Trifluorethyl, 5,5,5,4,4-Pentafluorpentyl, 6,6,6,5,5,4,4,3,3-Nonafluorhexyl etc..

Als C₃-C₇-Cycloalkylgruppe ist eine Cyclopropyl-, butyl-, pentyl-, hexyl- oder heptylgruppe zu nennen

Ein C₄-C₁₅-Cycloalkylalkylrest weist 3 bis 7 Kohlenstoffatome im Cycloalkylteil auf; typische Vertreter sind die direkt vorstehend genannten Cycloalkylgruppen. Der Alkylteil weist bis zu 8 Kohlenstoffatome auf.
Als Beispiele für einen C₄-C₁₅-Cycloalkylalkylrest seien die Cyclopropylmethyl-, Cyclopropylethyl-, Cyclopentylmethyl-, Cyclopentylpropylgruppe etc. genannt.

Beim einem Arylrest handelt es sich im Sinne der vorliegenden Erfindung um einen Phenyl-, 1- oder 2-Naphthylrest; der Phenylrest ist bevorzugt.

Aryl schließt immer auch einen Heteroarylrest mit ein. Beispiele für einen Heteroarylrest sind der 2-, 3- oder 4-Pyridinyl-, der 2- oder 3-Furyl-, der 2- oder 3-Thienyl-, der 2- oder 3-Pyrrolyl, der 2-, 4- oder 5-Imidazolyl-, der Pyrazinyl-, der 2-, 4- oder 5-Pyrimidinyl- oder 3- oder 4-Pyridazinylrest.

Als Substituenten für einen Aryl- oder Heteroarylrest seien zum Beispiel ein Methyl-, Ethyl-, Trifluormethyl- Pentafluorethyl-, Trifluormethylthio-, Methoxy-, Ethoxy-, Nitro-, Cyano-, Halogen- (Fluor, Chlor, Brom, lod), Hydroxy-, Amino-, Mono(C₁₋₈-alkyl)- oder Di(C₁₋₈-alkyl)amino, wobei beide Alkylgruppen identisch oder verschieden sind, Di(aralkyl)amino, wobei beide Aralkylgruppen identisch oder verschieden sind, erwähnt.

Bei einem Aralkylrest handelt es sich um einen Rest, der im Ring bis 14, bevorzugt 6 bis 10, C-Atome und in der Alkylkette 1 bis 8, bevorzugt 1 bis 4, C-Atome enthält. So kommen als Aralkylreste beispielsweise in Betracht Benzyl, Phenylethyl, Naphthylmethyl, Naphthylethyl, Furylmethyl, Thienylethyl, Pyridylpropyl. Die Ringe können einfach oder mehrfach substituiert sein durch Halogen, OH, O-Alkyl, CO₂H, CO₂-Alky -NO₂, -N₃, -CN, C₁-C₂₀-Alkyl, C₁-C₂₀-Acyl, C₁-C₂₀-Acyloxy-Gruppen.

Die Alkylgruppen bzw. Kohlenwasserstoffreste können teilweise oder vollständig fluoriert oder substituiert sein durch 1-5 Halogenatome, Hydroxygruppen oder C₁-C₄-Alkoxygruppen.

Mit einem C₂-C₅-Alkenylrest ist in erster Linie ein Vinyl- oder Allylrest gemeint.

Weitere Varianten der Erfindung sehen eine oder mehrere, gegebenenfalls konjugierte, Doppelbindurigen in den Ringen B, C und D des Estratrien-Gerüsts vor, und zwar eine oder mehrere Doppelbindungen in den Positionen 6, 7; 9, 11; 11, 12; 14, 15 sowie 15, 16. Bevorzugt ist dabei eine Doppelbindung in der Position 11, 12.

Eine oder mehrere Hydroxylgruppen an den C-Atomen 3, 16 und 17 können mit einer aliphatischen, gerad- oder verzweigtkettigen, gesättigten oder ungesättigten C₁-C₁₄-Mono- oder Polycarbonsäure oder einer aromatischen Carbonsäure oder mit einer α-oder β-Aminosäure verestert sein.
Als derartige Carbonsäuren zur Veresterung kommen beispielsweise in Betracht:
Monocarbonsäuren: Ameisensäure, Essigsäure, Propionsäure, Buttersäure,
Isobuttersäure, Valeriansäure, Isovaleriansäure, Pivalinsäure, Laurinsäure,
Myristinsäure, Acrylsäure, Propiolsäure, Methacrylsäure, Crotonsäure,
Isocrotonsäure, Ölsäure, Elaidinsäure.
Die Veresterung mit Essigsäure, Valeriansäure oder Pivalinsäure ist bevorzugt.
Dicarbonsäuren: Oxalsäure, Malonsäure, Bernsteinsäure, Glutarsäure, Adipinsäure,
Pimelinsäüre, Korksäure, Azelainsäure, Sebacinsäure, Maleinsäure, Fumarsäure,
Muconsäure, Citraconsäure, Mesaconsäure.
Aromatische Carbonsäuren: Benzoesäure, Phthalsäure, Isophthalsäure,
Terephthalsäure, Naphthoesäure, o-, m- und p-Toluylsäure, Hydratropasäure,
Atropasäure, Zimtsäure, Nicotinsäure, Isonicotinsäure.
Die Veresterung mit Benzoesäure ist bevorzugt.
Als Aminosäuren kommen die dem Fachmann hinlänglich bekannten Vertreter dieser
Substanzklasse in Frage, beispielsweise Alanin, β-Alanin, Arginin, Cystein, Cystin,
Glycin, Histidin, Leucin, Isoleucin, Phenylalanin, Prolin etc..
Die Veresterung mit β-Alanin ist bevorzugt.

Bevorzugt gemäß vorliegender Erfindung sind die nachstehenden Verbindungen:
8β-Vinyl-estra-1,3 5(10),9(11)-tetraen-3,17β-diol
3-Methoxy-8β-vinyl-estra-1,3,5(10), 9(11)-tetraen-17β-ol
8β-(2,2'-Difluorvinyl)-estra-1,3,5(10),9(11)-tetraen-3,17β-diol
8β-(2',2'-Difluorovinyl)-3-methoxy-estra-1,3,5(10)-tetraen-17β-ol
8β-Vinyl-estra-1,3,5(10)-trien-3,17β-diol
3-Methoxy-8β-vinyl-estra-1,3,5(10)trien-17β-ol
8β-(2',2'-Difluorvinyl)-estra-1,3,5(10)-trien-3,17β-diol
8β-(2',2'-Difluorvinyl)-3-methoxy-estra-1,3,5(10)-trien-17β-ol
8β-Ethyl-estra-1,3.5(10)-trien-3,17β-diol
8β-Ethyl-3-methoxy-estra-1,3,5(10)-trien-17β-ol
8β-Vinyl-estradiol-3-sulfamat
8β-Vinyi-estradioi-3,17-disuifamat
8β-Vinyl-estradiol-3-(N-acetyl)-sulfamat
8β-Vinyl-estron-3-sulfamat
8β-Vinyl-estron-3-acetat
8β-Vinyl-estriol
8β-Vinyl-estriol-3-sulfamat
8β-Methyl-estron-3-sulfamat
8β-Methyl-estriol
8β-(Prop-(Z)-enyl)-estradiol
8β-(n-Propyl)-estradiol
8β-Ethinyl-estradiol
17α-Ethinyl-8β-vinyl-estra-1.3,5(10)-trien,3,17β-diol
17α-Methyl-8β-vinyl-estra-1,3,5(10)-trien-3,17β-diol
16α-Fluor-8β-methyl-estra-1,3,5(10)-trien-3,17β-diol
8β-Vinyl-estra-1,3,5(10)-trien-3,17α-diol
8β-Methyl-estra-1,3,5(10)-trien-3,17α-diol
8β-Vinyl-estradiol-diacetat
8β-Methyl-estradiol-diacetat
8β-Vinyl-estradiol-17-valerianat
17β-Acetoxy-8β-vinyl-estra-1,3,5(10)-trien-3-ol

Die erfindungsgemäßen Ester der 8β-substituierten Estratriene weisen als Prodrugs Vorteile gegenüber den unveresterten Wirkstoffen hinsichtlich ihres Applikationsmodus, ihrer Wirkungsart, Wirkungsstärke und Wirkungsdauer auf.

Pharmakokinetische und pharmakodynamische Vorteile weisen auch die erfindungsgemäßen Sulfamate der 8β-substituierten Estratriene auf. Diesbezügliche Effekte wurden bereits bei anderen Steroid-Sulfamaten beschrieben (J. Steroid Biochem. Molec. Biol, 55, 395 - 403 (1995); Exp. Opinion Invest. Drugs 7, 575 - 589 (1998)).

In der vorliegenden Patentanmeldung werden Steroide, denen das 8β-substituierte Estra-1,3,5(10)trien-Gerüst zugrunde liegt, zur Behandlung von Estrogenrezeptor β-vermittelten Krankheiten und Zuständen als selektive Estrogene beschrieben, die in vitro Dissoziation hinsichtlich Bindung an Estrogenrezeptorpräparationen von Rattenprostata und Rattenuterus und die in vivo vorzugsweise eine Dissoziation beispielsweise hinsichtlich Knochen- im Vergleich zu Uteruswirkung aufweisen: über einen breiten Dosisbereich wirken diese Substanzen knochenprotektiv ohne den Uterus zu stimulieren.

Weiterhin können die Substanzen bei der männlichen Ratte protektive Wirkung gegen Orchiektomie-induzierten Knochenmasseverlust aufweisen, ohne die Sekretion der Hypophysenhormone LH und FSH zu hemmen. Im gleichen Dosisbereich ist ihre Leberwirkung gering.

Die Substanzen üben außerdem estrogenartige Wirkung auf das Gefäßsystem und Gehimfunktionen aus. Substanzen mit höherer Bindung an den Rattenprostataverglichen mit dem Rattenuterus-Estrogenrezeptor sind potenter hinsichtlich Erhöhung der Expression von Serotoninrezeptor und -transporter, im Vergleich zu ihrem positiven Effekt auf die LH-Ausschüttung. Daher werden Prozesse, an deren Regulation der Neurotransmitter Serotonin beteiligt ist, günstig beeinflußt und die erfindungsgemäßen Verbindungen üben insbesondere auf die Stimmung und Kognition einen günstigen Einfluß aus.

Sie können als Estrogene in dem in der WO 97/45125 beschriebenen Sinne für die Herstellung von Medikamenten zur Beeinflußung der Spiegel von Serotonin bzw. von Serotonin mRNA beim Menschen verwendet werden.

Es wurde gefunden, daß die erfindungsgemäßen 8β-substituierten Estra-1,3,5(10)triene als selektive Estrogene zur Behandlung verschiedener Zustände und Krankheiten, die durch einen höheren Gehalt an Estrogenrezeptor β als Estrogenrezeptor α im entsprechenden Zielgewebe oder-organ gekennzeichnet sind, geeignet sind.

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung der Verbindungen der allgemeinen Formel I' zur Herstellung von Arzneimitteln, insbesondere für die nachstehenden Indikationen.

Die Verbindungen können, sowohl nach oraler als auch parenteraler Gabe, für die folgenden Indikationen eingesetzt werden.

Die im vorliegenden Patent beschriebenen neuartigen selektiven Estrogene können als Einzelkomponente in pharmazeutischen Zubereitungen oder in Kombination insbesondere mit Antiestrogenen oder Gestagenen eingesetzt werden. Besonders bevorzugt ist die Kombination der selektiven Estrogene mit ERα-selektiven Antiestrogenen, oder mit Antiestrogenen, die peripherselektiv wirksam sind, d.h. die die Bluthirnschranke nicht passieren.

Die Substanzen und die sie enthaltenden Pharmaka sind besonders geeignet für die Behandlung peri- und postmenopausaler Beschwerden insbesondere Hitzewallungen, Schlafstörungen, Reizbarkeit, Stimmungsschwankungen, Inkontinenz, Vaginalatrophie, hormondefizienzbedingte Gemütserkrankungen. Ebenso sind die Substanzen für die Hormonsubstitution und die Therapie von hormondefizienz bedingten Beschwerden bei chirurgisch, medikamentös oder anders bedingter ovarieller Dysfunktion geeignet. Hierzu gehört auch die Vorbeugung gegen den Knochenmasseverlust bei postmenopausalen Frauen und andropausalen Männern, bei hysterektomierten Frauen oder bei Frauen, die mit LHRH-Agonisten oder Antagonisten behandelt wurden.

Die Verbindungen sind auch zur Linderung der Symptome der Andropause und Menopause, d.h. zur männlichen und weiblichen Hormonersatz-Therapie (HRT), und zwar sowohl zur Prävention als auch zur Behandlung, weiterhin zur Behandlung der mit einer Dysmenorrhoe einhergehenden Beschwerden sowie zur Behandlung der Akne geeignet.

Die Substanzen sind außerdem zur Prophylaxe gegen hormondefizienzbedingten Knochenmasseverlust und Osteoporose, zur Vorbeugung gegen Herzkreislauferkrankungen, insbesondere Gefäßerkrankungen wie Atherosklerose, zur Hemmung der Proliferation der arteriellen Glattmuskelzellen, zur Behandlung des primären pulmonaren Bluthochdrucks und zur Vorbeugung gegen hormondefizienzbedingte neurodegenerative Erkrankungen, wie Alzheimersche Krankheit, sowie hormondefizienzbedingte Beeinträchtigung von Gedächtnis- und Lernfähigkeit, einsetzbar.

Weiterhin sind die Substanzen zur Behandlung von entzündlichen und Erkrankungen des Immunsystems, insbesondere Autoimmunerkrankungen, wie z.B. Rheumatoide Arthritis, einsetzbar.

Außerdem können die Verbindungen zur Behandlung männlicher Fertilitätsstörungen und prostatischer Erkrankungen Verwendung finden..

Die Verbindungen können auch in Kombination mit dem natürlichen Vitamin D3 oder mit Calcitriol-Analoga für den Knochenaufbau oder als unterstützende Therapie zu Therapien, welche einen Knochenmassenverlust verursachen (beispielsweise eine Therapie mit Glucocorticoiden, Chemotherapie) eingesetzt werden.

Schließlich können die Verbindungen der allgemeinen Formel I' in Verbindung mit Progesteronrezeptor-Antagonisten verwendet werden, und zwar insbesondere zur Verwendung in der Hormonersatz-Therapie und zur Behandlung gynäkologischer Störungen.

Ein therapeutisches Produkt, enthaltend ein Estrogen und ein reines Antiestrogen für gleichzeitige, sequentielle oder getrennte Anwendung für die selektive Estrogentherapie perimenopausaler oder postmenopausaler Zustände ist bereits in der EP-A 0 346 014 beschrieben.

Die zu verabreichende Menge einer Verbindung der allgemeinen Formel I' schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 µg/kg -10 mg/kg Körpergewicht, vorzugsweise 0,04 µg/kg -1 mg/kg Körpergewicht, je Tag betragen. Beim Menschen entspricht dies einer Dosis von 0,8 µg bis 800 mg, vorzugsweise 3,2 µg bis 80 mg, täglich.

Eine Dosiseinheit enthält erfindungsgemäß 1,6 µg bis 200 mg einer oder mehrerer Verbindungen der allgemeinen Formel l'.

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2, 1961, Seite 72 u. ff.: Dr. H. P. Fiedler, Lexikon der Hilfsstoffe für Pharmazie, Kosmetik und angrenzende Gebiete, Cantor KG. Aulendorf in Württemberg 1971.

Die Verbindungen können oral oder parenteral, beispielsweise intraperitoneal, intramuskulär, subkutan oder perkutan verabreicht werden. Die Verbindungen können auch in das Gewebe implantiert werden.
Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.
Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.
Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel I' beladenen Intravaginal- (z.B. Vaginalringe) oder Intrauterinsystemen (z.B. Pessare, Spiralen, IUSs, Mirena^{®}) für die lokale Verabreichung eignen sich verschiedene Polymere wie zum Beispiel Silikonpolymere, Ethylenvinylacetat, Polyethylen oder Polypropylen.

Um eine bessere Bioverfügbarkeit des Wirkstoffes zu erreichen, können die Verbindungen auch als Cyclodextrinclathrate formuliert werden. Hierzu werden die Verbindungen mit α-, β- oder γ-Cyclodextrin oder Derivaten von diesen umgesetzt (PCT/EP95/02656).

Erfindungsgemäß können die Verbindungen der allgemeinen Formel l' auch mit Liposomen verkapselt werden.

### Methoden

### Estrogenrezeptorbindungsstudien

Die Bindungsaffinität der neuen selektiven Estrogene wurde in Kompetitionsexperimenten unter Verwendung von 3H-Estradiol als Ligand an Estrogenrezeptorpräparationen von Rattenprostata und Rattenuterus getestet. Die Präparation des Prostatacytosols und der Estrogenrezeptortest mit dem Prostatacytosol wurde, wie von Testas et al. (1981) beschrieben, durchgeführt (Testas J. et al., 1981, Endocrinology 109: 1287-1289).

Die Präparation von Rattenuteruscytosol, sowie der Rezeptortest mit dem ERhaltigen Cytosol wurden prinzipiell durchgeführt wie von Stack und Gorski, 1985, beschrieben (Stack, Gorski 1985, Endocrinology 117, 2024-2032) mit einigen Modifikationen wie bei Fuhrmann et al. (1995) beschrieben (Fuhrmann U. et al. 1995, Contraception 51: 45-52).

Die im vorliegenden Patent beschriebenen Substanzen weisen höhere Bindungsaffinität zu Estrogenrezeptor aus Rattenprostata als zu Estrogenrezeptor aus Rattenuterus auf. Dabei wird davon ausgegangen, daß ERβ gegenüber ERα in der Rattenprostata, in Rattenuterus ERα gegenüber ERβ überwiegt. Tabelle 1 zeigt, daß das Verhältnis der Bindung an Prostata- und Uterusrezeptor qualitativ mit dem Quotient der relativen Bindungsaffinität (RBA) an humanen ERβ und ERα von Ratte (nach Kuiper et al. (1996), Endocrinology 138: 863-870) übereinstimmt (Tabelle 1).

**Tabelle 1**

| **Estrogen** | **Struktur** | **hER α RBA*** | **hER β R8A*** | **ERß/ ERα** | **Rat uterus ER(RBA)** | **Rat prost. ER(RBA)** | **prost.ER/ uterusER** |
|---|---|---|---|---|---|---|---|
| **Estradiol** | 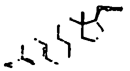 | 100 | 100 | 1 | 100 | 100 | 1 |
| **Estron** | 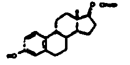 | 60 | 37 | 0.6 | 3 | 2 | 0.8 |
| **17α-Estradiol** | 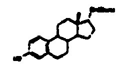 | 58 | 11 | 0.2 | 2.4 | 1.3 | 0.5 |
| **Estriol** | 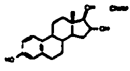 | 14 | 21 | 1.5 | 4 | 20 | 5 |
| **5-Androsten -diol** | 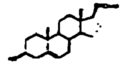 | 6 | 17 | 3 | 0.1 | 5 | 50 |
| **Genistein** | 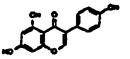 | 5 | 36 | 7 | 0.1 | 10 | 100 |
| **Coumestrol** | 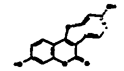 | 94 | 185 | 2 | 1.3 | 24 | 18 |

| | | | | | | | |
|---|---|---|---|---|---|---|---|
| *: zitiert aus: Kuiper et al. (1996), Endocrinology 138: 863-870 | | | | | | | |

**Tabelle 2 zeigt die Ergebnisse für die erfindungsgemäß zu verwendende Verbindung 8β-Methyl-estra-1,3,5(10)-trien-3,17β-diol (Verbindung D)**
sowie für die erfindungsgemäßen Verbindungen
8β-Vinyl-estra-1,3,5(10),9(11)-tetraen-3,17β-diol (A)
8β-Vinyl-estra-1,3,5(10)-trien-3,17β-diol (B)
8β-(2,2-Difluorvinyl)-estra-1,3,5(10)-trien-3,17β-diol (C) und
8β-Ethyl-estra-1,3,5(10)-trien-3,17β-diol (E).

**Tabelle 2**

| **Verbindung** | **RBA Rattenuterus** | **RBA Rattenprostata** |
|---|---|---|
| 8β-Vinyl-estra-1,3,5(10),9(11)-tetraen-3,17β-diol (A) | 1 | 83 |
| 8β-Vinyl-estra-1,3.5(10)-trien-3,17β-diol (B) | 0.7 | 63 |
| 8β-(2,2-Difluorvinyl)-estra-1,3,5(10)-trien-3,17β-diol (C) | 0.9 | 5 |
| 8β-Methyl-estra-1,3,5(10)-trien-3,17β-diol (D) | 1.3 | 67 |
| 8β-Ethyl-estra-1,3,5(10)-trien-3,17β-diol (E) | <0.3 | 7 |

Die Verbindungen A, B, C, D und E zeigen eine höhere Bindungsaffinität am Estrogenrezeptor aus Rattenprostata als am Estrogenrezeptor aus Rattenuterus.

Weiterhin wurde die Prädiktivität des 'Prostata-ER versus Uterus-ER-Testssystems' hinsichtlich gewebeselektiver Wirkung durch in vivo Untersuchungen bestätigt. Substanzen mit Präferenz für Prostata-ER sind in vivo vorzugsweise hinsichtlich Knochen- und Uteruswirkung zugunsten der Wirkung am Knochen dissoziiert. Substanzen mit höherer Bindung an den Rattenprostata- verglichen mit dem Rattenuterus-Estrogenrezeptor sind außerdem potenter hinsichtlich Erhöhung der Expression von Serotoninrezeptor und -transporter, im Vergleich zu ihrem positiven Effekt auf die LH-Ausschüttung.

### Knochenuntersuchungen

3 Monate alte weibliche Ratten werden ovarektomiert und unmittelbar nach der Operation 28 Tage lang 1 mal täglich mit der Testverbindung behandelt. Die Applikation erfolgt subcutan in Arachisöl/Ethanol. Die Tiere werden am Tag nach der letzten Applikation getötet und Tibia sowie die Uteri entnommen. Die Uteri werden gewogen, fixiert und für histologische Untersuchungen aufgearbeitet. Die Bestimmung der Knochendichte erfolgt ex vivo an präparierten Langknochen mittels pQCT (Quantitative Computertomographie). Die Messungen werden im Abstand von 4-6 mm vom Gelenkkopf der proximalen Tibia durchgeführt. Durch die Ovarektomie vermindert sich die Dichte des trabekulären Knochens im gemessenen Bereich von ca. 400 mg Ca²⁺/cm³ auf ca. 300 mg Ca²⁺/cm³. Durch die Behandlung mit einer Verbindung der allgemeinen Formel I gemäß vorliegender Erfindung wird der Abbau der Knochendichte verhindert bzw. gehemmt. Gemessen wurde die Knochendichte an der proximalen Tibia.

In vivo spiegelt sich die höhere Bindungsaffinität zu Estrogenrezeptor aus Rattenprostata als zu Estrogenrezeptor aus Rattenuterus vorzugsweise in deutlich niedrigeren Mengen der erindungsgemäßen Verbindungen wider, die eine 50%ige Knochenprotektion bewirken im Vergleich zu den Mengen, die eine 50%ige Uterusstimulation bewirken, bezogen auf den Knochenmasseverfust, der in ovarektomierten, unbehandelten weiblichen Ratten 28 Tage nach der Ovarektomie im Unterschied zu sham-oprierten, intakten Tieren meßbar ist.

Die Gefäßwirkung der erfindungsgemäßen Estrogene wird im Modell der ApoE-Knockout-Maus, wie von R. Elhage et al., 1997, beschrieben, sowie im Modell der ballonkatheterinduzierten Gefäßschädigung (Restenosemodell) ermittelt (Elhage R. et al. 1997, Arteriosclerosis, Thrombosis, and Vascular Biology 17: 2679-2684).

Zum Nachweis der Wirkung von Estrogenen auf die Gehimfunktion wird die Oxytocin-, Oxytozinrezeptor- oder Vasopressin-mRNA-Expression als Surrogatparameter verwendet (Hrabovszky E et al. 1998, Endocrinology 1339: 2600-2604). Ovariektomierte Ratten werden über 7 Tage mit der Testsubstanz oder Vehikel behandelt (Applikation: subkutan oder oral, 6-mal täglich). Am Tag 7 nach der ersten Applikation werden die Tiere dekapitiert, das Uterusgewicht wird bestimmt und der Oxytocin-, Oxytozinrezeptor- oder Vasopressin-mRNA Spiegel wird mittels in situ Hybridisierung an geeigneten Gehirnschnitten untersucht. Es werden die ED₅₀-Werte hinsichtlich Stimulierung von Utersuswachstum und Induktion der Oxytozinrezeptor mRNA bestimmt.

Eine andere Möglichkeit, die dissoziierte Estrogenwirkung der erfindungsgemäßen Substanzen in vivo nachzuweisen, besteht darin, nach Einmalapplikation der Substanzen bei Ratten Effekte auf die Expression von 5HT2a-Rezeptor- und Serotonintransporter-Protein- und mRNA-Level in ERß-reichen Gehimarealen zuvermessen. Vergleichend zum Effekt auf Serotoninrezeptor- und Transporterexpression wird der Effekt auf die LH-Sekretion gemessen. Substanzen mit höherer Bindung an den Rattenprosta- verglichen mit dem Rattenuterusestrogenrezeptor sind potenter hinsichtlich Erhöhung der Expression von Serotoninrezeptor- und transporter, im Vergleich zu ihrem positiven Effekt auf die LH-Ausschüttung. Die Dichte von Serotoninrezeptor und -Transporter wird an Gehirnschnitten mittels radioaktiver Liganden, die entsprechende mRNA mittels in situ Hybridisierung bestimmt. Die Methode ist in der Literatur beschrieben: G. Fink & B.E.H. Sumner 1996 Nature 383:306; B. E.H. Sumner et al. 1999 Molecular Brain Research, in press.

In Übereinstimmung mit ihrer stärkeren Bindung an den Rattenprostata- im Vergleich zum Rattenuterus-Estrogenrezeptor führen die erfindungsgemäßen Substanzen A, B, C, D und E zu einer erhöhten Expression des Serotoninrezeptors undtransporters.

### Herstellung der erfindungsgemäßen Verbindungen

Die erfindungsgemäßen Verbindungen der allgemeinen Formel I (bzw. I') werden wie in den Beispielen beschrieben hergestellt. Durch analoge Vorgehensweise unter Verwendung homologer Reagenzien zu den in den Beispielen beschriebenen Reagenzien lassen sich weitere Verbindungen der allgemeinen Formel I' erhalten.

Veretherung und/oder Veresterung freier Hydroxygruppen erfolgt nach dem Fachmann gängigen Methoden.

Die erfindungsgemäßen Verbindungen können an den Kohlenstoffatomen 6, 7, 11, 15, 16 und 17 als α,β-Stereoisomere vorliegen. Bei der Herstellung der Verbindungen gemäß den beschriebenen Verfahren fallen die Verbindungen meist als Gemische der entsprechenden α,β-Isomeren an. Die Gemische lassen sich beispielsweise durch chromatographische Verfahren trennen.

Gemäß der allgemeinen Formel I mögliche Substituenten können bereits in der endgültigen Form oder in Form eines Vorläufers schon im Ausgangsprodukt, einem bereits dem gewünschten Endprodukt entsprechend substituierten Estron, vorhanden sein.

So ist die Einführung eines Substituenten bzw. reaktiven Vorläufers am Kohlenstoffatom 7 durch nukleophile Addition des Substituenten bzw. Vorläufers an ein 6-Vinylsulfon möglich (DE 42 18 743 A1). Hierbei werden in unterschiedlichen Anteilen, abhängig von den Reaktionspartnern und den gewählten Reaktionsbedingungen, 7α- und 7β-substituierte Verbindungen erhalten, die sich beispielsweise durch chromatographische Verfahren trennen lassen.

17-Substituenten werden, ebenfalls nach bekannten Verfahren, durch nukleophile Addition des gewünschten Substituenten oder eines reaktiven Vorläufers davon, eingeführt und gegebebenenfalls weiter aufgebaut.

Die erfindungsgemäßen 8β-substituierten Estratrien-Carbonsäureester werden in Analogie zu ebenfalls bekannten Verfahren aus den entsprechenden Hydroxysteroiden hergestellt (siehe z.B. Pharmazeutische Wirkstoffe, Synthesen, Patente, Anwendungen; A. Kleemann, J. Engel', Georg Thieme Verlag Stuttgart 1978. Arzneimittel, Fortschritte 1972 bis 1985; A. Kleemann, E. Lindner, J. Engel (Hrsg.), VCH 1987, S. 773-814).

Die erfindungsgemäßen Estratrien-Sulfamate sind in an sich bekannter Weise aus den entsprechenden Hydroxy-Steroiden durch Veresterung mit Sulfamoylchloriden in Gegenwart einer Base zugänglich (Z. Chem. 15, 270-272 (1975); Steroids 61, 710 - 717 (1996)).
Nachfolgende Acylierung der Sulfamidgruppe führt zu den erfindungsgemäßen (N-Acyl)sulfamaten, für die bereits im Falle der Abwesenheit eines 8-Substituenten pharmakokinetische Vorteile nachgewiesen wurden (vgl. DE 195 40 233 A1).

Die regioselektive Veresterung von polyhydroxylierten Steroiden mit N-substituierten und N-unsubstituierten Sulfamoylchloriden erfolgt nach partiellem Schutz derjenigen Hydroxylgruppen, die unverestert bleiben sollen. Als Schutzgruppen mit hierfür geeigneter selektiver Reaktivität haben sich Silylether erwiesen, da diese unter den Bedingungen der Sulfamatbildung stabil sind und die Sulfamatgruppe intakt bleibt, wenn die Silylether zur Regenerierung der restlichen im Molekül noch enthaltenen Hydroxylgruppe(n) wieder abgespalten werden (Steroids 61, 710 - 717 (1996)). Die Herstellung der erfindungsgemäßen Sulfamate mit einer oder mehreren zusätzlichen Hydroxylgruppen im Molekül ist auch dadurch möglich, daß man von geeigneten Hydroxy-Steroidketonen ausgeht. Zunächst werden, je nach Zielstellung, eine oder mehrere vorhandene Hydroxylgruppen einer Sulfamoylierung unterworfen. Dann können die Sulfamatgrupen gegebenenfalls mit einem gewünschten Acylchlorid in Gegenwart einer Base in die betreffenden /N-Acyl)sulfamate überführt werden. Die nunmehr vorliegenden Oxosulfamate oder Oxo-(N-acyl)sulfamate werden durch Reduktion in die entsprechenden Hydroxysulfamate bzw. Hydroxy-(N-acyl)sulfamate umgewandelt (Steroids 61, 710 - 717 (1996)). Als geeignete Reduktionsmittel kommen Natriumborhydrid und der Boran-Dimethylsulfid-Komplex in Frage.

Funktionalisierungen am Kohlenstoffatom 2 sind beispielsweise durch elektrophile Substitution nach vorheriger Deprotonierung der Position 2 des entsprechenden 3-(2-Tetrahydropyranyl)- oder 3-Methylethers mit einer Lithium-Base (z. B. Methyllithium, Butyllithium) möglich. So kann zum Beispiel ein Fluoratom durch Umsetzung des C-H-aktivierten Substrats mit einem Flourierungsreagenz wie N-Fluormethansulfonimid (WO 94/24098) eingeführt werden.

Die Einführung variabler Substituenten in die Ringe B, C und D des Estratriengerüstes kann prinzipiell nach der dem Fachmann bekannten chemischen Lehre erfolgen, mit der die entsprechenden, in 8-Stellung nicht substituierten Estratrienderivate hergestellt werden (siehe unter anderem: Steroide, L.F. Fieser, M. Fieser, Verlag Chemie, Weinheim / Bergstr., 1961; Organic Reactions in Steroid Chemistry, J. Fried, J.A. Edwards, Van Nostrand Reinhold Company, New York, Cincinnati, Toronto, London, Melbourne, 1972; Medicinal Chemistry of Steroids, F.J. Zeelen, Elsevier, Amsterdam, Oxford, New York, Tokyo, 1990). Das betrifft beispielsweise die Einführung von Substituenten, wie Hydroxyl- oder Alkyloxygruppen, Alkyl, Alkenyl- oder Alkinylgruppen oder Halogen, insbesondere Fluor.

Substituenten gemäß der allgemeinen Formel I können aber auch auf der Stufe der bereits in 8-Stellung substituierten Estratriene eingeführt werden. Dies kann insbesondere bei Mehrfachsubstitution der gewünschten Endverbindung sinnvoll bzw. erforderlich sein.

Die nachfolgenden Beispiele dienen der näheren Erläuterung der Erfindung.

Die allgemeinen Syntheserouten für diese Beispiele sind in den Schemata 1 bis 3 gezeigt.

Als Ausgangsmaterial für derartige Synthesen dienen 11-Keto-estratetraenderivate der Art **1** bzw. **2** (US 3491089, Tetrahedron Letters, 1967, 37, 3603.), welche bei der Umsetzung mit Diethylaluminiumcyanid stereoselektiv in Position 8β substitiuiert werden. Durch anschließende Reduktion der Carbonylfunktion an C(11) und Eliminieren der entstandenen Hydroxylgruppe gelangt man zu 8β-substituierten Estra-1,3,5(10),9(11)-tetraenen, die wiederum in 8β-Aldehyde überführbar sind. Eine Funktionalisierung, z.B. durch Wittig-Reaktionen mit nachfolgendem Entfernen der Schutzgruppen, führt zu den erfindungsgemäßen 8β-Sterolden.

Die bei dieser Sequenz zunächst erhaltenen 11-oxygenierten Estradiolderivate lassen sich, wie auch die Doppelbindung C(9)-C(11), nach dem Fachmann bekannten Methoden weiter zu vielfältigen Substitutionsmustern am Steroid umsetzen. Beispielsweise kann eine 11α-Hydroxygruppe nach dem von Vorbrüggen et al. beschriebenen Verfahren in ein 11β-Fluoratom überführt werden.

Für die Herstellung der erfindungsgemäßen Derivate der 8β-substituierten Estra-1,3,5(10)-trien-3,16ξ-diole ohne 17-Substituenten findet vor allem die folgende Synthesestrategie Verwendung. Hierbei wird die 8β-Carbonylfunktion als Acetal geschützt. Nach anschließender Oxidation, kann das 17-Ketosteroid in ein Sulfonylhydrazon überführt werden, im einfachsten Falle durch Umsetzung mit Phenylsulfonylhydrazid. Durch eine Abbaureaktion erfolgt die Bildung des C(16)-C(17) Olefins (Z. Chem. 1970, 10, 221-2; Liebigs Ann. Chem. 1981, 1973-81), an das in regio/stereokontrollierter Weise Hypobromid angelagert wird. Reduktive Dehalogenierung und Entfernung der Acetalschutzgruppe an 8β geben den Weg für Transformationen zu den erfindungsgemäßen Verbindungen frei. Die nach dieser Art erhältlichen. 16β-Alkohole können durch bekannte Methoden in das 16α-Epimer überführt werden (Synthesis 1980, 1)

Eine weitere Variante für die Einführung der Hydroxylgruppe an C-Atom 16 besteht in der Hydroborierung der 16(17)-Doppelbindung mit sterisch anspruchsvollen Boranen. Von dieser Reaktion ist bekannt, daß sie zu 16-oxygenierten Produkten führt (Indian J. Chem. 1971, 9, 287-8). Dementsprechend ergibt die Umsetzung der Estra-1,3,5(10).16-tetraene **17** mit 9-Borabicyclo[3.3.1]nonan nach der Oxidation mit alkalischem Wasserstoffperoxid 16α-Hydroxyestratriene. In untergeordnetem Maße werden bei dieser Reaktion die epimeren 16β-Hydroxysteroide gebildet. Weitere Transformationen am 8β -Substituenten führen dann zu den erfindungsgemäßen Verbindungen der allgemeinen Formel I.

Charakteristische, aber nicht einschränkende Syntheseverfahren, die zur Schaffung repräsentativer Substitutionsmuster am Estrongerüst, auch in Kombination zu mehreren Substituenten, nützlich sind, finden sich etwa in: C(1) J. Chem. Soc. (C)1968, 2915; C(7) Steroids 54, 1989, 71; C(8α) Tetrahedron Letters 1991, 743; C(8β) Tetrahedron Letters 1964, 1763; J. Org. Chem. 1970, 35, 468; C(11) J. Steroid Biochem. 31, 1988, 549; Tetrahedron 33, 1977, 609 und J. Org. Chem. 60, 1995, 5316; C(9) DE-OS 2035879; J. Chem. Soc. Perk. 1 1973, 2095; C(15) J. Chem. Soc. Perk.1 1996, 1269.); C(13α) Mendeleev Commun. 1994, 187; C(14β) Z. Chem. 23, 1983,410.

In den Beispielen und den Schemata gelten die folgenden Abkürzungen:
THF = Tetrahydrofuran; THP = Tetrahydropyran-2-yl; DHP = Dihydropyran; DMSO = Dimethylsulfoxid; MTBE = Methyl-tert.-butylether; DIBAH = Diisobutyl-aluminiumhydrid; LTBAH = Lithium-tri-tert.-butoxyaluminiumhydrid;

### Beispiel 1

### 3-Methoxy-17β-(tetrahydropyran-2-yloxy)-estra-1,3,5(10),8-tetraen-11-on (2)

Zu 15.29 g 11-Keto-3-methoxy-estra-1,3,5(10),8-tetraen-17β-ol (1) in 35 ml Dichlormethan wurden bei Raumtemp. 47 ml Dihydropyran und 0.96 g Pyridintoluolsulfonat gegeben und 2 h gerührt. Danach wurde die Reaktionslösung mehrmals mit ges. Natriumhydrogencarbonatlösung geschüttelt, mit Wasser gewaschen und mit Magnesiumsulfat getrocknet. Das Lösungsmittel wurde im Vakuum abgedampft und der Rückstand an Kieselgel (Lösungsmittelgemisch: Cyclohexan/Essigester = 8/2) gereinigt. So wurden 16.8 g (83 %) leicht gelbliches, zähes Öl erhalten.

### 8β-Cyano-3-methoxy-17β-(tetrahydropyran-2-yloxy)-estra-1,3,5(10)-trien-11-on (3)

Bei einer Temp. von -5° C wurden unter Argon zu einer Lösung von 24.5 g 11-Ketosteroid 2 in 330 ml Toluol 195 ml Diethylaluminiumcyanid (1.0 M, in Toluol) hinzugetropft und 1.5 h unter fortgesetzter Kühlung gerührt. Dann wurde die Mischung auf 470 ml eisgekühlte 1 N Natronlauge gegossen, 1 h gerührt, mehrmals mit Essigester extrahiert, die gesammelten organischen Phasen mit Wasser und Brine gewaschen und mit Magnesiumsulfat getrocknet. Die Chromatographie des Eindampfrückstandes an Kieselgel (Lösungsmittelgemisch: Cyclohexan/Essigester = 4/1) ergab 3 als Schaum in einer Ausbeute von insgeamt 12.0 g (37 %).

### 8β-Cyano-3-methoxy-17β-(tetrahydropyran-2-yloxy)-estra-1,3,5(10)-trien-11-ol (4)

Eine Lösung von 33.1 g Steroid 3 in 400 ml THF wurde auf 0° C abgekühlt, portionsweise mit 51.0 g LTBAH versetzt und die Lösung 1 h unter fortgesetzter Kühlung und 1 h bei Raumtemp. gerührt. Zur Reaktionslösung wurden bei 0° C 25 ml ges. Natriumhydrogencarbonatlösung getropft, der enstandene Niederschlag durch Filtration über Celite abgetrennt und das Filtrat weitestgehend eingeengt. Der Rückstand wurde mehrmals mit Essigester extrahiert, die gesammelten organischen Phasen anschließend mit Brine gewaschen, über Magnesiumsulfat getrocknet und das Lösungsmittel i. Vak. entfernt. Auf diese Weise wurden 27.6 g (97 %) schaumiges 4 erhalten, welches ohne weitere Reinigung in die nächste Stufe eingesetzt wurde.

### 8β-Cyano-3-methoxy-17β-(tetrahydropyran-2-yloxy)-estra-1,3,5(10),9(11)-tetraen (5)

Zu einer Lösung von 27.6 g 4 in 275 ml Pyridin wurden bei einer Temp. von 0-5° C 27.6 ml Phosphoroxychlorid zugetropft und weitere 1.5 h bei dieser Temp. gerührt. Danach wurde das Gemisch in einen Tropftricher überführt und zu einer eisgekühlten, ges. Natriumhydrogencarbonatlösung getropft. Anschließend wurde mit Dichlormethan extrahiert, die gesammelten organischen Phasen mit Brine gewaschen, über Magnesiumsulfat getrocknet und das Lösungsmittel i. Vak. entfernt. Auf diese Weise wurd 23.5 g (89 %) fast farbloses, schaumiges 5 erhalten, welches ohne weitere Reinigung in die nächste Stufe eingesetzt wurde.

### 8β-Carbonyl- 3-Methoxy-estra-1,3,5(10),9(11)-tetraen-17β-ol (6)

Zu 11.4 g des 8β-Cyarlo-steroides 5 in 70 ml Toluol wurden unter Argon bei 0° C eine Lösung von 41 ml DIBAH in 100 ml Toluol getropft und 1.5 h bei dieser Temp. gerührt. Die Lösung wurde bei 0°C nacheinander mit 33 ml Ethanol, 33 ml Ethanol-Wassergemisch (v/v =1/1) und 120 ml halbkonz. Salzsäure versetzt und dann 2 h zum Rückfluß erhitz. Das Gemisch wurde mehrmals mit Essigester extrahiert, die gesammelten organischen Phasen mit Wasser gewaschen, mit Magnesiumsulfat getrocknet und i. Vak. zur Trockene eingeengt. Durch Chromatographie des Rückstandes an Kieselgel (Lösungsmittelgemisch: Cyclohexan/Essigester = 3/2) wurden 3.21 g (35 %) schaumiges 6 erhalten.

### 3-Methoxy-8β-Methyl-estra-1,3,5(10),9(11)-tetraen-17β-ol (7a)

Zu einer Lösung von 225 mg Kaliumhydroxid in 3.5 ml Triethylenglykol wurden bei Raumtemp. 0.18 ml Hydraziniumhydroxid (80 %ig, mit Wasser) und 50 mg 8β-Carbonyl-3-Methoxy-estra-1,3,5(10),9(11)-tetraen-17β-ol (6) in 6.5 ml Triethylenglykol gegeben und 2 h auf 200° C erwärmt. Nach dem Abkühlen wurde nacheinander mit 10 ml Wasser und 3 ml 10 %iger Schwefelsäure versetzt. Das Gemisch wurde mehrmals mit Ether extrahiert, die gesammelten organischen Phasen mit Wasser gewaschen, mit Magnesiumsulfat getrocknet und am Rotationsverdampfer zur Trockene eingeengt. Die Chromatographie des Rückstandes an Kieselgel (Lösungsmittelgemisch: Cyclohexan/Essigester = 8/2) ergab 36 mg (79 %) 3-Methoxy-8β-methyl-estra-1,3,5(10),9(11)-tetraen-17β-ol vom Schmp. 168 ° C.

### Beispiel 2

### Die Synthese von Substanz 7a wurde unter Beispiel 1, 1.1-1.6 beschrieben.

### 3-Methoxy-8β-methyl-estra-1,3,5(10)-trien-17β-ol (8a)

75 mg 3-Methoxy-8β-methyl-estra-1,3,5(10),9(11)-tetraen-17β-ol (7a) wurden in einem Lösungsmittelgemisch aus 3.5 ml THF und 1.5 ml Methanol gelöst und mit 75 mg Palladium (10%ig, auf Magnesiumcarbonat) 3.75 h bei Raumtemp. unter Wasserstoffatmosphäre gerührt. Dann wurde die Reaktionslösung über Celite filtriert, das Filtrat am Rotationsverdampfer zur Trockene eingeengt und das so erhaltene DC-einheitliche, schaumige Produkt (74 mg, 98 %) ohne weitere Reinigung in die nächste Stufe eingesetzt.

### 8β-Methyl-estra-1,3,5(10)-trien-3,17β-diol (8b)

74 mg 3-Methoxy-8β-methyl-estra-1,3,5(10-trien-17β-ol wurden in 3 ml wasserfreiem Toluol gelöst, auf 0° C abgekühlt und unter Argon vorsichtig mit 0.6 ml DIBAH versetzt. Die Reaktionsmischung wurde langsam zum Rückfluß erhitzt und diese Temp. für 3.5 h gehalten. Danach wurde erneut auf 0° C abgekühlt, die Lösung nacheinander mit 2 ml Ethanol, 2 ml Ethanol-Wassergemisch (v/v = 1/1) und 2 ml halbkonz. Salzsäure versetzt und mehrmals mit Essigester extrahiert. Die gesammeiten organischen Phasen wurden mit Wasser neutral gewaschen, mit Magnesiumsulfat getrocknet und i. Vak. zur Trockene eingeengt. Es wurden 70 mg (99 %) farblose Kristalle vom Schmp.: 168-170° C erhalten.

### Beispiel 3

### Die Synthese von Substanz 6 wurde unter Beispiel 1, 1.1-1.5 beschrieben.

### 8β-Carbonyl-3-methoxy-17β-(tetrahydropyran-2-yloxy)-estra-1,3,5(10),9(11)-tetraen (9)

Eine Lösung von 500 mg 6 in 10 ml Dichlormethan wurde mit 1.45 ml 3,4-Dihydro-2H-pyran und 28 mg (0.11 mmol) Pyridintoluolsulfonat versetzt und 16 h bei Raumtemp. gerührt. Die Mischung wurde nacheinander mehrmals mit ges. Natriumhydrogencarbonatlösung und Wasser gewaschen und die organische Phase, nach dem Trocknen mit Magnesiumsulfat, i. Vak. zur Trockene eingedampft. Das Produkt 9 fiel als Schaum in einer Ausbeute von 527 mg (86 %) an.

### 3-Methoxy-17β-(tetrahydropyran.2-yloxy)-8β-vinyl-estra-1,3,5(10),9(1 1)-tetraen (10a)

Eine Lösung von 585 mg 8β-Carbonyl-3-methoxy-1 7β-(tetrahydropyran-2-yloxy)-estra-1,3,5(10),9(11)-tetraen in 25 ml DMSO wurde unter Argon zunächst mit 4.92 g Methyltriphenylphosphoniumbromid, dann vorsichtig mit 394 mg Natriumhydrid (80%ig, in Paraffinöl) versetzt und anschließend für 2 h langsam auf eine Innentemp. von 55 °C erwärmt. Nach dem Abkühlen wurden 25 ml Wasser hinzugetropft, mehrmals mit Dietylether extrahiert, mit Wasser gewaschen und die gesammelten organischen Phasen mit Magnesiumsulfat getrocknet. Nach dem Entfernen des Lösungsmittels wurde der Rückstand durch Chromatographie an Kieselgel (Lösungsmittelgemisch: Cyclohexan/MTBE = 30/1) gereinigt. Es wurden 520 mg (89 %) 8β-Vinylsteroid in Form eines farblosen Schaumes erhalten.

### 8β-Vinyl-estra-1,3,5(10),9(11)-tetraen-3,17β-diol (11a)

550 mg 3-Methoxy-17β-(tetrahydropyran-2-yloxy)-8β-vinyl-estra-1,3,5(10),9(11)-tetraen wurden nach der allgemeinen Arbeitsvorschrift 19. umgesetzt. Die Ausbeute an farblosen Kristallen vom Schmp.: 149-150° C betrug 315 mg (76 %).

### 8β-(2,2-Difluorvinyl)-3-methoxy-17β-(tetrahydropyran-2-yloxy)-estra-1,3,5(10),9(11)-tetraen (10b)

Eine Lösung von 0.22 ml Diethyl(difluormethyl)phosphonat in 0.4 ml n-Pentan und 2 ml 1,2-Dimethoxyethan wurden unter Argon auf -78° C gekühlt, mit 0.82 ml tert.-Butyllithiumlösung (1.7 M, in n-Pentan) versetzt und 0.25 h bei dieser Temp gerührt. Bei gleicher Temp. wurde nun eine Lösung aus 220 mg 8β-Carbonyl-3-methoxy-17β-(tetrahydropyran-2-yloxy)-estra-1,3,5(10),9(11)-tetraen in 3.5 ml 1,2-Dimethoxyethan und 0.58 ml n-Pentan hinzugetropft und mit fortgesetzter Kühlung 0.5 h gerührt. Danach wurde zunächst auf Raumtemp. erwärmt und anschließend unter Abdestillieren des n-Pentans für 1 h auf eine Innentemp. von 84° C erhitzt. Nach dem Abkühlen wurde der Ansatz auf 20 ml Eiswasser gegossen, vom hellbraunen Niederschlag abfiltriert, mit Dichlormethan extrahiert und die gesammelten organischen Phasen mit Magnesiumsulfat getrocknet. Nach dem Entfernen des Lösungsmittels wurde der Rückstand durch Chromatographie an Kieselgel (Lösungsmittelgemisch: Cyclohexan/MTBE = 30/1) gereinigt. Die Ausbeute an öligem, fast farblosem Steroid betrug 108 mg (46 %).

### 8β-(2,2-Difluorvinyl)-estra-1,3,5(10),9(11)-tetraen-3,17β-diol (11b)

105 mg 8β-(2,2-Difluorvinyl)-3-methoxy-17β-(tetrahydropyran-2-yloxy)-estra-1,3,5(10),9(11)-tetraen wurden nach der allgemeinen Arbeitsvorschrift 19. zur Etherspaltung mit DIBAH / Säure umgesetzt. Die Ausbeute an farblosen Kristallen vom Schmp.: 103-106° C betrug 75 mg (93 %).

### Beispiel 4

### Die Synthese von Substanz 9 wurde unter Beispiel 3, 3.1 beschrieben.

### 8β-Carbonyl-3-methoxy-17β-(tetrahydropyran-2-yloxy)-estra-1,3,5(10)-trien (12)

1.73 g 9 wurden in 75 ml Lösungsmittelgemisch aus THF und Methanol (v/v = 7/3) gelöst und mit 1.0 g Palladium (10%ig, auf Magnesiumcarbonat) 3.75 h bei Raumtemp. unter Wasserstoffatmosphäre gerührt. Dann wurde die Reaktionslösung über Celite filtriert, das Filtrat am Rotationsverdampfer zur Trockene eingeengt und das so erhaltene DC-einheitliche, helle Öl ohne zusätzliche Reinigung für weitere Umsetzungen eingesetzt.

### 3-Methoxy-17β-(tetrahydropyran-2-yloxy)-8β-vinyl-estra-1,3,5(10)-trien (13a)

Eine Lösung von 2.47 g 8β-Carbonyl-3-methoxy-17β-(tetrahydropyran-2-yloxy)-estra-1,3,5(10),9(11)-tetraen in 100 ml DMSO wurde unter Argon zunächst mit 19.80 g Methyltriphenylphosphoniumbromid, dann vorsichtig mit 1.58 g Natriumhydrid (80%ig, in Paraffinöl) versetzt und anschließend für 2 h langsam auf eine Innentemp. von 55 °C erwärmt. Nach dem Abkühlen wurden 100 ml Wasser hinzugetropft, mehrmals mit Dietylether extrahiert, mit Wasser gewaschen und die gesammelten organischen Phasen mit Magnesiumsulfat getrocknet. Nach dem Entfernen des Lösungsmittels wurde der Rückstand durch Chromatographie an Kieselgel (Lösungsmittelgemisch: Cyclohexan/MTBE = 30/1) gereinigt. Es wurden 1.91 g (78 %) 8β-Vinylsteroid als farbloser Schaum erhalten.

### 8β-Vinyl-estra-1,3,5(10)-trien-3,17β-diol (14a)

1.86 g 3-Methoxy-17β-(tetrahydropyran-2-yloxy)-8β-vinyl-estra-1,3,5(10)-trien wurden nach der allgemeinen Arbeitsvorschrift 19. umgesetzt. Das rohe 8β-Vinyl-.estra-1.3.5(10)-trien-3.17β-diol wurde nach einer Reinigung an Kieselgel (Lösungsmittelgemisch: Cyclohexan/Essigester = 7/3) in Form farbloser Kristalle vom Schmp.: 163-165° C in einer Ausbeute von 1.20 g (86 %) erhalten.

### 8β-(2,2-Difluorvinyl)-3-methoxy-17β-(tetrahydropyran-2-yloxy)-estra-1,3,5(10)-trien (13b)

Eine Lösung von 0.6 ml Diethyl(difluormethyl)phosphonat in 1.0 ml n-Pentan und 5.6 ml 1,2-Dimethoxyethan wurden unter Argon auf -78° C gekühlt, mit 2.2. ml tert.-Butyllithiumlösung (1.7 M, in n-Pentan) versetzt und 0.25 h bei dieser Temp gerührt. Bei gleicher Temp. wurde nun eine Lösung aus 600 mg 8β-Carbonyl-3-methoxy-1 7β-(tetrahydropyran-2-yloxy)-estra-1,3,5(10),9(11)-tetraen in 9.2 ml 1,2-Dimethoxyethan und 1.6 ml n-Pentan hinzugetropft und mit fortgesetzter Kühlung 0.5 h gerührt. Danach wurde zunächst auf Raumtemp. erwärmt und anschließend unter Abdestillieren des n-Pentans für 1 h auf eine Innentemp. von 84° C erhitzt. Nach dem Abkühlen wurde der Ansatz auf 40 ml Eiswasser gegossen, vom hellbraunen Niederschlag abfiltriert, mit Dichlormethan extrahiert und die gesammelten organischen Phasen mit Magnesiumsulfat getrocknet. Nach dem Entfernen des Lösungsmittels wurde der Rückstand durch Chromatographie an Kieselgel (Lösungsmittelgemisch: Cyclohexan/MTBE = 30/1) gereinigt. Die Ausbeute an öligem, fast farblosem Steroid betrug 75 mg (12 %).

### 8β-(2,2-Difluorvinyl)-estra-1,3,5(10)-trien-3,17β-diol (14b)

Nach der allgemeinen Arbeitsvorschrift 19. wurden 78 mg 3-Methoxy-8β-(2,2-difluorvinyl)-17β-(tetrahydropyran-2-yloxy)-estra-1,3,5(10)-trien umgesetzt. Die Ausbeute an farblosen Kristallen vom Schmp.: 154-156° C betrug 56 mg (90 %)

### Beispiel 5

### Die Synthese von Substanz 13a wurde unter Beispiele 4, 4.2 beschrieben.

### 8β-Ethyl-3-methoxy-17β-(tetrahydropyran-2-yloxy)-estra-1,3,5(10)-trien

0.50 g 13a wurden in 25 ml Lösungsmittelgemisch aus THF und Methanol (v/v = 7/3) gelöst und mit 0.30 g Palladium (10%ig, auf Magnesiumcarbonat) 3.75 h bei Raumtemp. unter Wasserstoffatmosphäre gerührt. Dann wurde die Reaktionslösung Ober Celite filtriert, das Filtrat am Rotationsverdampfer zur Trockene eingeengt und der erhaltene, helle Schaum ohne zusätzliche Reinigung in die nächste Stufe eingesetzt.

### 8β-Ethyl-estra-1,3,5(10)-trien-3,17β-diol (15a, 15b)

330 mg Rohes 8β-Ethyl-3-methoxy-17β-(tetrahydropyran-2-ytoxy)-estra-1,3,5(10)-trien aus der letzten Stufe wurde nach der allgemeinen Arbeitsvorschrift 6.1 und 6.2 umgesetzt. Durch Chromathographie an Kieselgel können aus dem so anfallenden Rohprodukt die epimeren Estratriendiole **15a** und **15b** in Ausbeuten von 161 mg bzw. 20 mg isoliert werden. Der Schmp. für **15a** liegt bei 149-152 °C, der von **15b** bei 185-187 °C.

### Beispiel 6

### 3-Methoxy-8β-vinyl-estra-1,3,5(10)-trien-17-on

Eine Lösung von 700 mg 3-Methoxy-8β-vinyl-estra-1,3,5(10)-trien-17β-ol in 30 ml Dichlormethan wurde mit 740 mg Pyridiniumchlorochromat versetzt und 3 h bei Raumtemp. gerührt. Durch Filtration des Reaktionsgemisches über Kieselgel (Lösungsmittelgemisch: Cyclohexan/Essigester=7/3) und anschließendes Einengen des Filtrates am Rotationsverdampfer wurden 680 mg (98 %) 3-Methoxy-8β-vinyl-estra-1,3,5(10)-trien-17-on als fast farbloser Schaum erhalten, welcher ohne weitere Reinigung in die nächste Stufe eingesetzt wurde.

### 3-Hydroxy-8β-vinyl-estra-1,3,5(10)-trien-17-on

Zu 9.2 g Pyridiniumhydrochlorid wurden bei 180° C 460 mg 3-Methoxy-8β-vinyl-estra-1,3,5(10)-trien-17-on gegeben und bei gleicher Temp. 3 h gerührt. Anschließend wurde auf Eis gegossen, der ausgefallene Niederschlag abfiltriert, mit Wasser gewaschen und getrocknet. Die Ausb. an 2-Hydroxy-8β-Vinyl-estra-1,3,5(10)-trien-17-on vom Schmp.: 239-242° C betrug 400 mg (90 %).

### Beispiel 7

### 3-Sulfamoyloxy-8β-vinyl-estra-1,3,5(10)-trien-17-on

76 mg 3-Hydroxy-8β-vinyl-estra-1,3,5(10)-trien-17-on wurden in 7 ml Dichlormethan gelöst, mit 0.26 ml 2,6-Di-tert.-butylpyridin und 221 mg Sulfamoylchlorid versetzt und 1.5 h bei RT gerührt. Anschließend wurde das Reaktionsgemisch auf Wasser gegeben und mehrmals mit Dichlormethan extrahiert. Die gesammelten organischen Phasen wusch man mit ges. Natriumchloridlösung, trocknete über Magnesiumsulfat und engte i. Vak. weitestgehend ein. Durch Chromatographie des erhaltenen Rückstandes an Kieselgel (Lösungsmittelgemisch: Cyclohexan/Essigester=7/3) wurden 46 mg (48 %) 17-Oxo-8β-vinyl-estra-1,3,5(10)-trien-3-yl-amidosulfonat erhalten.

### 3-Sulfamoyloxy-8β-vinyl-estra-1,3,5(10)-trien-17β-ol

46 mg 17-Oxo-8β-vinyl-estra-1,3,5(10)-trien-3-yl-amidosulfonat wurden in 1.5 ml THF und 1.5 ml Methanol gelöst, bei 0°C mit 33 mg Natriumborhydrid versetzt und 1 h bei 0° C gerührt. Dann gab man 0.2 ml konz. Essigsäure hinzu und engte i. Vak. ein. Der Rückstand wurde in Essigester und Wasser aufgenommen, die organische Phase abgetrennt und die wässrige mehrmals mit Essigester extrahiert. Die gesammelten organischen Phasen wurden mit ges. Natriumchloridlösung gewaschen, über Magnesiumsulfat getrocknet und i. Vak. eingeengt. Das Rohprodukt wurde durch Chromatographie an Kieselgel (Lösungsmittelgemisch: Cyclohexan/Essigester=6/4) gereinigt und ergab 45 mg (98 %) 17β-Hydroxy-8β-vinyl-estra-1.3,5(10)-trien-3-yl-amidosulfonat in Form feiner Nädelchen vom Schmp.: 82-86° C.

### Beispiel 8

### 3-Methoxy-8β-prop-1-(Z)-enyl-17β-(tetrahydropyran-2-yloxy)-estra-1,3,5(10)-trien

Eine Lösung von 100 mg 8β-Formyl-3-methoxy-17β-(tetrahydropyran-2-yloxy)-estra-1,3,5(10)-trien in 5 ml DMSO wurde unter Argon zunächst mit 830 mg Ethyltriphenylphosphoniumbromid, dann vorsichtig mit 64 mg Natriumhydrid (80 %ig, in Paraffinöl) versetzt und anschließend für 2 h langsam auf eine Innentemp. von 60° C erwärmt. Nach dem Abkühlen wurden 10 ml Wasser hinzugetropft, mehrmals mit Essigester extrahiert, die gesammelten organischen Phasen mit Wasser gewaschen und über Magnesiumsulfat getrocknet. Nach dem Entfernen des Lösungsmittels wurde der Rückstand durch Chromatographie an Kieselgel (Lösungsmittelgemisch: Cyclohexan/Essigester=30/1) gereinigt. Es wurden 24 mg (23 %) 8β-Propenylsteroid in Form eines farblosen Schaumes erhalten.

### 8β-Prop-1-(Z)-enyl-estra-1,3,5(10)-trien-3,17β-diol

24 mg 3-Methoxy-8β-(prop-1-(Z)-enyl)-17β-(tetrahydropyran-2-yloxy)- estra-1,3,5(10)-trien wurden nach den allgemeinen Arbeitsvorschriften zur THP- und 3-Methyletherspaltung umgesetzt. Das rohe 8β-Prop-1-(Z)-enyl-estra-1,3,5(10)-trien-3,17β-diol wurde nach einer Reinigung an Kieselgel (Lösungsmittelgemisch: Cyclohexan/Essigester=7/3) in Form farbloser Kristalle vom Schmp.: 119-125° C in einer Ausbeute von 10 mg (66 %) erhalten.

### Beispiel 9

### 3-Methoxy-17α-ethinyl-8β-vinyl-estra-1,3,5(10)-trien-17β-ol

Unter Argon wurden 85 mg 3-Methoxy-8β-vinyl-estra-1,3,5(10)-trien-17-on in 8 ml THF gelöst, auf -78°C abgekühlt und mit 5.5 ml Ethinylmagnesiumbromidlösung (0.5 M in THF) und 100 mg Lithiumacetylid-Ethylendiamin-Komplex versetzt. Unter Erwärmung auf Raumtemp. wurde das Reaktionsgemisch 3 h gerührt, dann auf 0°C abgekühlt und 10 ml ges. Ammoniumchloridlösung versetzt. Das Gemisch wurde mehrmals mit Essigester extrahiert, die gesammelten organischen Phasen mit ges. Natriumchloridlösung gewaschen gewaschen, über Magnesiumsulfat getrocknet und i.Vak. eingeengt. Durch Chromatographie des Rückstandes an Kieselgel (Lösungsmittelgemisch: Cyclohexan/Essigester=9:1) wurden 30 mg (33 %) öliges 17α-Ethinyl-3-methoxy-8β-vinyl-estra-1,3,5(10)-trien-17β-ol gewonnen.

### 17α-Ethinyl-8β-vinyl-estra-1,3,5(10)-trien-3,17β-diol

Eine Lösung von 15 mg 17α-Ethinyl-3-methoxy-8β-vinyl-estra-1,3,5(10)-trien-17β-ol und 82 mg Tetrabutyl-ammoniumiodid in 2 ml Dichlormethan wurde unter Argon auf - 78° C abgekühlt, mit 0.3 ml einer Bortrichloridlösung (1 M in Dichlormethan) versetzt und 24 h bei 0°C gerührt. Anschließend wurde die Reaktionslösung zu einer auf 5° C abgekühlten ges. Ammoniumchloridlösung getropft, die Mischung mehrmals mit Diethylether extrahiert, die gesammelten organischen Phasen mit ges. Natriumchloridlösung gewaschen gewaschen, über Magnesiumsulfat getrocknet und i.Vak. eingeengt. Durch Chromatographie des Rückstandes an Kieselgel (Lösungsmittelgemisch: Cyclohexan/Essigester=7:3) wurden 5 mg (35 %) 17α-Ethinyl-8β-vinyl-estra-1,3,5(10)-trien-3,17β-diol gewonnen vom Schmp. 156° C.

### Beispiel 10

### 3-Methoxy-17α-methyl-8β-vinyl-estra-1,3,5(10)-trien-17β-diol

Zu einer auf -78° C abgekühlten Lösung von 1 ml Methyllithium-Lösung (1.6 M in Diethylether) wurden unter Argon eine Lösung von 50 mg 3-Methoxy-8β-vinyl-estra-1,3,5(10)-trien-17-on in 2 ml wasserfreiem THF getropft, dann 0.5 ml wasserfreies Dimethylforinamid hinzugefügt und unter Erwärmung auf Raumtemp. 1.5 h gerührt. Das Gemisch wurde mit ges. Natriumhydrogencarbonatlösung versetzt, mehrmals mit Essigester extrahiert, die gesammelten organischen Phasen mit Wasser gewaschen und über Magnesiumsulfat getrocknet. Durch Eindampfen der organischen Phasen wurden 42 mg (80 %) rohes 3-Methoxy-17α-methyl-8β-vinyl-estra-1,3,5(10)-trien-17β-ol erhalten, welches ohne weitere Reinigung zur 3-Methyletherspaltung eingesetzt wurde.

### 17α-Methyl-8β-vinyl-estra-1,3,5(10)-trien-3,17β-diol

40 mg 3-Methoxy-17α-methyl-8β-vinyl-estra-1,3,5(10)-trien-17β-ol wurden nach der allgemeinen Arbeitsvorschrift zur 3-Methyletherspaltung umgesetzt. Das so erhaltene 17α-Methyl-8β-vinyl-estra-1,3,5(10)-trien-3,17β-diol wurde nach einer Reinigung an Kieselgel (Lösungsmittelgemisch: Cyclohexan/Essigester=7/3) in einer Ausbeute von 30 mg (78 %) in vom Schmp.: 129-130° C erhalten.

### Beispiel 11

### 3-Methoxy-8β-vinyl-estra-1,3,5(I 0)-trien-17α-(4'-nitro)-benzoat

Zu einer Mischung aus 100 mg 3-Methoxy-8β-vinyl-estra-1,3,5(10)-trien-17β-ol, 277 mg Triphenylphosphin, 175 mg 4-Nitrobenzoesäure und 5 ml Toluen wurden 0.48 ml einer 40 %igen Lösung von Diethylazodicarboxylat in Toluen getropft und 3 h bei 60° C gerührt. Nach dem Abkühlen wurde mehrmals mit Essigester extrahiert, die gesammelten organischen Phasen nacheinander mit ges. Natriumhydrogencarbonatlösung und ges. Natriumchloridlösung gewaschen, über Magnesiumsulfat getrocknet und i. Vak. eingeengt. Durch Chromatographie an Kieselgel (Lösungsmittelgemisch: n-Hexan/Essigester=25/1) wurden 84 mg (57 %) gelbliches, öliges 3-Methoxy-8β-vinyl-estra-1,3,5(10)-trien-17α-(4'-nitro)-benzoat erhalten.

### 3-Methoxy-8β-vinyl-estra-1,3,5(10)-trien-17α-ol

Eine Lösung von 80 mg 3-Methoxy-8β-vinyl-estra-1,3,5(10)-trien-17α-(4'-nitro)-benzoat in 12 ml Methanol und 0.4 ml Wasser wurde mit 480 mg Kaliumcarbonat versetzt und 24 h bei Raumtemp gerührt. Danach wurde i. Vak. weitestgehend eingeengt, der Rückstand in Wasser aufgenommen und mehrmals mit Essigester extrahiert. Die gesammelten organischen Phasen wurden mit ges. Natriumchloridlösung gewaschen, über Magnesiumsulfat getrocknet und am Rotationsverdampfer eingeengt. Auf diese Weise wurden 40 mg (54 %) 3-Methoxy-8β-vinyl-estra-1,3,5(10)-trien-17α-ol erhalten.

### 8β-Vinyl-estra-1,3,5(10)-trien-3,17α-ol

40 mg (0.13 mmol) 3-Methoxy-8β-vinyl-estra-1,3,5(10)-trien-17α-ol wurden nach der allgemeinen Arbeitsvorschrift zur 3-Methyletherspaltung umgesetzt. Das dabei erhaltene 8β-Vinyl-estra-1,3,5(10)-trien-3,17α-diol wurde nach einer Reinigung an Kieselgel (Lösungsmittelgemisch: Cyclohexan/Essigester--7/3) in einer Ausbeute von 9 mg (24 %) vom Schmp.: 149-151 °C erhalten.

### Beispiel 12

### 16-Dimethyl-3-methoxy-8β-vinyl-estra-1,3,5(10)-trien·17-on

Eine auf - 40° C gekühlte Lösung von 150 mg 3-Methoxy-8β-vinyl-estra-1,3,5(10)-trien-17β-ol in 6 ml wasserfreiem THF wurde unter Argon mit 1.2 ml einer Lösung von Lithiumdiisopropylamid (2 M in THF/n-Heptan/Ethylbenzol) versetzt und 1 h bei dieser Temp gerührt. Danach wurden bei gleicher Temp. 0.24 ml Methyliodid hinzugefügt und unter Erwärmung auf Raumtemp. 1 h weiter gerührt. Anschließend wurde auf -5° C abgekühlt, 4 ml 2 N Natronlauge hinzugefügt und das Gemisch mehrmals mit Essigester extrahiert. Die gesammelten organischen Phasen wurden mit Wasser gewaschen, über MgSO₄ getrocknet und i. Vak. eingeengt.

Das so erhaltene Rohprodukt wurde nochmals unter den gleichen Reaktionsbedingungen eingesetzt.

Man erhielt 130 mg (80 %) gelb-braunes, öliges 16-Dimethyl-3-methoxy-8β-vinyl-estra-1,3,5(10)trien-17-on als Rohmaterial.

### 16-Dimethyl-8β-vinyl-estra-1,3,5(10)-trien-3,17β-ol

130 mg rohes 16-Dimethyl-3-methoxy-8β-vinyl-estra-1,3,5(10)-trien-17-on wurden nach der allgemeinen Arbeitsvorschrift zur 3-Methyletherspaltung umgesetzt. Das erhaltene Rohprodukt wurde durch Chromatographie an Kieselgel (Lösungsmittelgemisch: Cyclohexan/Essigester=85/15) gereinigt. Dabei fielen 50 mg (40 %) farbloses, kristallines 16-Dimethyl-8β-vinyl-estra-1,3,5(10)-trien-3,17β-ol vom Schmp.: 113-123° C (Zers.) an.

### Beispiel 13

### 3-Methoxy-8β-(prop-1-(E)-en-yl)-17β-(tetrahydropyran-2-yloxy)-estra-1,3,5(10)-trien

Eine auf-78° C abgekühlte Mischung aus 4 ml Pentan, 20 ml 1,2-Dimethoxyethan, 2 ml Diethylethylphosphonat wurde unter Argon mit 8 ml einer 1.7 M Lösung von tert. Butyllithium (in Pentan) versetzt und 15 min. bei dieser Temp. gerührt. Danach wurde eine Lösung aus 500 mg 8β-Formyl-3-methoxy-17β-(tetrahydropyran-2-yloxy)-estra-1,3,5(10)-triene in 8 ml 1,2-Dimethoxyethan und 1.5 ml Pentan zugetropft, 30 min. unter fortgesetzter Kühlung 1.5 h unter Erwärmung auf Raumtemp. gerührt. Anschließend wurde das Pentan abdestilliert und die verbleibende Reaktionslösung 3 h zum Rückfluß erhitzt.

Das Gemisch wurde auf zerstoßenes Eis gegossen und der feine weiße Niederschlag abfiltriert und getrocknet. Nach einer chromatographischen Reinigung an Kieselgel (Lösungsmittelgemisch: Cyclohexan/Essigester-20/1) wurden 275 mg (54%) 3-Methoxy-8β-(prop-1-(E)-enyl)-17β-(tetrahydropyran-2-yloxy)-estra-1,3,5(10)-trien in Form eines farblosen Schaumes erhalten.

### 8β-Prop-1-(E)enyl-estra-1,3,5(10)-trien-3,17β-diol

275 mg 3-Methoxy-8β-(prop-1-(E)-enyl)-17β-(tetrahydropyran-2-yloxy)- estra-1,3,5(10)-trien wurden nach den allgemeinen Arbeitsvorschriften zur THP- und 3-Methyletherspaltung umgesetzt. Das rohe 8β-Prop-1-(E)-enyl-estra-1,3,5(10)-trien-3.17β-diol wurde nach einer Reinigung an Kieselgel (Lösungsmittelgemisch: Cyclohexan/Essigester=8/2) mit einem Schmp. von 110-125° C in einer Ausbeute von 108 mg (52 %) erhalten.

### Beispiel 14

### 3-Methoxy-17α-trifluormethyl-17β-trimethylsilyloxy-8β-vinyl-estra-1,3,5(10)-trien

Eine auf 0° C abgekühlte Lösung von 80 mg 3-Methoxy-8β-vinyl-estra-1,3,5(10)-trien-17-on in 2 ml THF wurde unter Argon mit 0.2 ml Trifluormethyltrimethyl-silan, sowie 5 mg Tetrabutylammoniumfluorid Trihydrat versetzt und 24 h bei Raumtemp. gerührt. Die dunkle Reaktionslösung wurde auf eiskaltes Wasser gegossen, mehrmals mit Essigester extrahiert, mit ges. Natriumchloridlösung gewaschen, über Magnesiumsulfat getrocknet und i. Vak. eingeengt. Das erhaltene Rohprodukt wurde durch Flash-Chromatographie an Kieselgel (Lösungsmittelgemisch: Cyclohexan/Essigester=9/1) gereinigt. Man erhielt 63 mg (54 %) 3-Methoxy-17α-trifluormethyl-17β-trimethylsilyloxy-8β-vinyl-estra-1,3,5(10)-trien als dunkles Öl.

### 17α-Trifluormethyt-8β-vinyl-estra-1,3,5(10)-trien-3,17β-diol

Zu einer Lösung von 60 mg 3-Methoxy-17α-trifluormethyl-17β-trimethylsilyloxy-8β-vinyl-estra-1,3,5(10)-trien in 6 ml THF wurden 1.26 g Tetrabutylammoniumfluorid Trihydrat gegeben und 2 h bei Raumtemp. gerührt. Dann wurde ges.

Natriumchloridlösung hinzugefügt, mehrmals mit Essigester extrahiert, die gesammelten organischen Phasen über Magnesiumsulfat getrocknet und i. Vak. eingeengt. Der ölige, gelbe Rückstand (50 mg) wurde ohne weitere Reinigung in die nächste Stufe eingesetzt.

Eine auf -78°C abgekühlte Lösung von 50 mg rohem 3-Methoxy-17α-trifluormethyl-8β-vinyl-estra-1,3,5(10)-trien-17β-ol in 3 ml Dichlormethan wurde unter Argon nacheinander mit 243 mg Tetrabutylammonium-iodid und 0.7 ml einer 1 M Bortrichloridlösung in Dichlormethan versetzt und unter Erwärmen auf 0° C 2 h gerührt. Dann wurde das Reaktionsgemisch in eine 5° C kalte ges. Ammoniumchloridlösung getropft und mehrmals mit Essigester extrahiert. Die gesammelten organischen Phasen wurden mit ges. Natriumchloridlösung gewaschen, über Magnesiumsulfat getrocknet und i. Vak. eingeengt. Das Rohprodukt (90 mg) wurde durch Chromatographie an Kieselgel (Lösungmittelgemisch: Cyclohexan/Essigester=7/3) gereinigt. Man erhielt 25 mg (52 %) pulveriges 17α-Trifluormethyl-8β-vinyl-estra-1,3,5(10)-trien-3,17β-diol vom Schmp.: 76-79° C.

### Beispiel 15

### 2-Fluor-3,17β-bis-(tetrahydropyran-2-yloxy)-8β-vinyl-estra-1,3,5(10)-trien

Zu einer auf -78° C abgekühlten Lösung von 120 mg 3,17β-Bis-(tetrahydropyran-2-yloxy)-8β-vinyl-estra-1,3,5(10)-trien in 4 ml THF wurden unter Argon 3 ml 1.3 M s-Butyllithiumlösung getropft, 30 min. gerührt und dann unter fortgesetzter Kühlung eine Lösung aus 650 mg N-Fluordibenzolsulfonimid in 4 ml THF zugetropft. Das Reaktionsgemisch wurde zunächst 1 h bei -78° C dann unter Erwärmung auf Raumtemp. noch weitere 16 h gerührt. Die Reaktionslösung wurde auf Eiswasser gegossen, mehrmals mit Essigester extrahiert, die gesammelten organischen Phasen mit ges. Natriumchloridlösung gewaschen, über Magnesiumsulfat getrocknet und i. Vak. eingeengt. Das dunkle, ölige Rohprodukt (330 mg) wurde ohne weitere Aufreinigung in die nächste Stufe eingesetzt.

### 2-Fluor-8β-vinyl-estra-1,3,5(10)-trien-3,17β-diol

Das ölige Rohprodukt der letzten Stufe wurde in 10 ml Methanol gelöst, mit 1 ml Wasser und 250 mg Oxalsäure Dihydrat versetzt und 1 h auf 60°C erwärmt.
Zur Aufarbeitung wurde mit Essigester verdünnt, nacheinander mit ges. Natriumhydrogencarbonatlösung und ges. Natriumchloridlösung gewaschen, über Magnesiumsulfat getrocknet und i. Vak. eingeengt. Das erhaltene Rohprodukt wurde durch Chromatographie an Kieselgel (Lösungsmittelgemisch: Cyclohexan/Essigester =8/2) aufgetrennt. Das so erhaltene 2Fluor-8β-vinyl-estra-1.3.5(10)trien-3,17β-diol (15 mg, 18 %) wies einen Schmp. von 67-73° C auf.

### Beispiel 16

### 3,17β-Bis-(tetrahydropyran-2-yloxy)-8β-vinyl-estra-1,3,5(10)-trien-2-ol

Zu einer auf -78° C abgekühlten Lösung von 120 mg 3,17β-Bis-(tetrahydropyran-2-yloxy)-8β-vinyl-estra-1,3,5(10)-trien in 4 ml THF wurden unter Argon 3 ml 1.3 M s-Butyllithiumlösung getropft, 30 min. gerührt und dann im Schuss 0.5 ml Trimethylborat zugegeben. Unter Erwärmung auf 0° C wurde 2 h gerührt, dann 2 ml 3 N Natronlauge und 1 ml 30 %iges Wasserstoffperoxid hinzugefügt und schließlich weitere 4 h bei Raumtemp. gerührt.
Das Gemisch wurde mit Wasser verdünnt, ges. Natriumhydrogensulfitlösung hinzugegeben, mehrmals mit Methyl-tert.-butylether extrahiert, die gesammelten organischen Phasen mit ges. Natriumchloridlösung gewaschen, über Magnesiumsulfat getrocknet und i. Vak. eingeengt. Durch Chromatographie des Eindampfrückstandes an Kieselgel (Lösungsmittelgemisch: n-Hexan/Essigester=9:1) wurden 65 mg (52 %) farbloses, öliges 3,17β-Bis-(tetrahydropyran-2-yloxy)-8β-vinyl-estra-1,3,5(10)-trien-2-ol erhalten.

### 8β-Vinyl-estra-1,3,5(10)-trien-2,3,17β-triol

Das ölige Produkt der ietzien Stufe wurde in 3 mi Methanol gelöst, mit 0.3 ml Wasser und 50 mg Oxalsäure-Dihydrat versetzt und 1 h auf 60°C erwärmt.
Zur Aufarbeitung wurde mit Essigester verdünnt, nacheinander mit ges. Natriumhydrogencarbonatlösung und ges. Natriumchloridlösung gewaschen, über Magnesiumsulfat getrocknet und i. Vak. eingeengt. Das so erhaltene gelbliche, pulverige 8β-Vinyl-estra-1,3,5(10)-trien-2,3,17β-triol (38 mg, 95 %) wies einen Schmp. von 82-85° C (Zers.) auf.

### Allgemeine Arbeitsvorschrift zur Etherspaltung von 3-Methoxy-17-(tetrahydropyran-2-yloxy)-estratrienen und -tetraenen zu den entsprechenden 17-Alkoholen durch Säure

1.0 mmol Steroid werden in 22 ml Aceton gelöst und mit 1.5 ml 4 N Salzsäure 3 h bei Raumtemp. gerührt. Falls in dieser Zeit kein vollständiger Umsatz erzielt wird wird die Lösung zusätzlich für 1.5 h auf 50° C erwärmt. Danach wird mit 20 ml Wasser verdünnt, mehrfach mit Dichlormethan extrahiert, die gesammelten organischen Phasen mit Magnesiumsulfat getrocknet und am Rotationsverdampfer das Lösungsmittel abdestilliert. Die auf diese Art hergestellten rohen 17-Hydroxylverbindungen fallen als Schäume an und werden direkt weiterverarbeitet.

### Allgemeine Arbeitsvorschrift zur Etherspaltung von 3-Methoxy-17-(tetrahydropyran-2-yloxy)-estratrienen und -tetraenen zu den entsprechenden 3,17-Diolen mit Säure und DIBAH

1,0 mmol Steroid werden in 15 - 20 ml wasserfreiem Toluol gelöst, auf 0° C abgekühlt und unter Argon vorsichtig mit 3.0 ml DIBAH versetzt. Die Reaktionsmischung wird langsam zum Rückfluß erhitzt und diese Temp. für 3.5 h gehalten. Dann werden zu der auf 0° C abgekühlten Lösung nacheinander vorsichtig 10 ml Ethanol, 10 ml Ethanol-Wassergemisch (v/v = 1/1) und 10 ml halbkonz. Salzsäure getropft und mehrmals mit Essigester extrahiert. Die gesammelten organischen Phasen werden mit Wasser neutral gewaschen, mit Magnesiumsulfat getrocknet und am Rotationsverdampfer zur Trockene eingeengt. Die Ausbeuten liegen zwischen 90 und 99 %.

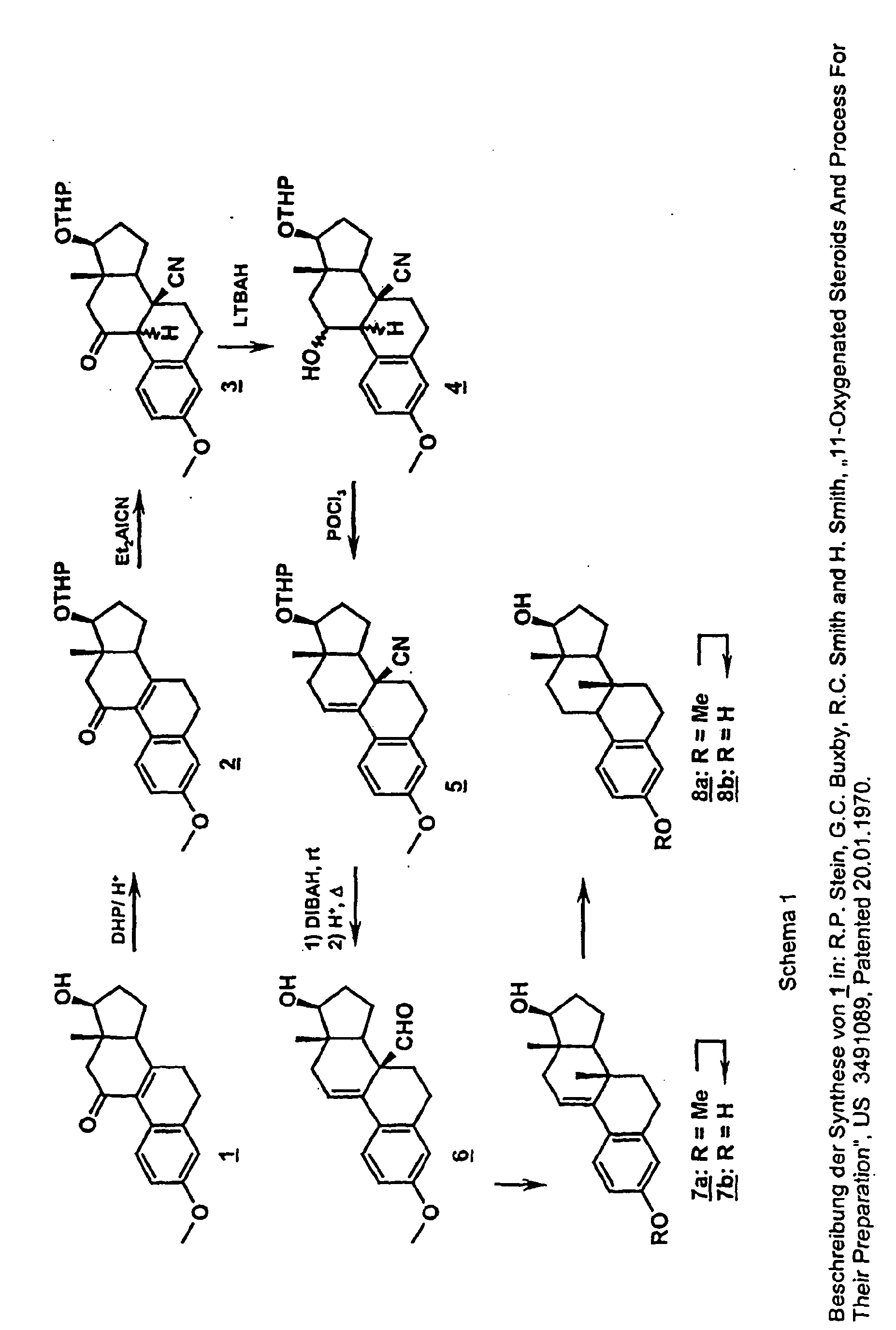

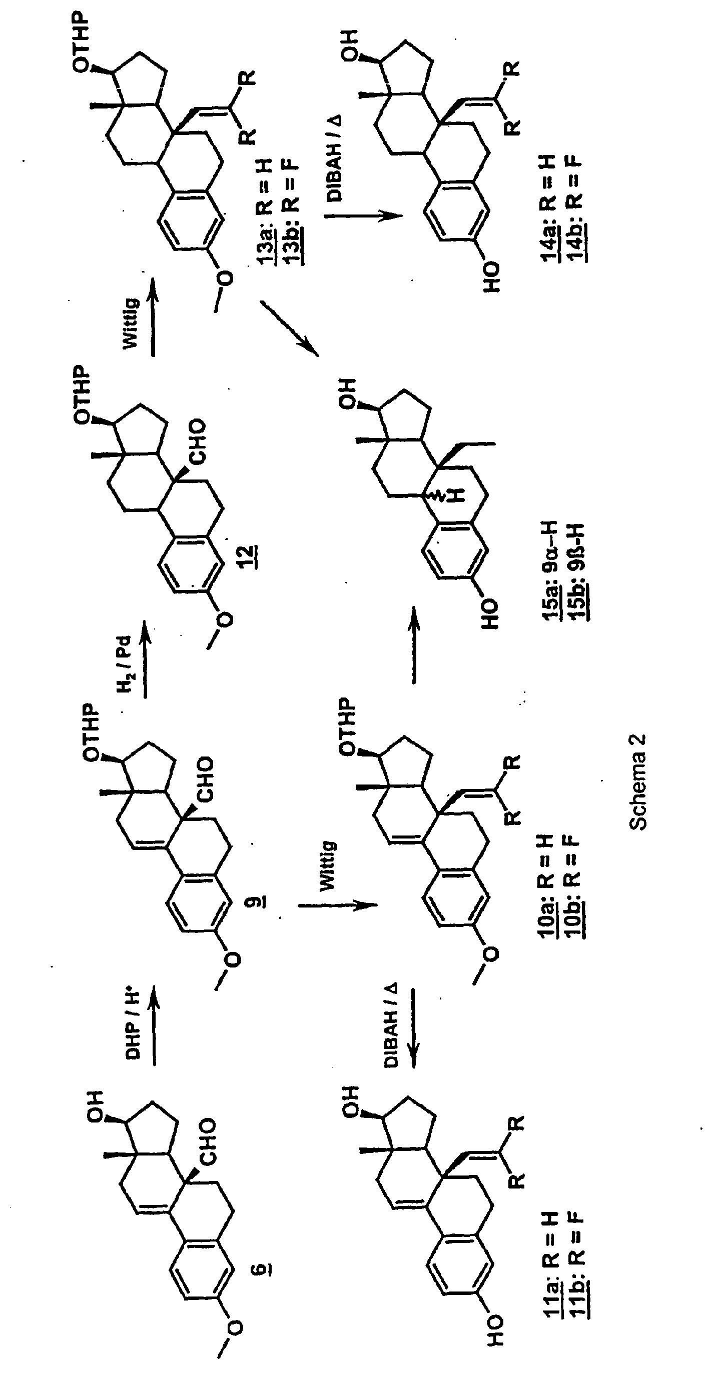

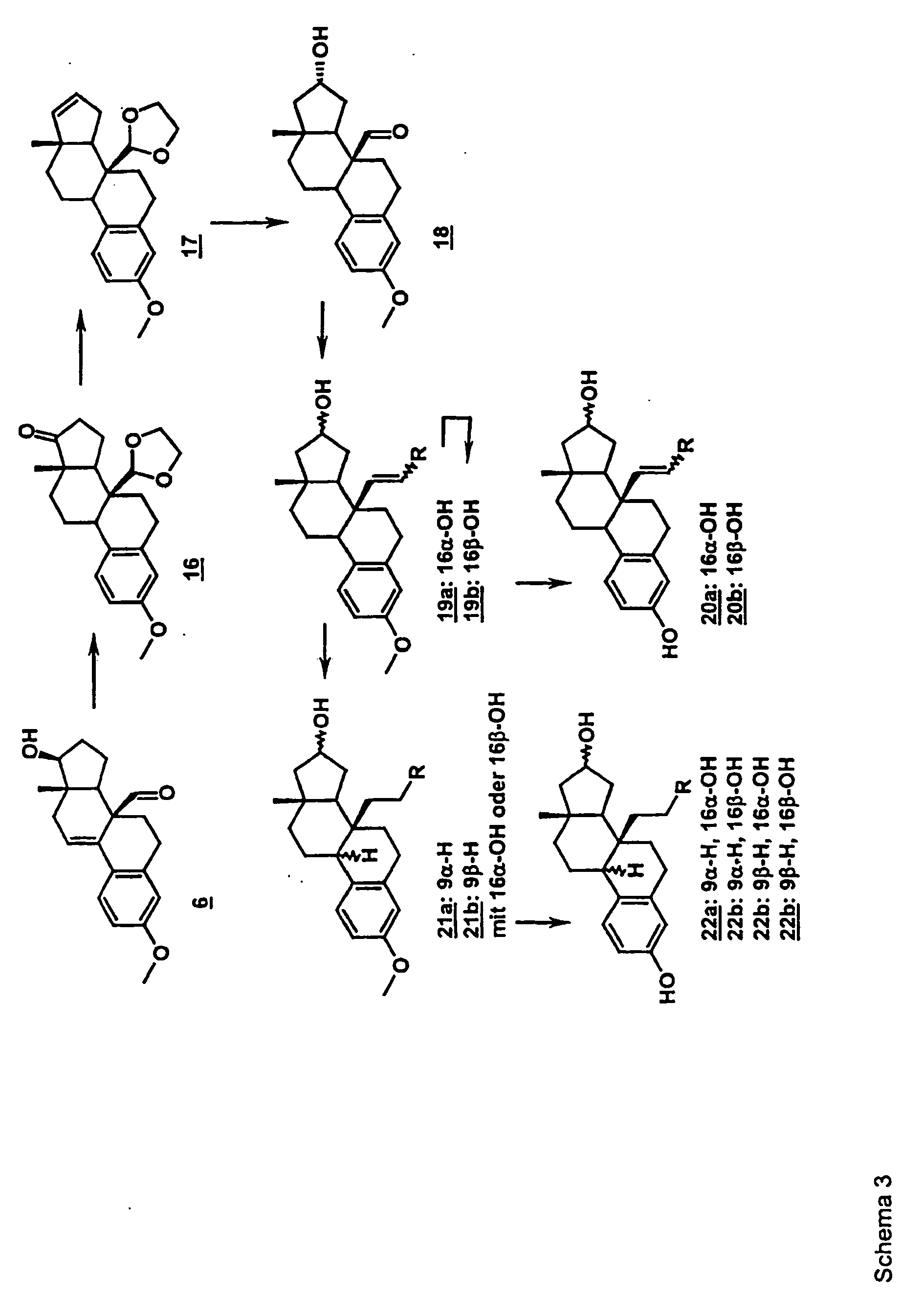

## Patentansprüche

1. 8β-substituierte Estra-1,3,5(10)-trienderivate der allgemeinen Formel 1 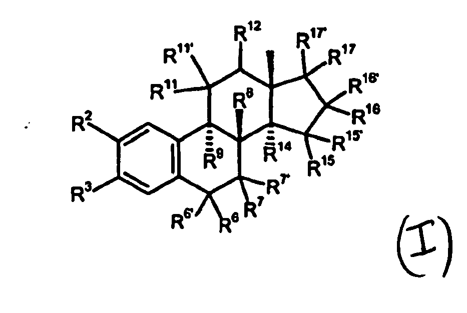 worin
R² ein Wasserstoffatom, ein Halogenatom; ein Rest R¹⁸- oder R¹⁸-O-, wobei R¹⁸ ein Wasserstoffatom oder einen gerad- oder verzweigtkettigen, gesättigten oder ungesättigten Kohlenwasserstoffrest mit bis zu 6 Kohlenstoffatomen, eine Trifluormethylgruppe bedeutet;
eine Gruppe R¹⁹SO₂-O-, worin R¹⁹ eine R²⁰R²¹N- Gruppe ist, wobei R²⁰ und R²¹ unabhängig voneinander ein Wasserstoffatom, einen C₁ - C₅ - Alkylrest, eine Gruppe C(O)R²², worin R²² einen gegebenenfalls substituierten, gerad- oder verzweigtkettigen, gesättigten oder bis zu dreifach ungesättigten, gegebenenfalls teilweise oder vollständig halogenierten Kohlenwasserstoffrest mit bis zu 10 Kohlenstoffatomen, einen gegebenenfalls substituierten C₃ - C₇ - Cycloalkylrest, einen gegebenenfalls substituierten C₄ - C₁₅ - Cycloalkylalkylrest oder einen gegebenenfalls substituierten Aryl-, Heteroaryl- oder Aralkylrest darstellt, oder, zusammen mit dem N-Atom, einen Polymethyleniminorest mit 4 bis 6 C-Atomen oder einen Morpholinorest, bedeuten;
R³ eine Gruppe R¹⁸-O-, R¹⁹SO₂-O- oder -O-C(O)R²², mit R¹⁹ und R²² jeweils in der unter R² angegebenen Bedeutung, wobei für R¹⁸ ein Wasserstoffatom, eine Trifluormethylgruppe, Isopropyl, Butyl, Isobutyl, Tert-butyl, Pentyl, Isopentyl, Neopentyl, Heptyl, Hexylrest oder ein Aryl-, Heteroaryl- oder Aralkylrest stehen kann;
R⁶ und R⁷ je ein Wasserstoffatom oder zusammen eine zusätzliche Bindung;
R^{6'} und R^{7'} unabhängig voneinander ein Wasserstoffatom, ein Halogenatom, eine Gruppe R¹⁸-O-, R¹⁹SO₂-O- oder -R²², mit R¹⁸, R¹⁹ und R²² jeweils in der unter R² angegebenen Bedeutung;
R⁸ einen gerad- oder verzweigtkettigen, gegebenenfalls teilweise oder vollständig halogenierten Alkyl- oder Alkenylrest mit bis zu 5 Kohlenstoffatomen, einen Ethinyl- oder Prop-1-inylrest;
R⁹ ein Wasserstoffatom, einen gerad- oder verzweigtkettigen, gesättigten oder ungesättigten Kohlenwasserstoffrest mit bis zu 5 Kohlenstoffatomen, oder gemeinsam mit R¹¹ eine zusätzliche Bindung;
R¹¹ ein Wasserstoffatom oder zusammen mit R⁹ oder zusammen mit R¹² eine zusätzliche Bindung;
R^{11'} ein Wasserstoffatom, ein Halogenatom, einen gesättigten oder ungesättigten, gegebenenfalls teilweise oder vollständig halogenierten Kohlenwasserstoffrest, der eine maximale lineare Kettenlänge von 4 Kohlenstoffatomen aufweist, oder eine Gruppe -X- R^{18'}, worin X ein Sauerstoff- oder Schwefelatom ist und R^{18'} ein Alkylrest mit 1 bis 3 Kohlenstoffatomen ist;
R¹² ein Wasserstoffatom oder zusammen mit R¹ eine zusätzliche Bindung;
R¹⁴ ein Wasserstoffatom oder zusammen mit R¹⁵ eine zusätzliche Bindung;
R¹⁵ ein Wasserstoffatom oder zusammen mit R¹⁴ oder zusammen mit R¹⁶ eine zusätzliche Bindung;
R¹⁶ ein Wasserstoffatom oder zusammen mit R¹⁵ eine zusätzliche Bindung;
R^{15'} und R^{16'} unabhängig voneinander ein Wasserstoffatom, ein Halogenatom, eine Gruppe R¹⁸-O-, R¹⁹SO₂-O- oder-R²², mit R¹⁸, R¹⁹ und R²² jeweils In der unter R² angegebenen Bedeutung;
R¹⁷ und R^{17'} je ein Wasserstoffatom; ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder-O-C(O)R²²; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R² angegebenen Bedeutung; oder
R¹⁷ und R^{17'} gemeinsam eine Gruppe =CR²³R²⁴, worin R²³ und R²⁴ unabhängig voneinander ein Wasserstoffatom und ein Halogenatom darstellen, oder gemeinsam ein Sauerstoffatom;
bedeuten,
ausgenommen der Verbindungen der allgemeinen Formel I', worin
R³ eine Hydroxy oder Acetyloxygruppe ist, und gleichzeitig
R² ein Wasserstoffatom,
R⁶, R^{6'}, R⁷ und R^{7'} jeweils ein Wasserstoffatom;
R⁸ eine Methylgruppe,
R⁹ ein Wasserstoffatom oder
R⁹ und R¹¹ zusammen eine zusätzliche Bindung,
R^{11'} und R¹² jeweils ein Wasserstoffatom,
R¹⁴, R¹⁵, R^{15'}, R¹⁶ und R^{16'} jeweils ein Wasserstoffatom, sowie
R¹⁷ und R^{17'} für eine β-Hydroxygruppe und ein Wasserstoffatom; für eine β-(2-Bromacetyl)oxygruppe und ein Wasserstoffatom; für eine β-Acetylgruppe und ein Wasserstoffatom; oder
R¹⁷ und R^{17'} gemeinsam ein Sauerstoffatom darstellen, worin: ein C₄₋₁₅-Cycloalkylalkylrest 3 bis 7 Kohlenstoffatome im Cycloalkyteil und bis zu 8 Kohlenstoffatomen im Alkylteil aufweist;
ein Arylrest sich um einen Phenyl-, 1- oder 2-Naphthyl- oder Heteroarylrest handelt;
ein Aralkylrest sich um einen Rest handelt, der im Ring bis zu 14 C-Atome und in der Alkylkette 1 bis 8 C-Atome enthält;
die Alkylgruppen bzw. Kohlenwasserstoffreste können teilweise oder vollständig fluoriert oder substituiert sein durch 1-5 Halogenatome, Hydroxygruppen oder C₁₋₄-Alkoxygruppen;
eine oder mehrere Hydroxygruppen an den C-Atomen 3,16 und 17 können mit einer aliphatischen, gerad- oder verzweigtkettigen, gesättigten oder ungesättigten C₁₋₁₄-Mono- oder Polycarbonsäure oder einer aromatischen Carbonsäure oder mit einer α- oder β-Aminosäure verestert sein.

2. Estratriene der allgemeinen Formel **I** nach Anspruch 1,
worin
R² ein Wasserstoff- oder Halogenatom oder eine Hydroxygruppe;
R³ eine Gruppe R¹⁸-O-, R¹⁹SO₂-O- oder -O-C(O)R²², mit R¹⁹ und R²² jeweils in der unter R² in Anspruch 1 angegebenen Bedeutung, wobei für R¹⁸ ein Wasserstoffatom, eine Trifluormethylgruppe, Isopropyl, Butyl, Isobutyl, Tert-butyl, Pentyl, Isopentyl, Neopentyl, Heptyl or Hexylrest oder ein Aryl- oder Aralkylrest stehen kann;
R⁶ und R⁷ je ein Wasserstoffatom;
R^{6'} ein Wasserstoffatom, ein Hydroxygruppe, eine Gruppe R²² in der unter R² in Anspruch 1 angegebenen Bedeutung;
R^{7'} ein Wasserstoffatom, ein Halogenatom, eine Gruppe R¹⁸-O-, R¹⁹SO₂-O-oder-R²², mit R¹⁸, R¹⁹ und R²² jeweils in der unter R² in Anspruch 1 angegebenen Bedeutung,
R⁸ einen gerad- oder verzweigtkettigen, gegebenenfalls teilweise oder vollständig halogenierten Alkyl- oder Alkenylrest mit bis zu 5 Kohlenstoffatomen, einen Ethinyl- oder Prop-1-inylrest;
R⁹ ein Wasserstoffatom oder zusammen mit R¹¹ eine zusätzliche Bindung;
R¹¹ ein Wasserstoffatom oder zusammen mit R⁹ eine zusätzliche Bindung;
R^{11'} ein Wasserstoffatom, ein Halogenatom, einen gesättigten oder ungesättigten, gegebenenfalls teilweise oder vollständig halogenierten Kohlenwasserstoffrest, der eine maximale lineare Kettenlänge von 4 Kohlenstoffatomen aufweist, oder eine Gruppe -X- R^{18'}, worin X ein Schwefelatom ist und R^{18'} ein Alkylrest mit 1 bis 3 Kohlenstoffatomen ist;
R¹², R¹⁴, R¹⁵, R¹⁵' und R¹⁶ jeweils ein Wasserstoffatom;
R^{16'} ein Wasserstoffatom, ein Halogenatom, eine Gruppe R¹⁸-O-, R¹⁹SO₂-O-oder-R²², mit R¹⁸, R¹⁹ und R²² jeweils in der unter R² in Anspruch 1 angegebenen Bedeutung;
R¹⁷ und R^{17'} jeweils ein Wasserstoffatom; ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder-O-C(O)R²²; eine Gruppe R¹⁸-O- und eine Gruppe R¹⁸-; eine Gruppe R¹⁸-O- und eine Gruppe -O-C(O)R²², In allen vorstehenden Fällen mit R¹⁸, R¹⁹ und R²² jeweils in der unter R² in Anspruch 1 angegebenen Bedeutung; sowie
R¹⁷ und R^{17'} gemeinsam eine Gruppe =CR²³R²⁴, worin R²³ und R²⁴ unabhängig voneinander ein Wasserstoffatom und ein Halogenatom darstellen, oder gemeinsam ein Sauerstoffatom;
bedeuten.

3. Estratriene der allgemeinen Formel I nach Anspruch 1,
worin
R² ein Wasserstoff- oder Fluoratom oder eine Hydroxygruppe,
R³ eine Gruppe R¹⁸-O-, R¹⁹SO₂-O- oder -O-C(O)R²², mit R¹⁹ und R²² jeweils in der unter R² in Anspruch 1 angegebenen Bedeutung, wobei für R¹⁸ ein Wasserstoffatom, eine Trifluormethylgruppe, Isopropyl, Butyl, Isobutyl, Tert-butyl, Pentyl, Isopentyl, Neopentyl, Heptyl or Hexylrest oder ein Aryl- oder Aralkylrest stehen kann;
R⁶ und R⁷ jeweils ein Wasserstoffatom;
R^{6'} ein Wasserstoffatom oder eine Hydroxygruppe,
R^{7'} ein Wasserstoffatom, ein Fluor- oder Chloratom, eine Gruppe R¹⁸-O-, R¹⁹SO₂-O- oder -R²², mit R¹⁸, R¹⁹ und R²² jeweils in der unter R² in Anspruch 1 angegebenen Bedeutung;
R⁸ einen gerad- oder verzweigtkettigen, gegebenenfalls teilweise oder vollständig fluorierten Alkyl- oder Alkenylrest mit bis zu 5 Kohlenstoffatomen, einen Ethinyl- oder Prop-1-inylrest;
R⁹ unabhängig voneinander ein Wasserstoffatom oder zusammen mit R¹¹ eine zusätzliche Bindung;
R^{11'} ein Wasserstoffatom, ein Fluor- oder Chloratom, eine gesättigte, gerad- oder verzweigtkettige C₁-C₄-Alkylgruppe, eine Gruppe -X- R^{18'}, worin X ein Schwefelatom ist und R^{18'} ein Alkylrest mit 1 bis 3 Kohlenstoffatomen ist;
R¹², R¹⁴, R¹⁵, R^{15'} und R¹⁶ jeweils ein Wasserstoffatom:
R^{16'} ein Wasserstoffatom, ein Fluor- oder Chloratom oder eine Gruppe R¹⁸-O oder -R²², mit R¹⁸ und R²² jeweils in der unter R² in Anspruch 1 angegebenen Bedeutung;
R¹⁷ und R^{17'} jeweils ein Wasserstoffatom; ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder -O-C(O)R²²; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R² in Anspruch 1 angegebenen Bedeutung; oder
R¹⁷ und R^{17'} gemeinsam eine Gruppe =CR²³R²⁴, worin R²³ und R²⁴ unabhängig voneinander ein Wasserstoffatom und ein Halogenatom darstellen, oder gemeinsam ein Sauerstoffatom;
bedeuten.

4. Estratriene der allgemeinen Formel I nach Anspruch 1,
worin
R^{6'}, R^{7'}, R⁹, R¹¹, R¹⁴, R¹⁵, R^{15'} und R¹⁶ jeweils für ein Wasserstoffatom oder R^{6'}, R^{7'}, R¹⁴, R¹⁵, R^{15'} und R¹⁶ jeweils für ein Wasserstoffatom sowie R⁹ und R¹¹ zusammen für eine zusätzliche Bindung stehen und alle anderen Substituenten die in Anspruch 1 angegebenen Bedeutungen haben.

5. Estratriene der allgemeinen Formel I nach Anspruch 1,
die in der Position 9(11), 14(15) oder 15(16) eine Doppelbindung oder in den Positionen 9(11) und 14(15) bzw. 15(16) zwei Doppelbindungen aufweisen.

6. Estratriene der allgemeinen Formel I nach Anspruch 1,
worin
R¹⁷und R^{17'} eine Gruppe R¹⁸-O- und eine Gruppe R¹⁸-; eine Gruppe R¹⁸- und eine Gruppe -O-C(O)R²², mit R¹⁸ und R²² jeweils in der unter R² in Anspruch 1 angegebenen Bedeutung ist.

7. Estratriene der allgemeinen Formel I nach Anspruch 6,
worin
R¹⁷und R^{17'} eine Hydroxygruppe und ein Wasserstoffatom, eine C₁- C₄-Alkyl- oder C₂ - C₄-Alkinylgruppe
Ist.

8. Estratriene der allgemeinen Formel I nach Anspruch 7,
worin
R¹⁷ und R^{17'} eine Hydroxygruppe und ein Wasserstoffatom, eine Methyl-, Ethinyl- oder Prop-1-inylgruppe
ist.

9. Estratriene der allgemeinen Formel I nach Anspruch 1,
worin
R^{16'} für eine Gruppe R¹⁸-O- oder R¹⁹SO₂-O- mit R¹⁸ und R¹⁹ jeweils in der unter R² in Anspruch 1 angegebenen Bedeutung steht, R¹⁷ und R^{17'} je für ein Wasserstoffatom steht sowie alle anderen Substituenten die in der allgemeinen Formel I angegebenen Bedeutungen haben können.

10. Estratriene, nämlich
8β-Vinyl-estra-1,3,5(10),9(11)-tetraen-3,17β-diol
3-Methoxy-8β-vinyl-estra-1,3,5(10),9(11)-tetraen-17β-ol
8β-(2',2'-Difluorvinyl)-estra-1,3,5(10),9(11)-tetraen-3,17β-diol
8β-(2',2'-Difluorovinyl-3-methoxy-estra-1,3,5(10),9(11)-tetraen-17β-ol
8β-Vinyl-estra-1,3.5(10)-trien-3,17β-diol
3-Methoxy-8β-vinyl-estra-1,3,5(10)-trien-17β-ol
8β-(2',2'-Difluorvinyl)-estra-1,3,5(10)-trien-3,17β-diol
8β-(2',2'-Difluorovinyl)-3-methoxy-estra-1,3,5(10)trien-17β-ol
8β-Ethyl-estra-1,3,5(10)-trien-3,17β-diol
8β-Ethyl-3 methoxy-estra-1,3,5,(10)-trien-17β-ol
8β-Vinyl-estradiol-3-sulfamat
8p-Vinyl-estradiol-3,17-disulfamat
8β-Vinyl-estradiol-3-(N-acetyl)-sulfamat
8β-Vinyl-estron-3-suifamat
8β-Vinyl-estron-3-acetat
8β-Vinyl-estriol
8β-Vinyl-estriol-3-sulfamat
8β-Methyl-estron-3-sulfamat
Sβ-Methyl-estriol
8β-(Prop-(Z)-enyl)-estradiol
8β-(n-Propyl)-estradiol
8β-Ethinyl-estradiol
17α-Ethinyl-8β-vinyl-estra-1,3,5(10)-trien-3,17β-diol
17α-Methyl-8β-vinyl-estra-1,3,5(10)-trien-3,17β-diol
16α-Fluor-8β-methyl-estra-1,3,5(10)-trien-3,17β-diol
8β-Vinyl-estra-1,3,5(10)-trien-3,1α-diol
8β-Methyl-estra-1,3,5(10)-trien-3,17α-diol
8β-Vinyl-estradiol-diacetat
8β-Methyl-estradiol-diacetat
8β-Vinyl-estradiol-17-valerianat
17β-Acetoxy-8β-vinyl-estra-1,3,5(10)-trien-3-ol

11. Verwendung von 8β-substituierten Estra-1,3,5(10)-trienderivaten der allgemeinen Formel I' 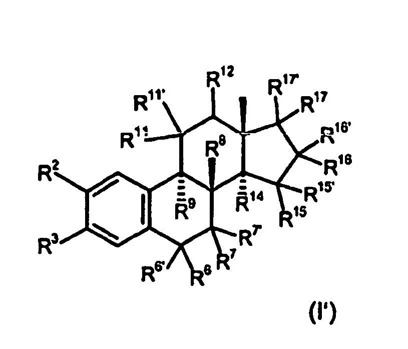 worin
R² ein Wasserstoffatom, ein Halogenatom;
ein Rest R¹⁸- oder R¹⁸-O-, wobei R¹⁸ ein Wasserstoffatom oder einen gerad- oder verzwelgtkettlgen, gesättigten oder ungesättigten Kohlenwasserstoffrest mit bis zu 6 Kohlenstoffatomen, eine
Trifluormethylgruppe;
eine Gruppe R¹⁹SO₂-O-, worin R¹⁹ eine R²⁰R²¹N - Gruppe ist, wobei R²⁰ und R²¹ unabhängig voneinander ein Wasserstoffatom, einen C₁ - C₅ - Alkylrest, eine Gruppe C(O)R²², worin R²² einen gegebenenfalls substituierten, gerad- oder verzweigtkettigen, gesättigten oder bis zu dreifach ungesättigten, gegebenenfalls teilweise oder vollständig halogenierten Kohlenwasserstoffrest mit bis zu 10 Kohlenstoffatomen, einen gegebenenfalls substituierten C₃ - C₇ - Cycloalkylrest, einen gegebenenfalls substituierten C₄ - C₁₅ - Cycloalkylalkylrest oder einen gegebenenfalls substituierten Aryl-, Heteroaryl- oder Aralkylrest darstellt, oder, zusammen mit dem N-Atom, einen Polymethyleniminorest mit 4 bis 6 C-Atomen oder einen Morpholinorest, bedeuten;
R³ eine Gruppe R¹⁸-O-, R¹⁹SO₂-O- oder-O-C(O)R²², mit R¹⁸, R¹⁹ und R²² jeweils in der unter R² angegebenen Bedeutung, wobei für R¹⁸ zusätzlich ein Aryl-, Heteroaryl- oder Aralkylrest stehen kann;
R⁶ und R⁷ je ein Wasserstoffatom oder zusammen eine zusätzliche Bindung;
R^{6'} und R^{7'} unabhängig voneinander ein Wasserstoffatom, ein Halogenatom, eine Gruppe R¹⁸-O-, R¹⁹SO₂-O- oder-R²², mit R¹⁸, R¹⁹ und R²² jeweils in der unter R² angegebenen Bedeutung;
R⁸ einen gerad- oder verzweigtkettigen, gegebenenfalls teilweise oder vollständig halogenierten Alkyl- oder Alkenylrest mit bis zu 5 Kohlenstoffatomen, einen Ethinyl- oder Prop-1-inylrest;
R⁹ ein Wasserstoffatom, einen gerad- oder verzweigtkettigen, gesättigten oder ungesättigten Kohlenwasserstoffrest mit bis zu 5 Kohlenstoffatomen, oder gemeinsam mit R¹¹ eine zusätzliche Bindung;
R¹¹ ein Wasserstoffatom oder zusammen mit R⁹ oder zusammen mit R¹² eine zusätzliche Bindung;
R^{11'} ein Wasserstoffatom, ein Halogenatom, einen gesättigten oder ungesättigten, gegebenenfalls teilweise oder vollständig halogenierten Kohlenwasserstoffrest, der eine maximale lineare Kettenlänge von 4 Kohlenstoffatomen aufweist, oder eine Gruppe -X- R^{18'}, worin X ein Sauerstoff- oder Schwefelatom ist und R^{18'} ein Alkylrest mit 1 bis 3 Kohlenstoffatomen ist;
R¹² ein Wasserstoffatom oder zusammen mit R¹¹ eine zusätzliche Bindung;
R¹⁴ ein Wasserstoffatom oder zusammen mit R¹⁵ eine zusätzliche Bindung;
R¹⁵ ein Wasserstoffatom oder zusammen mit R¹⁴ oder zusammen mit R¹⁶ eine zusätzliche Bindung;
R¹⁶ ein Wasserstoffatom oder zusammen mit R¹⁵ eine zusätzliche Bindung;
R^{15'} und R^{16'} unabhängig voneinander ein Wasserstoffatom, ein Halogenatom, eine Gruppe R¹⁸-O-, R¹⁹SO₂-O-oder-R²², mit R¹⁸, R¹⁹ und R²² jeweils in der unter R² angegebenen Bedeutung;
R¹⁷ und R^{17'} je ein Wasserstoffatom; ein Wasserstoffatom und ein Halogenatom; ein Wasserstoffatom und eine Benzyloxygruppe ; ein Wasserstoffatom und eine Gruppe R¹⁹SO₂-O-; eine Gruppe R¹⁸ und eine Gruppe -C(O)R²² oder-O-C(O)R²²; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R² angegebenen Bedeutung; oder
R¹⁷ und R^{17'} gemeinsam eine Gruppe =CR²³R²⁴, worin R²³ und R²⁴ unabhängig voneinander ein Wasserstoffatom und ein Halogenatom darstellen, oder gemeinsam ein Sauerstoffatom;
bedeuten worin: ein C₄₋₁₅-Cycloalkylalkylrest 3 bis 7 Kohlenstoffatome im Cycloalkyteil und bis zu 8 Kohlenstoffatomen im Alkylteil aufweist:
ein Arylrest sich um einen Phenyl-, 1- oder 2-Naphthyl- oder Heteroarylrest handelt;
ein Aralkylrest sich um einen Rest handelt, der im Ring bis zu 14 C-Atome und in der Alkylkette 1 bis 8 C-Atome enthält;
die Alkylgruppen bzw. Kohlenwasserstoffreste können teilweise oder vollständig fluoriert oder substituiert sein durch 1-5 Halogenatome, Hydroxygruppen oder C₁₋₄-Alkoxygruppen;
eine oder mehrere Hydroxygruppen an den C-Atomen 3, 16 und 17 können mit einer aliphatischen, gerad- oder verzweigtkettigen, gesättigten oder ungesättigten C₁₋₁₄-Mono- oder Polycarbonsäure oder einer aromatischen Carbonsäure oder mit einer α- oder β-Aminosäure verestert sein,
als Estrogene zur Herstellung eines Arzneimittels zur Behandlung von peri- und postmenopausalen Beschwerden.

12. Verwendung von Estratrienen der allgemeinen Formel I' nach Anspruch 11,
worin
R² ein Wasserstoff- oder Halogenatom oder eine Hydroxygruppe;
R³ eine Gruppe R¹⁸-O-, R¹⁹SO₂-O- oder-O-C(O)R²², mit R¹⁸, R¹⁹ und R²² jeweils in der unter R² in Anspruch 11 angegebenen Bedeutung, wobei für R¹⁸ zusätzlich ein Aryl- oder Aralkylrest stehen kann;
R⁶ und R⁷ je ein Wasserstoffatom;
R^{6'} ein Wasserstoffatom, ein Hydroxygruppe, eine Gruppe R²² in der unter R² in Anspruch 11 angegebenen Bedeutung:
R^{7'} ein Wasserstoffatom, ein Halogenatom, eine Gruppe R¹⁸-O-, R¹⁹SO₂-O- oder-R²², mit R¹⁸, R¹⁹ und R²² jeweils in der unter R² in Anspruch 11 angegebenen Bedeutung,
R⁸ einen gerad- oder verzweigtkettigen, gegebenenfalls teilweise oder vollständig halogenierten Alkyl- oder Alkenylrest mit bis zu 5 Kohlenstoffatomen, einen Ethinyl- oder Prop-1-inylrest;
R⁹ ein Wasserstoffatom oder zusammen mit R¹¹ eine zusätzliche Bindung;
R¹¹ ein Wasserstoffatom oder zusammen mit R⁹ eine zusätzliche Bindung;
R^{11'} ein Wasserstoffatom, ein Halogenatom, einen gesättigten oder ungesättigten, gegebenenfalls teilweise oder vollständig halogenierten Kohlenwasserstoffrest, der eine maximale lineare Kettenlänge von 4 Kohlenstoffatomen aufweist, oder eine Gruppe -X- R¹⁸', worin X ein Schwefelatom ist und R^{18'} ein Alkylrest mit 1 bis 3 Kohlenstoffatomen ist;
R¹², R¹⁴, R¹⁵, R^{15'} und R¹⁶ jeweils ein Wasserstoffatom:
R^{16'} ein Wasserstoffatom, ein Halogenatom, eine Gruppe R¹⁸-O-, R¹⁹SO₂-O- oder -R²², mit R¹⁸, R¹⁹ und R²² jeweils in der unter R² in Anspruch 11 1 angegebenen Bedeutung;
R¹⁷ und R^{17'} jeweils ein Wasserstoffatom; ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder-O-C(O)R²²; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R² in Anspruch 11 angegebenen Bedeutung; sowie
R¹⁷ und R^{17'} gemeinsam eine Gruppe =CR²³R²⁴, worin R²³ und R²⁴ unabhängig voneinander ein Wasserstoffatom und ein Halogenatom darstellen, oder gemeinsam ein Sauerstoffatom;
bedeuten.

13. Verwendung von Estratrienen der allgemeinen Formel I' nach Anspruch 11,
worin
R² ein Wasserstoff- oder Fluoratom oder eine Hydroxygruppe,
R³ eine Gruppe R¹⁸-O-, R¹⁹SO₂-O- oder-O-C(O)R²², mit R¹⁸, R¹⁹ und R²² jeweils in der unter R² in Anspruch 11 angegebenen Bedeutung, wobei für R¹⁸ zusätzlich ein Aryl- oder Aralkylrest stehen kann;
R⁶ und R⁷ jeweils ein Wasserstoffatom;
R^{6'} ein Wasserstoffatom oder eine Hydroxygruppe,
R^{7'} ein Wasserstoffatom, ein Fluor- oder Chloratom, eine Gruppe R¹⁸-O-, R¹⁹SO₂-O- oder -R²², mit R¹⁸, R¹⁹ und R²² jeweils in der unter R² in Anspruch 11 angegebenen Bedeutung;
R⁸ einen gerad- oder verzweigtkettigen, gegebenenfalls teilweise oder vollständig fluorierten Alkyl- oder Alkenylrest mit bis zu 5 Kohlenstoffatomen, einen Ethinyl- oder Prop-1-inylrest;
R⁹ unabhängig voneinander ein Wasserstoffatom oder zusammen mit R¹¹ eine zusätzliche Bindung;
R^{11'} ein Wasserstoffatom, ein Fluor- oder Chloratom, eine gesättigte, gerad- oder verzweigtkettige C₁-C₄-Alkylgruppe, eine Gruppe -X- R^{18'}, worin X ein Schwefelatom ist und R^{18'} ein Alkylrest mit 1 bis 3 Kohlenstoffatomen ist;
R¹², R¹⁴, R¹⁵, R^{15'} und R¹⁶ jeweils ein Wasserstoffatom;
R^{16'} ein Wasserstoffatom, ein Fluor- oder Chloratom oder eine Gruppe R¹⁸-O oder-R²², mit R¹⁸ und R²² jeweils in der unter R² in Anspruch 11 angegebenen Bedeutung;
R¹⁷ und R^{17'} jeweils ein Wasserstoffatom; ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder-O-C(O)R²²; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R² in Anspruch 11 angegebenen Bedeutung; oder
R¹⁷ und R^{17'} gemeinsam eine Gruppe =CR²³R²⁴, worin R²³ und R²⁴ unabhängig voneinander ein Wasserstoffatom und ein Halogenatom darstellen, oder gemeinsam ein Sauerstoffatom;
bedeuten.

14. Verwendung von Estratrienen der allgemeinen Formel I' nach Anspruch 11,
worin
R^{6'}, R^{7'}, R⁹, R¹¹, R¹⁴, R¹⁵, R^{15'} und R¹⁶ jeweils für ein Wasserstoffatom oder R^{6'}, R^{7'}, R¹⁴, R¹⁵, R^{15'} und R¹⁶ jeweils für ein Wasserstoffatom sowie R⁹ und R¹¹ zusammen für eine zusätzliche Bindung stehen und alle anderen Substituenten die in Anspruch 1 angegebenen Bedeutungen haben.

15. Verwendung von Estratrienen der allgemeinen Formel I' nach Anspruch 11, die
in der Position 9(11), 14(15) oder 15(16) eine Doppelbindung oder in den Positionen
9(11) und 14(15) bzw. 15(16) zwei Doppelbindungen aufweisen.

16. Verwendung von Estratrienen der allgemeinen Formel l' nach Anspruch 11,
worin
R¹⁷ und R^{17'} eine Gruppe R¹⁸-O- und eine Gruppe R¹⁸-; eine Gruppe R¹⁸- und eine Gruppe -O-C(O)R²², mit R¹⁸ und R²² jeweils in der unter R² in Anspruch 11 angegebenen Bedeutung ist.

17. Verwendung von Estratrienen der allgemeinen Formel I' nach Anspruch 16,
worin
R¹⁷ und R^{17'} eine Hydroxygruppe und ein Wasserstoffatom, eine C₁ - C₄-Alkyl- oder C₂ - C₄-Alkinylgruppe
ist.

18. Verwendung von Estratrienen der allgemeinen Formel I' nach Anspruch 17,
worin
R¹⁷ und R^{17'} eine Hydroxygruppe und ein Wasserstoffatom, eine Methyl-, Ethinyl- oder Prop-1-inylgruppe
ist.

19. Verwendung von Estratrienen der allgemeinen Formel I' nach Anspruch 11,
worin
R^{16'} für eine Gruppe R¹⁸-O- oder R¹⁹SO₂-O- mit R¹⁸ und R¹⁹ jeweils in der unter R² in Anspruch 11 angegebenen Bedeutung steht, R¹⁷ und R^{17'} je für ein Wasserstoffatom steht sowie alle anderen Substituenten die in der allgemeinen Formel I angegebenen Bedeutungen haben können.

20. Verwendung von Estratrienen der allgemeinen Formel I' nach Anspruch 11, ausgewählt aus der Gruppe der Verbindungen
8β-Methyl-estra-1,3,5(10),9(11)-tetraen-3,17β-diol
3-Methoxy-8β-methyl-estra-1,3,5(10),9(11)-tetraen-17β-ol
8β-Methyl-estra-1,3,5(10)-trien-3,17β-diol
3-Methoxy-8β-methyl-estra-1,3,5(10)-trien-17β-ol
8β-Vinyl-estra-1,3,5(10),9(11)-tetraen-3,17β-diol
3-Methoxy-8β-vinyl-estra-1,3,5(10),9(11)-tetraen-17β-ol
8β-(2',2'-Difluorvinyl)-estra-1,3,5(10),9(11)-tetraen-3,17β-diol
8β-(2',2'-Difluorvinyl)-3-methoxy-estra-1,3,5(1 0),9(11)-tetraen-17β-ol
8β-Vinyl-estra-1,3,5(10)-trien-3,17β-diol
3-Methoxy-8β-vinyl-estra-1,3,5(10)-trien-17β-ol
8β-(2',2'-Difluorvinyl)-estra-1,3,5(10)-trien-3,17β-diol
8β-(2',2'-Difluorvinyl)-3-methoxy-estra-1,3,5(10)-trien-17β-ol
8β-Ethyl-estra-1,3,5(10)-trien-3,17β-diol
8β-Ethyl-3-methoxy-estra-1,3,5(10)-trien-17β-ol
8β-Vinyl-estradiol-3-sulfamat
8β-Vinyl-estradiol-3,17-disulfamat
8β-Vinyl-estradiol-3-(N-acetyl)-sulfamat
8β-Vinyl-estron-3-sulfamat
8β-Vinyl-estron-3-acetat
8β-Vinyl-estriol
8β-Vinyl-estriol-3-sulfamat
8β-Methyl-estron-3-sulfamat
8β-Methyl-estriol
8β-(Prop-(Z)-enyl)-estradiol
8β-(n-Propyl)-estradiol
8β-Ethinyl-estradiol
17α-Ethinyl-8β-vinyl-estra-1,3,5(10)-trien-3,17β-diol
17α-Methyl-8β-vinyl-estra-1,3,5(10)-trien-3,17β-diol
16α-Fluor-8β-methyl-estra-1,3,5(10)-trien-3,17β-diol
8β-Vinyl-estra-1,3,5(10)-trien-3,17α-diol
8β-Methyl-estra-1,3,5(10)-trien-3,17α-diol
8β-Vinyl-estradiol-diacetat
8β-Methyl-estradiol-diacetat
8β-Vinyl-estradiol-17-valerianat
17β-Acetoxy-8β-vinyl-estra-1.3,5(1 0)-trien-3-ol

21. Verwendung einer Verbindung der allgemeinen Formel I' definiert nach Anspruch 11 bis 20 zur Herstellung eines Arzneimittels zur Behändlung von peri- und postandropausalen Beschwerden.

22. Verwendung nach Anspruch 11 zur Vorbeugung gegen und Behandlung von Hitzewallungen, Schlafstörungen, Reizbarkeit, Stimmungsschwankungen, Inkontinenz, Vaginalatrophie und hormondefizienzbedingter Gemütsericrankungen.

23. Verwendung nach Anspruch 22 zur Vorbeugung und Behandlung von Erkrankungen im Urogenitaltrakt.

24. Verwendung einer Verbindung der allgemeinen Formel I' definiert nach Anspruch 11 bis 20 zur Herstellung eines Arzneimittels zur Vorbeugung und Therapie von Magen- und Darmerkrankungen.

25. Verwendung nach Anspruch 24 zur Vorbeugung und Therapie von Ulcera und hemoragischen Diathesen im Magendarmtrakt.

26. Verwendung nach Anspruch 24 zur Vorbeugung und Therapie von Neoplasien.

27. Verwendung einer Verbindung der allgemeinen Formel I' definiert nach Anspruch 11 bis 20 zur Herstellung eines Arzneimittels für die in-vitro Behandlung der männlichen Infertilität.

28. Verwendung einer Verbindung der allgemeinen Formel I' definiert nach Anspruch 11 bis 20 zur Herstellung eines Arzneimittels für die in-vivo Behandlung der männlichen Infertilität.

29. Verwendung einer Verbindung der allgemeinen Formel I definiert nach Anspruch 1-10 zur Herstellung eines Arzneimittels für die in-vitro Behandlung der weiblichen Infertilität.

30. Verwendung einer Verbindung der allgemeinen Formel I definiert nach Anspruch 1-10 zur Herstellung eines Arzneimittels für die in-vivo Behandlung der weiblichen Infertilität.

31. Verwendung nach Ansprüche 11-20 einer Verbindung der allgemeinen Formel I' definiert nach Anspruch 11 bis 20 für die Hormonersatz-Therapie (HRT).

32. Verwendung einer Verbindung der allgemeinen Formel I' definiert nach Anspruch 11 bis 20 zur Herstellung eines Arzneimittels für die Therapie von hormondefizienz bedingten Beschwerden bei chirurgisch, medikamentös oder anders bedingter ovarieller Dysfunktion.

33. Verwendung nach Ansprüche 11-20 einer Verbindung der allgemeinen Formel I' definiert nach Anspruch 11 bis 20 zur Prophylaxe und Therapie eines hormondefizienzbedingten Knochenmasseverlustes.

34. Verwendung nach Anspruch 33 zur Prophylaxe und Therapie der Osteoporose.

35. Verwendung einer Verbindung der allgemeinen Formel l' definiert nach Anspruch 11 bis 20 zur Herstellung eines Arzneimittels zur Vorbeugung gegen und Therapie von Herzkreislauferkrankungen.

36. Verwendung einer Verbindung der allgemeinen Formel l' definiert nach Anspruch 11 bis 20 zur Herstellung eines Arzneimittels zur Vorbeugung gegen und Behandlung von Gefäßerkrankungen.

37. Verwendung nach Anspruch 36 zur Vorbeugung gegen und Behandlung von Atherosklerose.

38. Verwendung nach Anspruch 36 zur Vorbeugung und Behandlung neointimaler Hyperplasien.

39. Verwendung einer Verbindung der allgemeinen Formel I' definiert nach Anspruch 11 bis 20 zur Herstellung eines Arzneimittels zur Vorbeugung und Behandlung hormondefizienzbedingter neurodegenerativer Erkrankungen.

40. Verwendung einer Verbindung der allgemeinen Formel I' definiert nach Anspruch 11 bis 20 zur Herstellung eines Arzneimittels zur Vorbeugung und Behandlung der Alzheimerschen Krankheit sowie hormondefizienzbedingter Beeinträchtigung von Gedächtnis- und Lernfähigkeit.

41. Verwendung einer Verbindung der allgemeinen Formel I' definiert nach Anspruch 11 bis 20 zur Herstellung eines Arzneimittels zur Behandlung von entzündlichen Erkrankungen und Erkrankungen des Immunsystems.

42. Verwendung einer Verbindung der allgemeinen Formel I' definiert nach Anspruch 11 bis 20 zur Herstellung eines Arzneimittels zur Vorbeugung und Behandlung der benignen Prostatahyperplasie (BPH).

43. Pharmazeutische Zusammensetzungen, enthaltend mindestens eine Verbindung gemäß einem der Ansprüche 1 bis 10 sowie einen pharmazeutisch verträglichen Träger.

## Claims

1. 8β-Substituted estra-1,3,5(10)-triene derivatives of general formula I' 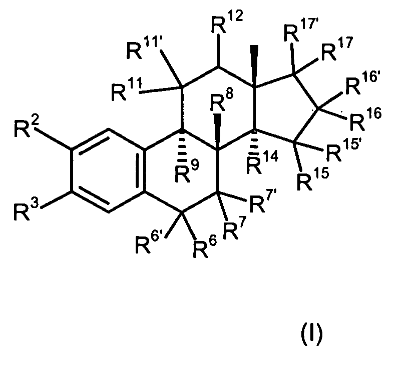 in which
R² means a hydrogen atom, a halogen atom;
a radical R¹⁸- or R¹⁸-O-, whereby R¹⁸ means a hydrogen atom or a straight-chain or branched-chain, saturated or unsaturated hydrocarbon radical with up to 6 carbon atoms, a trifluoromethyl group;
a group R¹⁹SO₂-O-, in which R¹⁹ is an R²⁰R²¹N group, whereby R²⁰ and R²¹, independently of one another, mean a hydrogen atom, a C₁-C₅-alkyl radical, a group C(O)R²², in which R²² represents an optionally substituted, straight-chain or branched-chain, saturated or unsaturated in up to three places, optionally partially or completely halogenated hydrocarbon radical with up to 10 carbon atoms, an optionally substituted C₃-C₇-cycloalkyl radical, an optionally substituted C₄-C₁₅-cycloalkylalkyl radical or an optionally substituted aryl, heteroaryl or aralkyl radical, or, together with the N-atom, means a polymethylenimino radical with 4 to 6 C atoms or a morpholino radical;
R³ means a group R¹⁸-O-, R¹⁹SO₂-O- or -O-C(O)R²², with R¹⁹ and R²² in each case in the meaning that is indicated under R², whereby a hydrogen atom, a trifluoromethyl group, isopropyl, butyl, isobutyl, tert-butyl, pentyl, isopentyl, neopentyl, heptyl or hexyl radical, or an aryl, heteroaryl or aralkyl radical can stand for R¹⁸;
R⁶ and R⁷ each mean a hydrogen atom or together an additional bond;
R^{6'} and R^{7'}, independently of one another, mean a hydrogen atom, a halogen atom, a group R¹⁸-O-, R¹⁹SO₂-O- or -R²², with R¹⁸, R¹⁹ and R²² in each case in the meaning that is indicated under R²;
R⁸ means a straight-chain or branched-chain, optionally partially or completely halogenated alkyl or alkenyl radical with up to 5 carbon atoms, an ethinyl or prop-1-inyl radical;
R⁹ means a hydrogen atom, a straight-chain or branched-chain, saturated or unsaturated hydrocarbon radical with up to 5 carbon atoms, or together with R¹¹ means an additional bond;
R¹¹ means a hydrogen atom or together with R⁹ or together with R¹² means an additional bond;
R^{11'} means a hydrogen atom, a halogen atom, a saturated or unsaturated, optionally partially or completely halogenated hydrocarbon radical, which has a maximum linear chain length of 4 carbon atoms, or a group -X-R^{18'}, in which X is an oxygen or sulfur atom, and R^{18'} is an alkyl radical with 1 to 3 carbon atoms;
R¹² means a hydrogen atom or together with R¹¹ means an additional bond;
R¹⁴ means a hydrogen atom or together with R¹⁵ means an additional bond;
R¹⁵ means a hydrogen atom or together with R¹⁴ or together with R¹⁶ means an additional bond;
R¹⁶ means a hydrogen atom or together with R¹⁵ means an additional bond;
R^{15'} and R^{16'}, independently of one another, mean a hydrogen atom, a halogen atom, a group R¹⁸-O-, R'⁹SO₂-O- or -R²², with R¹⁸, R¹⁹ and R²² in each case in the meaning that is indicated under R²;
R¹⁷ and R^{17'} each mean a hydrogen atom; a hydrogen atom and a halogen atom; a hydrogen atom and a benzyloxy group; a hydrogen atom and a group R¹⁹SO₂-O-; a group R¹⁸ and a group -C(O)R ²² or -O-C(O)R²²; a group R¹⁸ -O- and a group R¹⁸-; a group R¹⁸-O- and a group -O-C(O)R²², in all above cases with R¹⁸, R¹⁹ and R²² in each case in the meaning that is indicated under R²; or
R¹⁷ and R^{17'} together mean a group =CR²³R²⁴, in which R²³ and R²⁴, independently of one another, represent a hydrogen atom and a halogen atom, or together an oxygen atom;
excluding the compounds of general formula I', in which
R³ is a hydroxy, or acetyl group, and simultaneously
R² represents a hydrogen atom,
R⁶, R^{6'}, R⁷ and R^{7'} in each case represent a hydrogen atom;
R⁸ represents a methyl group,
R⁹ represents a hydrogen atom or
R⁹ and R¹¹ together represent an additional bond,
R^{11'} and R¹² in each case represent a hydrogen atom,
R¹⁴, R¹⁵, R^{15'}, R¹⁶ and R^{16'} in each case represent a hydrogen atom, and
R¹⁷ and R^{17'} for a β-hydroxy group and a hydrogen atom; for a β-(2-bromoacetyl)oxy group and a hydrogen atom; for a β-acetyl group and a hydrogen atom; or
R¹⁷ and R^{17'} together represent an oxygen atom,
wherein:
a C₄-C₁₅-cycloalkylalkyl radical has 3 to 7 carbon atoms in the cycioaikyi portion; and the alkyl portion has up to 8 carbon atoms;
an aryl radical is a phenyl, 1- or 2-naphthyl radical or a heteroaryl radical;
an aralkyl radical is a radical that contains in the ring up to 14, preferably 6 to 10 C atoms and in the alkyl chain 1 to 8 C atoms;
the alkyl groups or hydrocarbon radicals can be partially or completely fluorinated or substituted by 1-5 halogen atoms, hydroxy groups or C₁₋₄-alkoxy groups;
one or more hydroxyl groups at C atoms 3, 16 and 17 can be esterified with an aliphatic, straight-chain or branched-chain, saturated or unsaturated C₁₋₁₄-mono- or polycarboxylic acid or an aromatic carboxylic acid or with an α-or β-amino acid.

2. Estratrienes of general formula I according to claim 1, in which
R² means a hydrogen or halogen atom or a hydroxy group;
R³ means a group R¹⁸-O- , R¹⁹SO₂-O- or -O-C(O)R²², with R¹⁹ and R²² in each case in the meaning that is indicated under R² in claim 1, whereby a hydrogen atom, a trifluoromethyl group, isopropyl, butyl, isobutyl, tert-butyl, pentyl, isopentyl, neopentyl, heptyl or hexyl radical, or an aryl or aralkyl radical can stand for R¹⁸;
R⁶ and R⁷ each mean a hydrogen atom;
R^{6'} means a hydrogen atom, a hydroxy group, a group R²² in the meaning that is indicated under R² in claim 1;
R^{7'} means a hydrogen atom, a halogen atom, a group R¹⁸-O-, R¹⁹SO₂-O- or -R²², with R¹⁸, R¹⁹ and R²² in each case in the meaning that is indicated under R² in claim 1;
R⁸ means a straight-chain or branched-chain, optionally partially or completely halogenated alkyl or alkenyl radical with up to 5 carbon atoms, an ethinyl- or prop-1-inyl radical;
R⁹ means a hydrogen atom or together with R¹¹ an additional bond;
R¹¹ means a hydrogen atom or together with R⁹ an additional bond;
R^{11'} means a hydrogen atom, a halogen atom, a saturated or unsaturated, optionally partially or completely halogenated hydrocarbon radical, which has a maximum linear chain length of 4 carbon atoms, or a group -X-R^{18'}, in which X is a sulfur atom, and R^{18'} is an alkyl radical with 1 to 3 carbon atoms;
R¹², R¹⁴, R¹⁵, R^{15'} and R¹⁶ in each case mean a hydrogen atom;
R^{16'} means a hydrogen atom, a halogen atom, a group R¹⁸-O-, R¹⁹SO₂-O- or -R²², with R¹⁸, R¹⁹, and R²² in each case in the meaning that is indicated under R² in claim 1;
R¹⁷ and R^{17'} in each case mean a hydrogen atom; a hydrogen atom and a halogen atom; a hydrogen atom and a benzyloxy group; a hydrogen atom and a group R¹⁹SO₂-O-; a group R¹⁸ and a group -C(O)R²² or -O-C(O)R²²; a group R¹⁸-O- and a group R¹⁸-; a group R¹⁸-O- and a group -O-C(O)R²², in all above cases with R¹⁸, R¹⁹ and R²² in each case in the meaning that is indicated under R² in claim 1; and
R¹⁷ and R^{17'} together mean a group =CR²³R²⁴, in which R²³ and R²⁴, independently of one another, represent a hydrogen atom and a halogen atom, or together an oxygen atom.

3. Estratrienes of general formula I according to claim 1, in which
R² means a hydrogen atom or a fluorine atom or a hydroxy group,
R³ means a group R¹⁸-O-, R¹⁹SO₂-O- or -O-C(O)R²², with R¹⁹, and R²² in each case in the meaning that is indicated under R² in claim 1, whereby hydrogen atom, a trifluoromethyl group, isopropyl, butyl, isobutyl, tert-butyl, pentyl, isopentyl, neopentyl, heptyl or hexyl radical, or an aryl or aralkyl radical can stand for R¹⁸;
R⁶ and R⁷ in each case mean a hydrogen atom;
R^{6'} means a hydrogen atom or a hydroxy group,
R^{7'} means a hydrogen atom, a fluorine or chlorine atom, a group R¹⁸-O-, R¹⁹SO₂-O- or -R²², with R¹⁸, R¹⁹ and R²² in each case in the meaning that is indicated under R² in claim 1;
R⁸ means a straight-chain or branched-chain, optionally partially or completely fluorinated alkyl or alkenyl radical with up to 5 carbon atoms, an ethinyl radical or prop-1-inyl radical;
R⁹, independently of one another, mean a hydrogen atom or together with R¹¹ an additional bond;
R^{11'} means a hydrogen atom, a fluorine or chlorine atom, a saturated, straight-chain or branched-chain C₁-C₄-alkyl group, a group -X-R^{18'}, in which X is a sulfur atom and R^{18'} means a saturated, straight-chain or branched-chain C₁-C₃-alkyl group, a chloromethyl or chloroethyl group;
R¹², R¹⁴, R¹⁵, R^{15'} and R¹⁶ in each case mean a hydrogen atom;
R^{16'} means a hydrogen atom, a fluorine or chlorine atom or a group R¹⁸-O or -R²², with R¹⁸ and R²² in each case in the meaning that is indicated under R² in claim 1;
R¹⁷ and R^{17'} in each case mean a hydrogen atom; a hydrogen atom and a halogen atom; a
hydrogen atom and a benzyloxy group; a hydrogen atom and a group R¹⁹SO₂-O-; a group R¹⁸ and a group -C(O)R ²² or -O-C(O)R²²; a group R¹⁸ -O- and a group R¹⁸-; a group R¹⁸-O- and a group -O-C(O)R²², in all above
cases with R¹⁸, R¹⁹ and R²² in each case in the meaning that is indicated under R² in claim 1; or
R¹⁷ and R^{17'} together mean a group =CR²³R²⁴, in which R²³ and R²⁴, independently of one another, represent a hydrogen atom and a halogen atom, or together an oxygen atom.

4. Estratrienes of general formula I according to claim 1, in which
R^{6'}, R^{7'} , R⁹, R¹¹, R¹⁴, R¹⁵, R^{15'} and R¹⁶ in each case stand for a hydrogen atom or R^{6'} , R^{7'} , R¹⁴, R¹⁵, R¹⁵ and R¹⁶ in each case stand for a hydrogen atom and R⁹ and R¹¹ together stand for an additional bond, and all other substituents have the meanings that are indicated in claim 1.

5. Estratrienes of general formula I according to claim 1, which have a double bond in position 9(11), 14(15) or 15(16) or two double bonds in positions 9(11) and 14(15) or 15(16).

6. Estratrienes of general formula I according to claim 1, in which
R¹⁷ and R^{17'} are a group R¹⁸-O- and a group R¹⁸-; a group R¹⁸- and a group -O-C(O)R²², with R¹⁸ and R²² in each case in the meaning that is indicated under R².

7. Estratrienes of general formula I according to claim 6, in which
R¹⁷ and R^{17'} are a hydroxy group and a hydrogen atom, a C₁-C₄-alkyl group or C₂-C₄-alkinyl group.

8. Estratrienes of general formula I according to claim 7, in which
R¹⁷ and R^{17'} are a hydroxy group and a hydrogen atom, a methyl, ethinyl, or prop-1-inyl group.

9. Estratrienes of general formula I according to claim 1, in which
R^{16'} stands for a group R¹⁸-O- or R¹⁹SO₂-O- with R¹⁸ and R¹⁹ in each case in the meaning that is indicated under R² in claim 1, R¹⁷ and R^{17'} each stand for a hydrogen atom, and all other substituents can have the meanings that are indicated in general formula I.

10. Estratrienes of formula
8β-Vinyl-estra-1,3,5(10),9(11)-tetraene-3,17β-diol
3-methoxy-8β-vinyl-estra-1,3,5(10),9(11)-tetraen-17β-ol
8β-(2',2'-difluorovinyl)-estra-1,3,5(10),9(11)-tetraene-3,17β-diol
8β-(2',2'-difluorovinyl)-3-methoxy-estra-1,3,5(10),9(11)-tetraen-17β-ol
8β-vinyl-estra-1,3,5(10)-triene-3,17β-diol
3-methoxy-8β-vinyl-estra-1,3,5(10)-trien-17β-ol
8β-(2',2'-difluorovinyl)-estra-1,3,5(10)-triene-3,17β-diol
8β-(2',2'-difluorovinyl)-3-methoxy-estra-1,3,5(10)-trien-17β-ol
8β-ethyl-estra-1,3,5(10)-triene-3,17β-diol
8β-ethyl-3-methoxy-estra-1,3,5(10)-trien-17β-ol
8β-vinyl-estradiol-3-sulfamate
8β-vinyl-estradiol-3,17-disulfamate
8β-vinyl-estradiol-3-(N-acetyl)-sulfamate
8β-vinyl-estrone-3-sulfamate
8β-vinyl-estron-3-acetate
8β-vinyl-estriol
8β-vinyl-estriol-3-sulfamate
8β-methyl-estrone-3-sulfamate
8β-methyl-estriol
8β-(prop-(Z)-enyl)-estradiol
8β-(n-propyl)-estradiol
8β-ethinyl-estradiol
17α-ethinyl-8β-vinyl-estra-1,3,5(10)-triene-3,17β-diol
17α-methyl-8β-vinyl-estra-1,3,5,(10)-triene-3,17β-diol
16α-fluoro-8β-methyl-estra-1,3,5(10)-triene-3,17β-diol
8β-vinyl-estra-1,3,5(10)-triene-3,17α-diol
8β-methyl-estra-1,3,5(10)-triene-3,17α-diol
8β-vinyl-estradiol-diacetate
8β-methyl-estradiol-diacetate
8β-vinyl-estradiol-17-valerianate
17β-acetoxy-8β-vinyl-estra-1,3,5(10)-trien-3-ol

11. Use of 8β-substituted estra-1,3,5(10)-triene derivatives of general formula I' 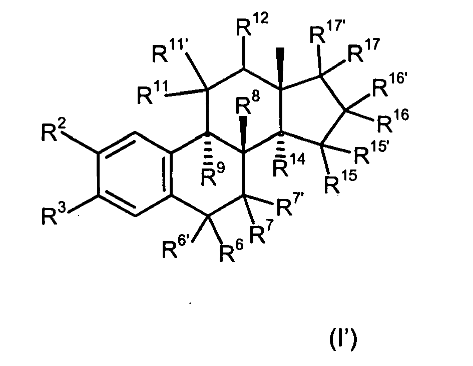 in which
R² means a hydrogen atom, a halogen atom;
a radical R¹⁸- or R¹⁸-O-, whereby R¹⁸ means a hydrogen atom or a straight-chain or branched-chain, saturated or unsaturated hydrocarbon radical with up to 6 carbon atoms, a trifluoromethyl group;
a group R¹⁹SO₂-O-, in which R¹⁹ is an R²⁰R²¹N group, whereby R²⁰ and
R²¹, independently of one another, mean a hydrogen atom, a C₁-C₅-alkyl radical, a group C(O)R²², in which R²² represents an optionally substituted, straight-chain or branched-chain, saturated or unsaturated in up to three places, optionally partially or completely halogenated hydrocarbon radical with up to 10 carbon atoms, an optionally substituted C₃-C₇-cycloalkyl radical, an optionally substituted C₄-C₁₅-cycloalkylalkyl radical or an optionally substituted aryl, heteroaryl or aralkyl radical, or, together with the N-atom, means a polymethylenimino radical with 4 to 6 C atoms or a morpholino radical;
R³ means a group R¹⁸-O-, R¹⁹SO₂-O- or -O-C(O)R²², with R¹⁸, R¹⁹ and R²² in each case in the meaning that is indicated under R², whereby in addition an aryl, heteroaryl or aralkyl radical can stand for R¹⁸;
R⁶ and R⁷ each mean a hydrogen atom or together an additional bond;
R^{6'} and R^{7'} , independently of one another, mean a hydrogen atom, a halogen atom, a group R¹⁸-O-, R¹⁹SO₂-O- or -R²², with R¹⁸, R¹⁹ and R²² in each case in the meaning that is indicated under R²;
R⁸ means a straight-chain or branched-chain, optionally partially or completely halogenated alkyl or alkenyl radical with up to 5 carbon atoms, an ethinyl or prop-1-inyl radical;
R⁹ means a hydrogen atom, a straight-chain or branched-chain, saturated or unsaturated hydrocarbon radical with up to 5 carbon atoms, or together with R¹¹ means an additional bond;
R¹¹ means a hydrogen atom or together with R⁹ or together with R¹² means an additional bond;
R^{11'} means a hydrogen atom, a halogen atom, a saturated or unsaturated, optionally partially or completely halogenated (F, Cl) hydrocarbon radical, which has a maximum linear chain length of 4 carbon atoms, or a group -X-R^{18'}, in which X is an oxygen or sulfur atom, and R^{18'} is an alkyl radical with 1 to 3 carbon atoms;
R¹² means a hydrogen atom or together with R¹¹ means an additional bond;
R¹⁴ means a hydrogen atom or together with R¹⁵ means an additional bond;
R¹⁵ means a hydrogen atom or together with R¹⁴ or together with R¹⁶ means an additional bond;
R¹⁶ means a hydrogen atom or together with R¹⁵ means an additional bond;
R^{15'} and R^{16'} , independently of one another, mean a hydrogen atom, a halogen atom, a group R¹⁸-O-, R¹⁹SO₂-O- or -R²², with R¹⁸, R¹⁹ and R²² in each case in the meaning that is indicated under R²;
R¹⁷ and R^{17'} each mean a hydrogen atom; a hydrogen atom and a halogen atom; a hydrogen atom and a benzyloxy group; a hydrogen atom and a group R¹⁹SO₂-O-; a group R¹⁸ and a group -C(O)R²² or -O-C(O)R²²; a group R¹⁸-O- and a group R¹⁸-; a group R¹⁸-O- and a group -O-C(O)R²², in all above cases with R¹⁸, R¹⁹ and R²² in each case in the meaning that is indicated under R²; or
R¹⁷ and R^{17'} together mean a group =CR²³R²⁴, in which R²³ and R²⁴, independently of one another, represent a hydrogen atom and a halogen atom, or together an oxygen atom;
wherein:
a C₄-C₁₅-cycloalkylalkyl radical has 3 to 7 carbon atoms in the cycloalkyl portion; and the alkyl portion has up to 8 carbon atoms;
an aryl radical is a phenyl, 1- or 2-naphthyl radical or a heteroaryl radical;
an aralkyl radical is a radical that contains in the ring up to 14, preferably 6 to 10 C atoms and in the alkyl chain 1 to 8 C atoms;
the alkyl groups or hydrocarbon radicals can be partially or completely fluorinated or substituted by 1-5 halogen atoms, hydroxy groups or C₁₋₄-alkoxy groups;
one or more hydroxyl groups at C atoms 3, 16 and 17 can be esterified with an aliphatic, straight-chain or branched-chain, saturated or unsaturated C₁₋₁₄-mono- or polycarboxylic acid or an aromatic carboxylic acid or with an α-or β-amino acid.
as an estrogen for the production of a medicament for treatment of perimenopausal and postmenopausal symptoms.

12. Use of estratrienes of general formula I' according to claim 11, in which
R² means a hydrogen or halogen atom or a hydroxy group;
R³ means a group R¹⁸-O-, R¹⁹SO₂-O- or -O-C(O)R²², with R¹⁸, R¹⁹ and R²² in each case in the meaning that is indicated under R² in claim 11, whereby in addition an aryl or aralkyl radical can stand for R¹⁸;
R⁶ and R⁷ each mean a hydrogen atom;
R^{6'} means a hydrogen atom, a hydroxy group, a group R²² in the meaning that is indicated under R² in claim 11;
R^{7'} means a hydrogen atom, a halogen atom, a group R¹⁸-O-, R¹⁹SO₂-O- or -R²², with R¹⁸, R¹⁹ and R²² in each case in the meaning that is indicated under R² in claim 11;
R⁸ means a straight-chain or branched-chain, optionally partially or completely halogenated alkyl or alkenyl radical with up to 5 carbon atoms, an ethinyl- or prop-1-inyl radical;
R⁹ means a hydrogen atom or together with R¹¹ an additional bond;
R¹¹ means a hydrogen atom or together with R⁹ an additional bond;
R^{11'} means a hydrogen atom, a halogen atom, a saturated or unsaturated, optionally partially or completely halogenated hydrocarbon radical, which has a maximum linear chain length of 4 carbon atoms, or a group -X-R^{18'}, in which X is a sulfur atom, and R^{18'} is an alkyl radical with 1 to 3 carbon atoms;
R¹², R¹⁴, R¹⁵ and R¹⁶ in each case mean a hydrogen atom;
R^{16'} means a hydrogen atom, a halogen atom, a group R¹⁸-O-, R¹⁹SO₂-O- or -R²², with R¹⁸, R¹⁹, and R²² in each case in the meaning that is indicated under R² in claim 11;
R¹⁷ and R^{17'} in each case mean a hydrogen atom; a hydrogen atom and a halogen atom; a hydrogen atom and a benzyloxy group; a hydrogen atom and a group R¹⁹SO₂-O-; a group R¹⁸ and a group -C(O)R²² or -O-C(O)R²²; a group R¹⁸-O- and a group R¹⁸-; a group R¹⁸-O- and a group -O-C(O)R²², in all above cases with R¹⁸, R¹⁹ and R²² in each case in the meaning that is indicated under R² in claim 11; and
R¹⁷ and R^{17'} together mean a group =CR²³R²⁴, in which R²³ and R²⁴, independently of one another, represent a hydrogen atom and a halogen atom, or together an oxygen atom.

13. Use of estratrienes of general formula I' according to claim 11, in which
R² means a hydrogen atom or a fluorine atom or a hydroxy group,
R³ means a group R¹⁸-O-, R¹⁹SO₂-O- or -O-C(O)R²², with R¹⁸, R¹⁹, and R²² in each case in the meaning that is indicated under R² in claim 11, whereby in addition an aryl or aralkyl radical can stand for R¹⁸;
R⁶ and R⁷ in each case mean a hydrogen atom;
R^{6'} means a hydrogen atom or a hydroxy group,
R^{7'} means a hydrogen atom, a fluorine or chlorine atom, a group R¹⁸-O-, R¹⁹SO₂-O- or -R²², with R¹⁸, R¹⁹ and R²² in each case in the meaning that is indicated under R² in claim 11;
R⁸ means a straight-chain or branched-chain, optionally partially or completely fluorinated alkyl or alkenyl radical with up to 5 carbon atoms, an ethinyl radical or prop-1-inyl radical;
R⁹, independently of one another, mean a hydrogen atom or together with R¹¹ an additional bond;
R^{11'} means a hydrogen atom, a fluorine or chlorine atom, a saturated, straight-chain or branched-chain C₁-C₄-alkyl group, a group -X-R^{18'}, in which X is a sulfur atom and R^{18'} means a saturated, straight-chain or branched-chain C₁-C₃-alkyl group, a chloromethyl or chloroethyl group;
R¹², R¹⁴, R¹⁵ and R¹⁶ in each case mean a hydrogen atom;
R^{16'} means a hydrogen atom, a fluorine or chlorine atom or a group R¹⁸-O or -R²², with R¹⁸ and R²² in each case in the meaning that is indicated under R² in claim 11;
R¹⁷ and R^{17'} in each case mean a hydrogen atom; a hydrogen atom and a halogen atom; a hydrogen atom and a benzyloxy group; a hydrogen atom and a group R¹⁹SO₂-O-; a group R¹⁸ and a group -C(O)R²² or -O-C(O)R²²; a group R¹⁸-O- and a group R¹⁸-; a group R¹⁸-O- and a group -O-C(O)R²², in all above cases with R¹⁸, R¹⁹ and R²² in each case in the meaning that is indicated under R²; or
R¹⁷and R^{17'} together mean a group =CR²³R²⁴, in which R²³ and R²⁴, independently of one another, represent a hydrogen atom and a halogen atom, or together an oxygen atom.

14. Use of estratrienes of general formula I' according to claim 11, in which R^{6'}, R^{7'}, R⁹, R¹¹, R¹⁴, R¹⁵, R^{15'} and R¹⁶ in each case stand for a hydrogen atom or R^{6'}, R^{7'}, R¹⁴, R¹⁵, R^{15'} and R¹⁶ in each case stand for a hydrogen atom and R⁹ and R¹¹ together stand for an additional bond, and all other substituents have the meanings that are indicated in claim 1.

15. Use of estratrienes of general formula I' according to claim 11, which have a double bond in position 9(11), 14(15) or 15(16) or two double bonds in positions 9(11) and 14(15) or 15(16).

16. Use of estratrienes of general formula I' according to claim 11, in which
R¹⁷ and R^{17'} are a group R¹⁸-O- and a group R¹⁸-; a group R¹⁸- and a group -O-C(O)R²², with R¹⁸ and R²² in each case in the meaning that is indicated under R² in claim 11.

17. Use of estratrienes of general formula I' according to claim 16, in which
R¹⁷ and R^{17'} are a hydroxy group and a hydrogen atom, a C₁-C₄-alkyl group or a C₂-C₄-alkinyl group.

18. Use of estratrienes of general formula I' according to claim 17, in which
R¹⁷ and R^{17'} are a hydroxy group and a hydrogen atom, a methyl, ethinyl or prop-1-inyl group.

19. Use of estratrienes of general formula I' according to claim 11, in which
R^{16'} stands for a group R¹⁸-O- or R¹⁹SO₂-O- with R¹⁸ and R¹⁹ in each case in the meaning that is indicated under R² in claim 11, R¹⁷ and R^{17'} each stand for a hydrogen atom, and all other substituents can have the meanings that are indicated in general formula I.

20. Use of estratrienes of general formula I' according to claim 11, selected from the group of compounds
8β-Methyl-estra-1,3,5(10),9(11)-tetraene-3,17β-diol
3-methoxy-8β-methyl-estra-1,3,5(10),9(11)-tetraen-17β-ol
8β-methyl-estra-1,3,5(10)-triene-3,17β-diol
3-methoxy-8β-methyl-estra-1,3,5(10)-trien-17β-ol
8β-vinyl-estra-1,3,5(10),9(11)-tetraene-3,17β-diol
3-methoxy-8β-vinyl-estra-1,3,5(10),9(11)-tetraen-17β-ol
8β-(2',2'-difluorovinyl)-estra-1,3,5(10),9(11)-tetraene-3,17β-diol
8β-(2',2'-difluorovinyl)-3-methoxy-estra-1,3,5(10),9(11)-tetraen-17β-ol
8β-vinyl-estra-1,3,5(10)-triene-3,17β-diol
3-methoxy-8β-vinyl-estra-1,3,5(10)-trien-17β-ol
8β-(2',2'-difluorovinyl)-estra-1,3,5(10)-triene-3,17β-diol
8β-(2',2'-difluorovinyl)-3-methoxy-estra-1,3,5(10)-trien-17β-ol
8β-ethyl-estra-1,3,5(10)-triene-3,17β-diol
8β-ethyl-3-methoxy-estra-1,3,5(1 0)-trien-17β-ol
8β-vinyl-estradiol-3-sulfamate
8β-vinyl-estradiol-3,17-disulfamate
8β-vinyl-estradiol-3-(N-acetyl)-sulfamate
8β-vinyl-estrone-3-sulfamate
8β-vinyl-estron-3-acetate
8β-vinyl-estriol
8β-vinyl-estriol-3-sulfamate
8β-methyl-estrone-3-sulfamate
8β-methyl-estriol
8β-(prop-(Z)-enyl)-estradiol
8β-(n-propyl)-estradiol
8β-ethinyl-estradiol
17α-ethinyl-8β-vinyl-estra-1,3,5(10)-triene-3,17β-diol
17α-methyl-8β-vinyl-estra-1,3,5,(10)-triene-3,17β-diol
16α-fluoro-8β-methyl-estra-1,3,5(10)-triene-3,17β-diol
8β-vinyl-estra-1,3,5(10)-triene-3,17α-diol
8β-methyl-estra-1,3,5(10)-triene-3,17α-diol
8β-vinyl-estradiol-diacetate
8β-methyl-estradiol-diacetate
8β-vinyl-estradiol-17-valerianate
17β-acetoxy-8β-vinyl-estra-1,3,5(10)-trien-3-ol

21. Use of a compound defined according to claim 11 to 20 for the production of a medicament for the treatment of peri- and post-male-menopausal symptoms.

22. Use according to claim 11 for prevention and treatment of hot flashes, sleep disturbances, irritability, mood swings, incontinence, vaginal atrophy, and hormone-deficiency-induced emotional diseases.

23. Use according to claim 22 for prevention and treatment of diseases in the urogenital tract.

24. Use of a compound defined according to claim 11 for the production of a medicament for the prevention and therapy of gastrointestinal diseases.

25. Use according to claim 24 for prevention and therapy of ulcers and hemorrhagic diatheses in the gastrointestinal tract.

26. Use according to claim 24 for prevention and therapy of neoplasias.

27. Use according to claim 11 to 20 for in-vitro treatment of male infertility.

28. Use of a compound defined according to claim 11 to 20 for the production of a medicament for the in-vivo treatment of male infertility.

29. Use of a compound of general formula I defined according to claim 1 to 10 for the production of a medicament for the in-vitro treatment of female infertility.

30. Use of a compound of general formula I defined according to claim 1 to 10 for the production of a medicament for the in-vivo treatment of female infertility.

31. Use according to claim 11 to 20 of a compound of general formula I' defined according to claim 11 to 20 for hormone replacement therapy (HRT).

32. Use of a compound of general formula I' defined according to claim 11 to 20 for the production of a medicament for the therapy of hormone-deficiency-induced symptoms in the case of surgical, medicinal or ovarian dysfunction.

33. Use according to claim 11 to 20 of a compound of general formula I' defined to claim 11 to 20 for prophylaxis and therapy of a hormone-deficiency-induced bone mass loss.

34. Use according to claim 33 for prophylaxis and therapy of osteoporosis.

35. Use of a compound of general formula l' defined according to claim 11 to 20 for the production of a medicament for the prevention and therapy of cardiovascular diseases.

36. Use of a compound of general formula I' defined according to claim 11 to 20 for the production of a medicament for the prevention and treatment of vascular diseases.

37. Use according to claim 36 for prevention and treatment of arteriosclerosis.

38. Use according to claim 36 for prevention and treatment of neointimal hyperplasias.

39. Use of a compound of general formula I' defined according to claim 11 to 20 for the production of a medicament for the prevention and treatment of hormone-deficiency-induced neurodegenerative diseases.

40. Use of a compound of general formula I' defined according to claim 11 to 20 for the production of a medicament for the prevention and treatment of Alzheimer's disease as well as hormone-deficiency-induced impairment of memory and learning capacity.

41. Use of a compound of general formula I' defined according to claim 11 to 20 for the production of a medicament for the treatment of inflammatory diseases and diseases of the immune system.

42. Use of a compound of general formula I' defined according to claim 11 to 20 for the production of a medicament for the prevention and treatment of benign prostate hyperplasia (BPH).

43. Pharmaceutical compositions that contain at least one compound according to one of claims 1 to 10 as well as a pharmaceutically compatible vehicle.

## Revendications

1. Dérivé 8β-substitués d'estra-1,3,5(10)-triène suivant la formule générale I 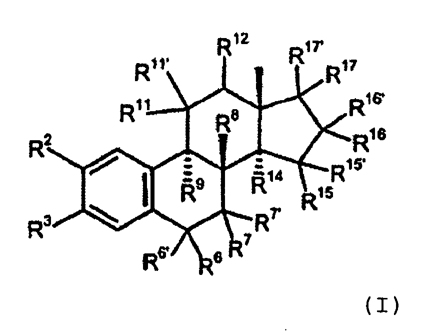 dans laquelle :
R² désigne un atome d'hydrogène, un atome d'halogène;
un radical R¹⁸ ou R¹⁸-O-, R¹⁸ désignant un atome d'hydrogène ou un radical hydrocarbure à chaîne droite ou ramifiée, saturé ou insaturé avec 6 atomes de carbone maximum, un groupe trifluorométhyle;
un groupe R¹⁹SO₂-O- dans lequel R¹⁹ est un groupe R²⁰R²¹N, R²⁰ et R²¹ représentent indépendamment l'un de l'autre un atome d'hydrogène, un radical alkyle en C₁ - C₅, un groupe C(O)R²², dans lequel R²² représente un radical hydrocarbure éventuellement substitué, à chaîne droite ou ramifiée, saturé ou jusqu'à trois fois insaturé, éventuellement partiellement ou complètement halogéné, avec 10 atomes de carbone au maximum, un radical cycloalkyle en C₃ - C₇ éventuellement substitué, un radical cycloalkylalkyle en C₄ - C₁₅ éventuellement substitué ou un radical aryle, hétéroaryle ou aralkyle éventuellement substitué ou désigne, avec l'atome N, un radical polyméthylènenimino avec 4 à 6 atomes de carbone ou un radical morpholino;
R³ désigne un groupe R¹⁸-O- , R¹⁹ SO₂-O- ou -O-C(O)R²², R¹⁹ et R²² ayant chaque fois la signification indiquée sous R², R¹⁸ pouvant désigner un atome d'hydrogène, un groupe trifluorométhyle, un radical isopropyle, butyle, isobutyle, tert-butyle, pentyle, isopentyle, néopentyle, heptyle, hexyle ou un radical aryle, hétéroaryle ou aralkyle;
R⁶ et R⁷ désignent chacun un atome d'hydrogène ou en commun une liaison supplémentaire;
R^{6'} et R^{7'} désignent indépendamment l'un de l'autre un atome d'hydrogène, un atome d'halogène, un groupe R¹⁸-O-, R¹⁹SO₂-O- ou -R²², R¹⁸, R¹⁹ et R²² ayant chaque fois la signification indiquée sous R²;
R⁸ désigne un radical alkyle ou alkényle à chaîne droite ou ramifiée, éventuellement partiellement ou complètement halogéné, avec 5 atomes de carbone au maximum, un radical éthinyle ou prop-1-inyle;
R⁹ désigne un atome d'hydrogène, un radical hydrocarbure à chaîne droite ou ramifiée, saturé ou insaturé avec 5 atomes de carbone au maximum, ou une liaison supplémentaire en commun avec _{R}11_{;}
R¹¹ désigne un atome d'hydrogène ou une liaison supplémentaire avec R⁹ ou avec R¹²;
R^{11'} désigne un atome d'hydrogène, un atome d'halogène, un radical hydrocarbure saturé ou insaturé, éventuellement partiellement ou complètement halogéné, qui présente une longueur de chaîne linéaire maximale de 4 atomes de carbone, ou est un groupe -X-R^{18'} dans lequel X est un atome d'oxygène ou de soufre et R^{18'} est un radical alkyle avec 1 à 3 atomes de carbone;
R¹² désigne un atome d'hydrogène ou une liaison supplémentaire avec R¹¹;
R¹⁴ désigne un atome d'hydrogène ou une liaison supplémentaire avec R¹⁵;
R¹⁵ désigne un atome d'hydrogène ou une liaison supplémentaire avec R¹⁴ ou avec R¹⁶;
R¹⁶ désigne un atome d'hydrogène ou une liaison supplémentaire avec R¹⁵;
R^{15'} et R^{16'} désignent indépendamment l'un de l'autre un atome d'hydrogène, un atome d'halogène, un groupe R¹⁸-O-, R¹⁹SO₂-O- ou -R²² , R¹⁸, R¹⁹ et R²² ayant chacun la signification indiquée sous R²;
R¹⁷ et R^{17'} désignent chacun un atome d'hydrogène; un atome d'hydrogène et un atome d'halogène; un atome d'hydrogène et un groupe benzyloxy; un atome d'hydrogène et un groupe R¹⁹SO₂-O-; un groupe R¹⁸ et un groupe -C (0) R²² ou -O-C(O)R²²; un groupe R¹⁸-O- et un groupe R¹⁸; un groupe R¹⁸-O- et un groupe -O-C(O)R²², dans tous les cas qui précèdent R¹⁸, R¹⁹ et R²² ayant chacun la signification indiquée sous R²; ou
R¹⁷ et R¹⁷ désignent en commun un groupe =CR²³R²⁴, dans lequel R²³ et R²⁴ désignent indépendamment l'un de l'autre un atome d'hydrogène et un atome d'halogène ou en commun un atome d'oxygène;
à l'exception des composés de la formule générale I', dans laquelle :
R³ est un groupe hydroxy ou acétyloxy, et en même temps
R² un atome d'hydrogène,
R⁶, R^{6'}, R⁷ et R^{7'} chacun un atome d'hydrogène,
R⁸ un groupe méthyle,
R⁹ un atome d'hydrogène ou
R⁹ et R¹¹ en commun une liaison supplémentaire,
R^{11'} et R¹² un atome d'hydrogène chacun,
R¹⁴, R¹⁵, R^{15'}, R¹⁶ et R^{16'} un atome d'hydrogène chacun, de même que
R¹⁷ et R^{17'} désignent un groupe β-hydroxy et un atome d'hydrogène; un groupe β-(2-bromacétyl)oxy et un atome d'hydrogène; un groupe β-acétyle et un atome d'hydrogène; ou
R¹⁷ et R^{17'} représentent en commun un atome d'hydrogène
où :
un radical cycloalkylalkyle en C₄₋₁₅ présente 3 à 7 atomes de carbone dans la partie cycloalkyle et 8 atomes de carbone au maximum dans la partie alkyle ;
il s'agit, pour un radical aryle, d'un radical phényle, 1-ou 2-naphtyle ou hétéroaryle;
pour un radical aralkyle, il s'agit d'un radical qui contient dans le noyau 14 atomes de carbone au maximum et dans la chaîne alkyle 1 à 8 atomes de carbone;
les groupes alkyle ou les radicaux hydrocarbure peuvent être fluorés ou substitués partiellement ou complètement par 1 à 5 atomes d'halogène, des groupes hydroxy ou des groupes alcoxy en C₁₋₄;
un ou plusieurs groupes hydroxy sur les atomes de carbone 3, 16 et 17 peuvent être estérifiés avec un acide monocarboxylique ou polycarboxylique en C₁₋₁₄ aliphatique, à chaîne droite ou ramifiée, saturé ou insaturé ou un acide carboxylique aromatique ou avec un α- ou β-aminoacide.

2. Estratriènes de la formule générale I suivant la revendication 1,
dans laquelle :
R² désigne un atome d'hydrogène ou d'halogène ou un groupe hydroxy;
R³ désigne un groupe R¹⁸-O-, R¹⁹SO₂-O- ou -O-C(O)R²², R¹⁹ et R²² ayant chaque fois la signification indiquée sous R² dans la revendication 1, R¹⁸ pouvant être un atome d'hydrogène, un groupe trifluorométhyle, un radical isopropyle, butyle, isobutyle, tert-butyle, pentyle, isopentyle, néopentyle, heptyle ou hexyle ou un radical aryle ou aralkyle;
R⁶ et R⁷ désignent chacun un atome d'hydrogène;
R^{6'} désigne un atome d'hydrogène, un groupe hydroxy, un groupe R²² dans la signification indiquée sous R² dans la revendication 1;
R^{7'} désigne un atome d'hydrogène, un atome d'halogène, un groupe R¹⁸-O-, R¹⁹SO₂-O- ou -R²², R¹⁸, R¹⁹ et R²² ayant la signification indiquée sous R² dans la revendication 1;
R⁸ désigne un radical alkyle ou alkényle à chaîne droite ou ramifiée, éventuellement partiellement ou complètement halogéné avec 5 atomes de carbone au maximum, un radical éthinyle ou prop-1-inyle;
R⁹ désigne un atome d'hydrogène ou une liaison supplémentaire avec R¹¹;
R¹¹ désigne un atome d'hydrogène ou une liaison supplémentaire avec R⁹;
R^{11'} désigne un atome d'hydrogène, un atome d'halogène, un radical hydrocarbure saturé ou insaturé, éventuellement partiellement ou complètement halogéné qui présente une longueur de chaîne linéaire maximale de 4 atomes de carbone, ou est un groupe -X-R^{18'}, dans lequel X est un atome de soufre et R^{18'} un radical alkyle avec 1 à 3 atomes de carbone;
R¹², R¹⁴, R¹⁵, R^{15'} et R¹⁶ désignent chaque fois un atome d'hydrogène;
R^{16'} désigne un atome d'hydrogène, un atome d'halogène, un groupe R¹⁸-O-, R¹⁹ SO₂-O- ou -R²², R¹⁸, R¹⁹ et R²² ayant chaque fois la signification indiquée sous R² dans la revendication 1;
R¹⁷ et R^{17'} désignent chaque fois un atome d'hydrogène; un atome d'hydrogène et un atome d'halogène; un atome d'hydrogène et un groupe benzyloxy; un atome d'hydrogène et un groupe R¹⁹SO₂-O-; un groupe R¹⁸ et un groupe -C(O)R²² ou -O-C(O)R²²; un groupe R¹⁸-O- et un groupe R¹⁸; un groupe R¹⁸-O-et un groupe -O-C(O)R²², dans tous les cas qui précèdent, R¹⁸, R¹⁹ et R²² ayant chaque fois la signification indiquée sous R² dans la revendication 1; de même que
R¹⁷ et R^{17'} désignent en commun un groupe =CR²³R²⁴ dans lequel R²³ et R²⁴ représentent indépendamment l'un de l'autre un atome d'hydrogène et un atome d'halogène, ou en commun un atome d'oxygène;

3. Estratriènes de la formule générale I suivant la revendication 1,
dans laquelle :
R² désigne un atome d'hydrogène ou de fluor ou un groupe hydroxy;
R³ désigne un groupe R¹⁸-O-, R¹⁹SO₂-O- ou -O-C(O)R²², R¹⁹ et R²² ayant chaque fois la signification indiquée sous R² dans la revendication 1, R¹⁸ pouvant représenter un atome d'hydrogène, un groupe trifluorométhyle, un radical isopropyle, butyle, isobutyle, tert-butyle, pentyle, isopentyle, néopentyle, heptyle ou hexyle ou un radical aryle ou aralkyle;
R⁶ et R⁷ désignent chacun un atome d'hydrogène;
R^{6'} désigne un atome d'hydrogène ou un groupe hydroxy;
R^{7'} désigne un atome d'hydrogène, un atome de fluor ou de chlore, un groupe R¹⁸-O-, R¹⁹SO₂-O- ou -R²², R¹⁸ R¹⁹ et R²² ayant chacun la signification indiquée sous R² dans la revendication 1;
R⁸ désigne un radical alkyle ou alkényle à chaîne droite ou ramifiée, éventuellement partiellement ou totalement fluoré avec 5 atomes de carbone au maximum, un radical éthinyle ou prop-1-inyle;
R⁹ désigne indépendamment l'un de l'autre un atome d'hydrogène ou une liaison supplémentaire avec R¹¹;
R^{11'} désigne un atome d'hydrogène, un atome de fluor ou de chlore, un groupe alkyle en C₁-C₄ saturé, à chaîne droite ou ramifiée, un groupe -X-R^{18'} dans lequel X est un atome de soufre et R^{18'} un radical alkyle avec 1 à 3 atomes de carbone;
R¹², R¹⁴, R¹⁵, R^{15'} et R¹⁶ désignent chacun un atome d'hydrogène;
R^{16'} désigne un atome d'hydrogène, un atome de fluor ou de chlore ou un groupe R¹⁸-O ou -R²², R¹⁸ et R²² ayant chacun la signification indiquée sous R² dans la revendication 1;
R¹⁷ et R^{17'} désignent chacun un atome d'hydrogène; un atome d'hydrogène et un atome d'halogène; un atome d'hydrogène et un groupe benzyloxy; un atome d'hydrogène et un groupe R¹⁹SO₂-O- ; un groupe R¹⁸ et un groupe -C(O)R²² ou -O-C(O)R²²; un groupe R¹⁸-O- et un groupe R¹⁸-; un groupe R¹⁸-O- et un groupe -O-C(O)R²², dans tous les cas qui précèdent, R¹⁸, R¹⁹ et R²² ayant chacun la signification indiquée sous R² dans la revendication 1; ou
R¹⁷ et R^{17'} désignent en commun un groupe =CR²³R²⁴, dans lequel R²³ et R²⁴ représentent indépendamment l'un de l'autre un atome d'hydrogène et un atome d'halogène ou en commun un atome d'oxygène;

4. Estratriènes de la formule générale I suivant la revendication 1,
dans laquelle :
R^{6'}, R^{7'}, R⁹, R¹¹, R¹⁴, R¹⁵, R^{15'} et R¹⁶ désignent chacun un atome d'hydrogène ou R^{6'}, R^{7'}, R¹⁴, R¹⁵, R^{15'} et R¹⁶ désignent chacun un atome d'hydrogène de même que R⁹ et R¹¹ en commun une liaison supplémentaire et tous les autres substituants ont la signification indiquée dans la revendication 1.

5. Estratriènes de la formule générale I suivant la revendication 1, qui présentent dans la position 9(11), 14(15) ou 15(16), une liaison double ou, dans les positions 9(11), 14(15) et 15(16), deux liaisons doubles.

6. Estratriènes de la formule générale I suivant la revendication 1,
dans laquelle :
R¹⁷ et R^{17'} désignent un groupe R¹⁸-O- et un groupe R¹⁸-; un groupe R¹⁸- et un groupe -O-C (O)R²², R¹⁸ et R²² ayant chacun la signification indiquée sous R² dans la revendication 1.

7. Estratriènes de la formule générale I suivant la revendication 6,
dans laquelle :
R¹⁷ et R^{17'} désignent un groupe hydroxy et un atome d'hydrogène, un groupe alkyle en C₁-C₄ ou un groupe alkinyle en C₂-C₄.

8. Estratriènes de la formule générale I suivant la revendication 7,
dans laquelle :
R¹⁷ et R^{17'} désignent un groupe hydroxy et un atome d'hydrogène, un groupe méthyle, éthinyle ou prop-1-inyle.

9. Estratriènes de la formule générale I suivant la revendication 1,
dans laquelle :
R^{16'} désigne un groupe R¹⁸-O- ou R¹⁹SO₂-O-_{,} R¹⁸ et R¹⁹ ayant chacun la signification indiquée sous R² dans la revendication 1, R¹⁷ et R^{17'} représentant chacun un atome d'hydrogène, de même que tous les autres substituants qui peuvent avoir les significations indiquées dans la formule générale I.

10. Estratriènes de la formule
8β-vinyle-estra-1,3,5(10),9(11)-tétraène-3,17β-diol
3-méthoxy-8β-vinyle-estra-1,3,5(10),9(11)-tétraène-17β-ol
8β-(2',2'-difluorovinyle)-estra-1,3,5(10),9(11)-tétraène-3,17β-diol
8β-(2',2'-difluorovinyle)-3-méthoxy-estra-1,3,5(10),9(11)-tétraène-17β-ol
8β-vinyle-estra-1,3,5(10)-triène-3,17β-diol 3-méthoxy-8β-vinyle-estra-1,3,5(10)-triène-17β-ol
8β-(2',2'-difluorovinyle)-estra-1,3,5(10)-triène-3,17β-diol
8β-(2',2'-difluorovinyle)-3-méthoxy-estra-1,3,5(10)-triène-17β-ol
8β-éthyle-estra-1,3,5(10)-triène-3,17β-diol 8β-éthyle-3-méthoxy-estra-1,3,5(10)-triène-17β-ol
8β-vinyle-estradiol-3-sulfamate
8β-vinyle-estradiol-3,17-disulfamate
8β-vinyle-estradiol-3-(N-acétyle)-sulfamate
8β-vinyle-estrone-3-sulfamate
8β-vinyle-estrone-3-acétate
8β-vinyle-estriol
8β-vinyle-estriol-3-sulfamate
8β-méthyle-estrone-3-sulfamate
8β-méthyle-estriol
8β-(prop-(Z)-enyle)-estradiol
8β-(n-propyle)-estradiol
8β-éthinyle-estradiol
17α-éthinyle-8β-vinyle-estra-1,3,5(10)-triène-3,17β-diol
17α-méthyle-8β-vinyle-estra-1,3,5(10)-triène-3,17β-diol
16α-fluor-8β-méthyle-estra-1,3,5(10)-triène-3,17β-diol
8β-vinyle-estra-1,3,5(10)-triène-3,17α-diol
8β-méthyle-estra-1,3,5(10)-triène-3,17α-diol
8β-vinylé-estradiol-diacétate
8β-méthyle-estradiol-diacétate
8β-vinyle-estradiol-17-valérianate
17β-acetoxy-8β-vinyle-estra-1,3,5(10)-triène-3-ol

11. Utilisation de dérivés d'estra-1,3,5(10)-triène à substitution 8β de la formule générale I' 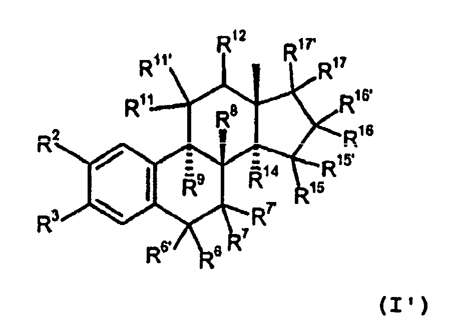 dans laquelle :
R² désigne un atome d'hydrogène, un atome d'halogène;
un radical R¹⁸- ou R¹⁸-O-, R¹⁸ désignant un atome d'hydrogène ou un radical hydrocarbure à chaîne droite ou ramifiée, saturé ou insaturé, avec 6 atomes de carbone au maximum, un groupe trifluorométhyle;
un groupe R¹⁹SO₂-O-, où R¹⁹ est un groupe R²⁰R²¹N, R²⁰ et R²¹ désignant indépendamment l'un de l'autre un atome d'hydrogène, un radical alkyle en C₁ - C₅, un groupe C(O) R²², dans lequel R²² désigne un radical hydrocarbure éventuellement substitué, à chaîne droite ou ramifiée, saturé ou jusqu'à trois fois insaturé, éventuellement partiellement ou complètement halogéné avec 10 atomes de carbone au maximum, un radical cycloalkyle en C₃ - C₇ éventuellement substitué, un radical cycloalkylalkyle en C₄ - C₁₅ éventuellement substitué ou un radical aryle, hétéroaryle ou aralkyle éventuellement substitué ou désigne, avec l'atome N, un radical polyméthylèneimino avec 4 à 6 atomes de carbone ou un radical morpholino;
R³ désigne un groupe R¹⁸-O-, R¹⁹SO₂-O- ou -O-C(O)R²², R¹⁸, R¹⁹ et R²² pouvant avoir dans chaque cas la signification indiquée sous R², R¹⁸ pouvant en outre représenter un radical aryle, hétéroaryle ou aralkyle;
R⁶ et R⁷ désignent chacun un atome d'hydrogène ou en commun une liaison supplémentaire;
R^{6'} et R⁷ désignent indépendamment l'un de l'autre un atome d'hydrogène, un atome d'halogène, un groupe R¹⁸-O-, R¹⁹SO₂-O- ou -R²², R¹⁸, R¹⁹ et R²² ayant chacun la signification indiquée sous R²;
R⁸ désigne un radical alkyle ou alkényle à chaîne droite ou ramifiée, éventuellement partiellement ou complètement halogéné avec 5 atomes de carbone au maximum, un radical éthinyle ou prop-1-inyle;
R⁹ désigne un atome d'hydrogène, un radical hydrocarbure à chaîne droite ou ramifiée, saturé ou insaturé avec 5 atomes de carbone au maximum, ou une liaison supplémentaire avec R¹¹;
R¹¹ désigne un atome d'hydrogène ou une liaison supplémentaire avec R⁹ ou avec R¹²;
R^{11'} désigne un atome d'hydrogène, un atome d'halogène, un radical hydrocarbure saturé ou insaturé, éventuellement partiellement ou complètement halogéné qui présente une longueur de chaîne linéaire maximale de 4 atomes de carbone, ou un groupe -X-R^{18'} dans lequel X est un atome d'oxygène ou de soufre et R^{18'} un radical alkyle avec 1 à 3 atomes de carbone;
R¹² désigne un atome d'hydrogène ou une liaison supplémentaire avec R¹¹;
R¹⁴ désigne un atome d'hydrogène ou une liaison supplémentaire avec R¹⁵;
R¹⁵ désigne un atome d'hydrogène ou une liaison supplémentaire avec R¹⁴ ou R¹⁶;
R¹⁶ désigne un atome d'hydrogène ou une liaison supplémentaire avec R¹⁵;
R^{15'} et R^{16'} désignent indépendamment l'un de l'autre un atome d'hydrogène, un atome d'halogène, un groupe R¹⁸-O-, R¹⁹SO₂-O- ou -R²², R¹⁸, R¹⁹ et R²² ayant chacun la signification indiquée sous R²;
R¹⁷ et R^{17'} désignent chacun un atome d'hydrogène; un atome d'hydrogène et un atome d'halogène; un atome d'hydrogène et un groupe benzyloxy; un atome d'hydrogène et un groupe R¹⁹SO₂-O-; un groupe R¹⁸ et un groupe -C(O)R²² ou -O-C(O)R²² ; un groupe R¹⁸-O- et un groupe R¹⁸-; un groupe R¹⁸-O- et un groupe -O-C(O)R²², dans tous les cas qui précèdent, R¹⁸, R¹⁹ et R²² ayant chacun la signification indiquée sous R²; ou
R¹⁷ et R^{17'} désignent en commun un groupe =CR²³R²⁴, où R²³ et R²⁴ représentent indépendamment l'un de l'autre un atome d'hydrogène et un atome d'halogène ou en commun un atome d'oxygène;
où :
un radical cycloalkylalkyle en C₄₋₁₅ présente 3 à 7 atomes de carbone dans la partie cycloalkyle et 8 atomes de carbone au maximum dans la partie alkyle;
il s'agit, pour un radical aryle, d'un radical phényle, 1-ou 2-naphtyle ou hétéroaryle;
pour un radical aralkyle, il s'agit d'un radical qui contient dans le noyau 14 atomes de carbone au maximum et dans la chaîne alkyle 1 à 8 atomes de carbone;
les groupes alkyle ou les radicaux hydrocarbure peuvent être fluorés ou substitués partiellement ou complètement par 1 à 5 atomes d'halogène, des groupes hydroxy ou des groupes alcoxy en C₁₋₄;
un ou plusieurs groupes hydroxy sur les atomes de carbone 3, 16 et 17 peuvent être estérifiés avec un acide monocarboxylique ou polycarboxylique en C₁₋₁₄ aliphatique, à chaîne droite ou ramifiée, saturé ou insaturé ou un acide carboxylique aromatique ou avec un α- ou β-aminoacide,
en tant qu'estrogènes pour la préparation d'un médicament pour le traitement de plaintes périménopausiques et post-ménopausiques.

12. Utilisation d'estratriènes de la formule générale I' suivant la revendication 11,
dans laquelle :
R² désigne un atome d'hydrogène ou d'halogène ou un groupe hydroxy;
R³ désigne un groupe R¹⁸-O-, R¹⁹SO₂-O- ou -O-C(O)R²², R¹⁸, R¹⁹ et R²² ayant chacun la signification indiquée sous R² dans la revendication 11, R¹⁸ pouvant désigner en outre un radical aryle ou aralkyle;
R⁶ et R⁷ désignent chacun un atome d'hydrogène;
R^{6'} désigne un atome d'hydrogène, un groupe hydroxy, un groupe R²² dans la signification indiquée sous R² dans la revendication 11;
R^{7'} désigne un atome d'hydrogène, un atome d' halogène, un groupe R¹⁸-O-, R¹⁹SO₂-O- ou -_{R}²², R¹⁸, R¹⁹ et R²² ayant chacun la signification indiquée sous R² dans la revendication 11;
R⁸ désigne un radical alkyle ou alkényle à chaîne droite ou ramifiée, éventuellement partiellement ou complètement halogéné avec 5 atomes de carbone au maximum, un radical éthinyle ou prop-1-inyle;
R⁹ désigne un atome d'hydrogène ou une liaison supplémentaire avec R¹¹;
R¹¹ désigne un atome d'hydrogène ou une liaison supplémentaire avec R⁹;
R^{11'} désigne un atome d'hydrogène, un atome d'halogène, un radical hydrocarbure saturé ou insaturé, éventuellement partiellement ou complètement halogéné, qui présente une longueur de chaîne linéaire maximale de 4 atomes de carbone, ou un groupe -X-R^{18'}, dans lequel X est un atome de soufre et R^{18'} un radical alkyle avec 1 à 3 atomes de carbone;
R¹², R¹⁴, R¹⁵, R¹⁵' et R¹⁶ désignent chacun un atome d'hydrogène;
R^{16'} désigne un atome d'hydrogène, un atome d'halogène, un groupe R¹⁸-O-, R¹⁹SO₂-O- ou -R²², R¹⁸, R¹⁹ et R²² ayant chacun la signification indiquée sous R² dans la revendication 11;
R¹⁷ et R^{17'} désignent chacun un atome d'hydrogène; un atome d'hydrogène et un atome d'halogène; un atome d'hydrogène et un groupe benzyloxy; un atome d'hydrogène et un groupe R¹⁹SO₂-O-; un groupe R¹⁸ et un groupe -C(O)R²² ou -O-C(O)R²² ; un groupe R¹⁸-O- et un groupe R¹⁸-; un groupe R¹⁸-O- et un groupe -O-C(O)R²², dans tous les cas qui précèdent, R¹⁸, R¹⁹ et R²² ayant chacun la signification indiquée sous R² dans la revendication 11; de même que
R¹⁷ et R^{17'} désignent en commun un groupe =CR²³R²⁴, dans lequel R²³ et R²⁴ représentent indépendamment l'un de l'autre un atome d'hydrogène et un atome d'halogène ou en commun un atome d'oxygène.

13. Utilisation d'estratriènes de la formule générale I' suivant la revendication 11,
dans laquelle :
R² désigne un atome d'hydrogène ou de fluor ou un groupe hydroxy;
R³ désigne un groupe R¹⁸-O-, R¹⁹SO₂O- ou -O-C(O)R²², R¹⁸, R¹⁹ et R²² ayant chaque fois la signification indiquée sous R² dans la revendication 11, R¹⁸ pouvant en outre représenter un radical aryle ou aralkyle;
R⁶ et R⁷ désignent chacun un atome d'hydrogène;
R^{6'} désigne un atome d'hydrogène ou un groupe hydroxy;
R^{7'} désigne un atome d'hydrogène, un atome de fluor ou de chlore, un groupe R¹⁸-O-, R¹⁹SO₂-O- ou -R²², R¹⁸, R¹⁹ et R²² ayant chacun la signification indiquée sous R² dans la revendication 11;
R⁸ désigne un radical alkyle ou alkényle à chaîne droite ou ramifiée, éventuellement partiellement ou complètement fluoré avec 5 atomes de carbone au maximum, un radical éthinyle ou prop-1-inyle;
R⁹ désigne indépendamment l'un de l'autre un atome d'hydrogène ou une liaison supplémentaire avec R¹¹;
R^{11'} désigne un atome d'hydrogène, un atome de fluor ou de chlore, un groupe alkyle en C₁-C₄ saturé, à chaîne droite ou ramifiée, un groupe -X-R^{18'} dans lequel X est un atome de soufre et R^{18'} un radical alkyle avec 1 à 3 atomes de carbone;
R¹², R¹⁴, R¹⁵, R^{15'} et R¹⁶ désignent chacun un atome d'hydrogène;
R^{16'} désigne un atome d'hydrogène, un atome de fluor ou de chlore ou un groupe R¹⁸-O- ou -R²², R¹⁸ et R²² ayant chacun la signification indiquée sous R² dans la revendication 11;
R¹⁷ et R^{17'} désignent chacun un atome d'hydrogène; un atome d'hydrogène et un atome d'halogène; un atome d'hydrogène et un groupe benzyloxy; un atome d'hydrogène et un groupe R¹⁹SO₂-O-; un groupe R¹⁸ et un groupe -C (O) R²² ou -O-C (O) R²² ; un groupe R¹⁸-O- et un groupe R¹⁸-; un groupe R¹⁸-O- et un groupe -O-C(O)R²², dans tous les cas qui précèdent, R¹⁸, R¹⁹ et R²² ayant chacun la signification indiquée sous R² dans la revendication 11; ou
R¹⁷ et R^{17'} désignent en commun un groupe =CR²³R²⁴, dans lequel R²³ et R²⁴ représentent indépendamment l'un de l'autre un atome d'hydrogène et un atome d'halogène ou en commun un atome d'oxygène.

14. Utilisation d'estratriènes de la formule générale I' suivant la revendication 11,
dans laquelle :
R^{6'}, R^{7'}, R⁹, R¹¹, R¹⁴, R¹⁵, R^{15'} et R¹⁶ désignent chacun un atome d'hydrogène ou R⁶', R⁷', R¹⁴, R¹⁵, R¹⁵, et R¹⁶ désignent chacun un atome d'hydrogène de même que R⁹ et R¹¹ en commun une liaison supplémentaire et tous les autres substituants ont la signification indiquée dans la revendication 1.

15. Utilisation d'estratriènes de la formule générale I' suivant la revendication 11,
qui présentent, dans la position 9(11), 14(15) ou 15(16) une liaison double, ou dans les positions 9(11) et 14(15) ou 15(16), deux liaisons doubles.

16. Utilisation d'estratriènes de la formule générale I' suivant la revendication 11,
dans laquelle :
R¹⁷ et R^{17'} désignent un groupe R¹⁸-O- et un groupe R¹⁸-; un groupe R¹⁸- et un groupe -O-C (O) R²², R¹⁸ et R²² ayant chacun la signification indiquée sous R² dans la revendication 11.

17. Utilisation d'estratriènes de la formule générale I' suivant la revendication 16,
dans laquelle :
R¹⁷ et R^{17'} désignent un groupe hydroxy et un atome d'hydrogène, un groupe alkyle en C₁-C₄ ou un groupe alkinyle en C₂-C₄.

18. Utilisation d'estratriènes de la formule générale I' suivant la revendication 17,
dans laquelle :
R¹⁷ et R^{17'} désignent un groupe hydroxy et un atome d'hydrogène, un groupe méthyle, éthinyle ou prop-1-inyle.

19. Utilisation d'estratriènes de la formule générale I' suivant la revendication 11,
dans laquelle :
R^{16'} désigne un groupe R¹⁸-O- ou R¹⁹SO²-O-, R¹⁸ et R¹⁹ ayant chacun la signification indiquée sous R² dans la revendication 11, R¹⁷ et R^{17'} représentant chacun un atome d'hydrogène et tous les autres substituants pouvant avoir les significations indiquées dans la formule générale I.

20. Utilisation d'estratriènes de la formule générale I' suivant la revendication 11, sélectionnés dans le groupe des composés
8β-méthyle-estra-1,3,5(10),9(11)-tétraène-3,17β-diol 3-méthoxy-8β-méthyle-estra-1,3,5(10),9(11)-tétraène-17β-ol
8β-méthyle-estra-1,3,5(10)-triène-3,17β-diol
3-méthoxy-8β-méthyle-estra-1,3,5(10)-triène-17β-ol
8β-vinyle-estra-1,3,5(10),9(11)-tétraène-3,17β-diol
3-méthoxy-8β-vinyle-estra-1,3,5(10),9(11)-tétraène-17β-ol
8β-(2',2'-difluorovinyle)-estra-1,3,5(10),9(11)-tétraène-3,17β-diol
8β-(2',2'-difluorovinyle)-3-méthoxy-estra-1,3,5(10),9(11)-tétraène-17β-ol
8β-vinyle-estra-1,3,5(10)-triène-3,17β-diol
3-méthoxy-8β-vinyle-estra-1,3,5(10)-triène-17β-ol
8β-(2',2'-difluorovinyle)-estra-1,3,5(10)-triène-3,17β-diol
8β-(2',2'-difluorovinyle)-3-méthoxy-estra-1,3,5(10)-triène-17β-ol
8β-éthyle-estra-1,3,5(10)-triène-3,17β-diol
8β-éthyle-3-méthoxy-estra-1,3,5(10)-triène-17β-ol
8β-vinyle-estradiol-3-sulfamate
8β-vinyle-estradiol-3,17-disulfamate
8β-vinyle-estradiol-3-(N-acétyle)-sulfamate
8β-vinyle-estrone-3-sulfamate
8β-vinyle-estrone-3-acétate
8β-vinyle-estriol
8β-vinyle-estriol-3-sulfamate
8β-méthyle-estrone-3-sulfamate
8β-méthyle-estriol
8β-(prop-(Z)-ényle)-estradiol
8β-(n-propyle)-estradiol
8β-éthinyle-estradiol
17α-éthinyle-8β-vinyle-estra-1,3,5(10)-triène-3,17β-diol
17α-méthyle-8β-vinyle-estra-1,3,5(10)-triène-3,17β-diol
16α-fluor-8β-méthyle-estra-1,3,5(10)-triène-3,17β-diol
8β-vinyle-estra-1,3,5(10)-triène-3,17α-diol
8β-méthyle-estra-1,3,5(10)-triène-3,17α-diol
8β-vinyle-estradiol-diacétate
8β-méthyle-estradiol-diacétate
8β-vinyle-estradiol-17-valérianate
17β-acetoxy-8β-vinyle-estra-1,3,5(10)-triène-3-ol

21. Utilisation d'un composé de la formule générale I' défini suivant les revendications 11 à 20 pour la préparation de médicaments pour le traitement de plaintes périandropausiques et postandropausiques.

22. Utilisation suivant la revendication 11 pour la prévention et le traitement de : bouffées de chaleur, troubles du sommeil, irritabilité, changements d'humeur, incontinence, atrophie vaginale et dépressions conditionnées par des déficiences hormonales.

23. Utilisation suivant la revendication 22 pour la prévention et le traitement d'affections des voies urogénitales.

24. Utilisation d'un composé de la formule générale I' défini suivant les revendications 11 à 20 pour la préparation d'un médicament pour la prévention et la thérapie d'affections gastro-intestinales.

25. Utilisation suivant la revendication 24 pour la prévention et la thérapie d'ulcères et de diathèses hémorragiques du tube gastro-intestinal.

26. Utilisation suivant la revendication 24 pour la prévention et la thérapie de néoplasies.

27. Utilisation d'un composé de la formule générale I' défini suivant les revendications 11 à 20 pour la préparation d'un médicament pour le traitement in vitro de la stérilité masculine.

28. Utilisation d'un composé de la formule générale I' défini suivant les revendications 11 à 20 pour la préparation d'un médicament pour le traitement in vivo de la stérilité masculine.

29. Utilisation d'un composé de la formule générale I défini suivant les revendications 1 à 10 pour la préparation d'un médicament pour le traitement in vitro de la stérilité féminine.

30. Utilisation d'un composé de la formule générale I défini suivant les revendications 1 à 10 pour la préparation d'un médicament pour le traitement in vivo de la stérilité féminine.

31. Utilisation suivant les revendications 11 à 20 d'un composé de la formule générale I' défini suivant les revendications 11 à 20 pour la thérapie de substitution hormonale (TSH).

32. Utilisation d'un composé de la formule générale I' défini suivant les revendications 11 à 20 pour la préparation d'un médicament pour la thérapie de plaintes conditionnées par des déficiences hormonales en cas de dysfonctions ovariennes conditionnées par la chirurgie, les médicaments ou autrement.

33. Utilisation selon les revendications 11 à 20 d'un composé de la formule générale I' défini suivant les revendications 11 à 20 pour la prophylaxie et la thérapie d'une perte de masse osseuse conditionnée par des déficiences hormonales.

34. Utilisation suivant la revendication 33 pour la prophylaxie et la thérapie de l'ostéoporose.

35. Utilisation d'un composé de la formule générale I' défini suivant les revendications 11 à 20 pour la préparation d'un médicament pour la prévention et la thérapie de maladies de la circulation.

36. Utilisation d'un composé de la formule générale I' défini suivant les revendications 11 à 20 pour la préparation d'un médicament pour la prévention et le traitement de maladies des vaisseaux.

37. Utilisation suivant la revendication 36 pour la prévention et le traitement de l'athérosclérose.

38. Utilisation suivant la revendication 36 pour la prévention et le traitement d'hyperplasies néointimes.

39. Utilisation d'un composé de la formule générale I' défini suivant les revendications 11 à 20 pour la préparation d'un médicament pour la prévention et le traitement d'affections neurodégénératives conditionnées par des déficiences hormonales.

40. Utilisation d'un composé de la formule générale I' défini suivant les revendications 11 à 20 pour la préparation d'un médicament pour la prévention et le traitement de la maladie d'Alzheimer, de même que pour la diminution conditionnée par des déficiences hormonales des facultés de mémorisation et d'apprentissage.

41. Utilisation d'un composé de la formule générale I' défini suivant les revendications 11 à 20 pour la préparation d'un médicament pour le traitement de maladies inflammatoires et de maladies du système immunitaire.

42. Utilisation d'un composé de la formule générale I' défini suivant les revendications 11 à 20 pour la préparation d'un médicament pour la prévention et le traitement de l'hyperplasie bénigne de la prostate (HBP).

43. Composés pharmaceutiques, comprenant au moins un composé suivant l'une des revendications 1 à 10, de même qu'un support pharmaceutiquement compatible.
